(19) Europäisches Patentamt
European Patent Office
Office européen des brevets

(11) EP 1 265 920 B1

(12) # EUROPEAN PATENT SPECIFICATION

(45) Date of publication and mention of the grant of the patent:
**08.11.2006 Bulletin 2006/45**

(21) Application number: **01922679.4**

(22) Date of filing: **22.03.2001**

(51) Int Cl.:
*C07K 14/47* (2006.01) *C12N 15/12* (2006.01)
*C12N 15/11* (2006.01) *A61P 35/00* (2006.01)
*A61P 9/10* (2006.01) *C12N 5/10* (2006.01)
*C12Q 1/68* (2006.01) *A01K 67/027* (2006.01)

(86) International application number:
**PCT/US2001/009609**

(87) International publication number:
**WO 2001/070808 (27.09.2001 Gazette 2001/39)**

(54) **ANGIOGENESIS-ASSOCIATED PROTEINS, AND NUCLEIC ACIDS ENCODING THE SAME**

ANGIOGENESE-ASSOZIIERTE PROTEINE UND DAFÜR KODIERENDE NUKLEINSÄURE

PROTEINES ASSOCIEES A L'ANGIOGENESE ET ACIDES NUCLEIQUES CODANT CES PROTEINES

(84) Designated Contracting States:
**AT BE CH CY DE DK ES FI FR GB GR IE IT LI LU MC NL PT SE TR**

(30) Priority: **22.03.2000 US 191134 P**

(43) Date of publication of application:
**18.12.2002 Bulletin 2002/51**

(73) Proprietors:
- **Curagen Corporation**
  **New Haven, CT 06511 (US)**
- **GENENTECH, INC.**
  **South San Francisco**
  **California 94080 (US)**

(72) Inventors:
- **RASTELLI, Luca, K.**
  **Guilford, CT 06437 (US)**
- **GERRITSEN, Mary**
  **San Mateo,CA 94402 (US)**

(74) Representative: **MacLean, Martin Robert et al**
**Mathys & Squire**
**120 Holborn**
**London EC1N 2SQ (GB)**

(56) References cited:
- **DATABASE EMBL [Online] 3 July 1998 (1998-07-03) OKABE,S. ET AL.: "Mus musculus mRNA for BAZF, complete cds." Database accession no. AB011665 XP002237974 -& OKABE,S. ET AL.: "BAZF, a Novel Bcl6 Homolog, Functions as a Transcriptional Repressor" MOLECULAR AND CELLULAR BIOLOGY, vol. 18, no. 7, July 1998 (1998-07), pages 4235-4244, XP002237973**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 265 920 B1

SEQUENCE LISTING

<110> CURAGEN CORPORATION
GENENTECH, INC.
<120> ANGIOGENESIS ASSOCIATED PROTEINS AND NUCLEIC ACIDS
ENCODING THE SAME
<130> 10716/41
<140> PCT/US01/09609
<141> 2001-03-22
<150> 60/191,134
<151> 2000-03-22
<160> 17
<170> Patent In Ver. 2.1

<210> 1
<211> 2577
<212> DNA
<213> Homo sapiens
<400> 1

```
ctggcctaga tactacaact gaactttttt tctttttagt tactccacag gatccgctga 60
acataggatg ttgccacaaa atctacctcg tgtatttttc tctttcactc atgagctgca 120
caattgcaga tttgagcaca atgtctgcag actgtgttga aaaactctga agaacctaat 180
taacacagga tgacctagga gtgattctaa gtctgtgtaa caagatatta ctcattagtg 240
aatgtgtcag tcttggtact gaatgctgca gataacagca agtaggttct cctttatttc 300
tgaagtattc acttgacctt ccatcagtaa gacggacttt tctaatctgt tcctggagat 360
attaatggaa tacagtcatg tccactcaag acgagaggca gatcaatact gaatatgctg 420
tgtcattgtt ggaacagttg aaactgtttt atgaacagca gttgtttact gacatagtgt 480
taattgttga gggcactgaa ttcccttgtc ataagatggt tcttgcaaca tgtagctctt 540
atttcagggc catgtttatg agtggactaa gtgaaagcaa acaaacccat gtacacctga 600
ggaatgtcga tgctgccacc ttacagataa taataactta tgcatacacg ggtaacttgg 660
caatgaatga cagcactgta gaacagcttt atgaaacagc ttgcttccta caggtagaag 720
atgtgttaca acgttgtcga gaatatttaa ttaaaaaaat aaatgcagag aattgtgtac 780
gattgttgag ttttgctgat ctcttcagtt gtgaggaatt aaaacagagt gctaaaagaa 840
tggtggagca caagttcact gctgtgtatc atcaggacgc gttcatgcag ctgtcacatg 900
acctactgat agatattctc agtagtgaca atttaaatgt agaaaaggaa gaaaccgttc 960
gagaagctgc tatgctgtgg ctagagtata acacagaatc acgatcccag tatttgtctt 1020
ctgttcttag ccaaatcaga attgatgcac tttcagaagt aacacagaga gcttggtttc 1080
aaggtctgcc acccaatgat aagtcagtgg tggttcaagg tctgtataag tccatgccca 1140
agtttttcaa accaagactt gggatgacta aagaggaaat gatgattttc attgaagcat 1200
cttcagaaaa tccttgtagt ctttactctt ctgtctgtta cagcccccaa gcagaaaaag 1260
tttacaagtt atgtagccca ccagctgatt tgcataaggt tgggaccgtt gtaactcctg 1320
ataatgatat ctacatagca ggggggtcag ttcctctgaa aaacacaaaa acaaatcaca 1380
gtaaaacaag caaacttcag actgccttca gaactgtgaa ttgcttttat tggtttgatg 1440
cacagcaaaa tacctggttt ccaaagaccc caatgctttt tgtccgcata aagccatctt 1500
tggtttgctg tgaaggctat atctatgcaa ttggaggaga tagcgtaggt ggagaactta 1560
atcggaggac cgtagaaaga tacgacactg agaaagatga gtggacgatg gtaagccctt 1620
taccttgtgc ttggcaatgg agtgcagcag ttgtggttca tgactgcatt tatgtgatga 1680
cactgaacct catgtactgt tattttccaa ggtctgactc atgggtagaa atggccatga 1740
gacagactag taggtccttt gcttcagctg cagcttttgg tgataaaatt ttctatattg 1800
gagggttgca tattgctacc aattccggca taagactccc ctctggcact gtagatgggt 1860
cttcagtaac tgtggaaatt tatgatgtga ataaaaatga gtggaaaatg gcagccaaca 1920

tccctgctaa gaggtactct gacccctgtg ttagagctgt tgtgatctca aattctctat 1980
gtgtgtttat gcgagaaacc cacttaaatg agcgagctaa atacgtcacc taccaatatg 2040
acctggaact tgaccggtgg tctctgcggc agcatatatc tgaacgtgta ctgtgggact 2100
tggggagaga ttttcgatgc actgtgggga aactctatcc atcctgcctt gaagagtctc 2160
catggaaacc accaacttat ctttttttcaa cggatgggac agaagagttt gaactggatg 2220
gagaaatggt tgcactacca cctgtatagt ggggaagttc agggagtgca cgcctgagtt 2280
atgtgctttg tcattttctt tgctaaacaa aagaggctat gaaagaacta aatatgagta 2340
cataaaattc tatctttgat aaattttatt tttatgccct acttaatatt tgcatcagta 2400
taatatatat cagtgagtct tacagaaaga tatgcttcca taatatgaaa tagattattc 2460
aataattgag aaactttatg tgtaatcatg agagtataag aatctggatt atctaacatt 2520
gttagccctg tgtatgtaca gttcaaaaag ttcatttata aaagtagttt cctgttc    2577
```

<210> 2
<211> 623
<212> PRT
<213> Homo sapiens

<400> 2

```
Met Ser Thr Gln Asp Glu Arg Gln Ile Asn Thr Glu Tyr Ala Val Ser
 1               5                  10                  15

Leu Leu Glu Gln Leu Lys Leu Phe Tyr Glu Gln Gln Leu Phe Thr Asp
            20                  25                  30

Ile Val Leu Ile Val Glu Gly Thr Glu Phe Pro Cys His Lys Met Val
        35                  40                  45

Leu Ala Thr Cys Ser Ser Tyr Phe Arg Ala Met Phe Met Ser Gly Leu
    50                  55                  60

Ser Glu Ser Lys Gln Thr His Val His Leu Arg Asn Val Asp Ala Ala
65                  70                  75                  80

Thr Leu Gln Ile Ile Ile Thr Tyr Ala Tyr Thr Gly Asn Leu Ala Met
                85                  90                  95

Asn Asp Ser Thr Val Glu Gln Leu Tyr Glu Thr Ala Cys Phe Leu Gln
            100                 105                 110

Val Glu Asp Val Leu Gln Arg Cys Arg Glu Tyr Leu Ile Lys Lys Ile
        115                 120                 125

Asn Ala Glu Asn Cys Val Arg Leu Leu Ser Phe Ala Asp Leu Phe Ser
    130                 135                 140

Cys Glu Glu Leu Lys Gln Ser Ala Lys Arg Met Val Glu His Lys Phe
145                 150                 155                 160

Thr Ala Val Tyr His Gln Asp Ala Phe Met Gln Leu Ser His Asp Leu
                165                 170                 175

Leu Ile Asp Ile Leu Ser Ser Asp Asn Leu Asn Val Glu Lys Glu Glu
            180                 185                 190

Thr Val Arg Glu Ala Ala Met Leu Trp Leu Glu Tyr Asn Thr Glu Ser
        195                 200                 205
```

```
Arg Ser Gln Tyr Leu Ser Ser Val Leu Ser Gln Ile Arg Ile Asp Ala
    210                 215                 220

Leu Ser Glu Val Thr Gln Arg Ala Trp Phe Gln Gly Leu Pro Pro Asn
225                 230                 235                 240

Asp Lys Ser Val Val Val Gln Gly Leu Tyr Lys Ser Met Pro Lys Phe
                245                 250                 255

Phe Lys Pro Arg Leu Gly Met Thr Lys Glu Glu Met Met Ile Phe Ile
            260                 265                 270

Glu Ala Ser Ser Glu Asn Pro Cys Ser Leu Tyr Ser Ser Val Cys Tyr
        275                 280                 285

Ser Pro Gln Ala Glu Lys Val Tyr Lys Leu Cys Ser Pro Pro Ala Asp
    290                 295                 300

Leu His Lys Val Gly Thr Val Val Thr Pro Asp Asn Asp Ile Tyr Ile
305                 310                 315                 320

Ala Gly Gly Gln Val Pro Leu Lys Asn Thr Lys Thr Asn His Ser Lys
                325                 330                 335

Thr Ser Lys Leu Gln Thr Ala Phe Arg Thr Val Asn Cys Phe Tyr Trp
            340                 345                 350

Phe Asp Ala Gln Gln Asn Thr Trp Phe Pro Lys Thr Pro Met Leu Phe
        355                 360                 365

Val Arg Ile Lys Pro Ser Leu Val Cys Cys Glu Gly Tyr Ile Tyr Ala
    370                 375                 380

Ile Gly Gly Asp Ser Val Gly Gly Glu Leu Asn Arg Arg Thr Val Glu
385                 390                 395                 400

Arg Tyr Asp Thr Glu Lys Asp Glu Trp Thr Met Val Ser Pro Leu Pro
                405                 410                 415

Cys Ala Trp Gln Trp Ser Ala Ala Val Val Val His Asp Cys Ile Tyr
            420                 425                 430

Val Met Thr Leu Asn Leu Met Tyr Cys Tyr Phe Pro Arg Ser Asp Ser
        435                 440                 445

Trp Val Glu Met Ala Met Arg Gln Thr Ser Arg Ser Phe Ala Ser Ala
    450                 455                 460

Ala Ala Phe Gly Asp Lys Ile Phe Tyr Ile Gly Gly Leu His Ile Ala
465                 470                 475                 480

Thr Asn Ser Gly Ile Arg Leu Pro Ser Gly Thr Val Asp Gly Ser Ser
                485                 490                 495

Val Thr Val Glu Ile Tyr Asp Val Asn Lys Asn Glu Trp Lys Met Ala
            500                 505                 510
```

Ala Asn Ile Pro Ala Lys Arg Tyr Ser Asp Pro Cys Val Arg Ala Val
515 520 525

Val Ile Ser Asn Ser Leu Cys Val Phe Met Arg Glu Thr His Leu Asn
530 535 540

Glu Arg Ala Lys Tyr Val Thr Tyr Gln Tyr Asp Leu Glu Leu Asp Arg
545 550 555 560

Trp Ser Leu Arg Gln His Ile Ser Glu Arg Val Leu Trp Asp Leu Gly
565 570 575

Arg Asp Phe Arg Cys Thr Val Gly Lys Leu Tyr Pro Ser Cys Leu Glu
580 585 590

Glu Ser Pro Trp Lys Pro Pro Thr Tyr Leu Phe Ser Thr Asp Gly Thr
595 600 605

Glu Glu Phe Glu Leu Asp Gly Glu Met Val Ala Leu Pro Pro Val
610 615 620

<210> 3
<211> 1749
<212> DNA
<213> Homo sapiens
<400> 3

caagggagcg agggtgtcgt agagggcaga atgaacaaga agaattagga gggaggctgc 60
gtgtgccggg gctaggggct ggaagtcctg gctctagttg cacctcggaa ggaaaaggca 120
aacagaggag ggaaggcgtc ttaggactgc ctggatccag agcactttcc tcggcctcta 180
caggcctgtg tcgctatggg ttcccccgcc gccccggagg gagcgctggg ctacgtccgc 240
gagttcactc gccactcctc cgacgtgctg ggcaacctca acgagctgcg cctgcgcggg 300
atcctcactg acgtcacgct gctggttggc gggcaacccc tcagagcaca caaggcagtt 360
ctcatcgcct gcagtggctt cttctattca attttccggg gccgtgcggg agtcggggtg 420
gacgtgctct ctctgcccgg gggtcccgaa gcgagaggct tcgcccctct attggacttc 480
atgtacactt cgcgcctgcg cctctctcca gccactgcac cagcagtcct agcggccgcc 540
acctatttgc agatggagca cgtggtccag gcatgccacc gcttcatcca ggccagctat 600
gaacctctgg gcatctccct gcgccccctg gaagcagaac cccaacaccc cccaacggcc 660
cctccaccag gtagtcccag gcgctccgaa ggacacccag acccacctac tgaatctcga 720
agctgcagtc aaggcccccc cagtccagcc agccctgacc ccaaggcctg caactggaaa 780
aagtacaagt acatcgtgct aaactctcag gcctcccaag cagggagcct ggtcggggag 840
agaagttctg gtcaaccttg cccccaagcc aggctcccca gtggagacga ggcctccagc 900
agcagcagca gcagcagcag cagcagcagt gaagaaggac ccattcctgg tccccagagc 960
aggctctctc caactgctgc cactgtgcag ttcaaatgtg gggctccagc cagtaccccc 1020
tacctcctca catcccaggc tcaagacacc tctggatcac cctctgaacg ggctcgtcca 1080
ctacccggga gtgaattttt cagctgccag aactgtgagg ctgtggcagg gtgctcatcg 1140
gggctggact ccttggttcc tggggacgaa gacaaaccct ataagtgtca gctgtgccgg 1200
tcttcgttcc gctacaaggg caaccttgcc agtcaccgta cagtgcacac aggggaaaag 1260
ccttaccact gctcaatctg cggagcccgt tttaaccggc cagcaaacct gaaaacgcac 1320
agccgcatcc attcgggaga gaagccgtat aagtgtgaga cgtgcggctc gcgctttgta 1380
caggtacgga gccagcctcc aagtggcttc caaggcaaac ctgcaagagg tggggtgggc 1440
caaaagggag ggttctgttc ctcccagagg caggacttga agtctcctcc ctcccaggtg 1500
gcacatctgc gggcgcacgt gctgatccac accggggaga agccctaccc ttgccctacc 1560
tgcggaaccc gcttccgcca cctgcagacc ctcaagagcc acgttcgcat ccacaccgga 1620
gagaagcctt accactgcga cccctgtggc ctgcatttcc ggcacaagag tcaactgcgg 1680
ctgcatctgc gccagaaaca cggagctgct accaacacca aagtgcacta ccacattctc 1740
gggggggccc 1749

<210> 4
<211> 518
<212> PRT
<213> Homo sapiens
<400> 4

```
Met Gly Ser Pro Ala Ala Pro Glu Gly Ala Leu Gly Tyr Val Arg Glu
1               5                   10                  15

Phe Thr Arg His Ser Ser Asp Val Leu Gly Asn Leu Asn Glu Leu Arg
            20                  25                  30

Leu Arg Gly Ile Leu Thr Asp Val Thr Leu Leu Val Gly Gly Gln Pro
        35                  40                  45

Leu Arg Ala His Lys Ala Val Leu Ile Ala Cys Ser Gly Phe Phe Tyr
    50                  55                  60

Ser Ile Phe Arg Gly Arg Ala Gly Val Gly Val Asp Val Leu Ser Leu
65                  70                  75                  80

Pro Gly Gly Pro Glu Ala Arg Gly Phe Ala Pro Leu Leu Asp Phe Met
                85                  90                  95

Tyr Thr Ser Arg Leu Arg Leu Ser Pro Ala Thr Ala Pro Ala Val Leu
            100                 105                 110

Ala Ala Ala Thr Tyr Leu Gln Met Glu His Val Val Gln Ala Cys His
        115                 120                 125

Arg Phe Ile Gln Ala Ser Tyr Glu Pro Leu Gly Ile Ser Leu Arg Pro
    130                 135                 140

Leu Glu Ala Glu Pro Pro Thr Pro Pro Thr Ala Pro Pro Pro Gly Ser
145                 150                 155                 160

Pro Arg Arg Ser Glu Gly His Pro Asp Pro Pro Thr Glu Ser Arg Ser
                165                 170                 175

Cys Ser Gln Gly Pro Pro Ser Pro Ala Ser Pro Asp Pro Lys Ala Cys
            180                 185                 190

Asn Trp Lys Lys Tyr Lys Tyr Ile Val Leu Asn Ser Gln Ala Ser Gln
        195                 200                 205

Ala Gly Ser Leu Val Gly Glu Arg Ser Ser Gly Gln Pro Cys Pro Gln
    210                 215                 220

Ala Arg Leu Pro Ser Gly Asp Glu Ala Ser Ser Ser Ser Ser Ser Ser
225                 230                 235                 240

Ser Ser Ser Ser Ser Glu Glu Gly Pro Ile Pro Gly Pro Gln Ser Arg
                245                 250                 255

Leu Ser Pro Thr Ala Ala Thr Val Gln Phe Lys Cys Gly Ala Pro Ala
            260                 265                 270
```

```
Ser Thr Pro Tyr Leu Leu Thr Ser Gln Ala Gln Asp Thr Ser Gly Ser
        275                 280                 285

Pro Ser Glu Arg Ala Arg Pro Leu Pro Gly Ser Glu Phe Phe Ser Cys
    290                 295                 300

Gln Asn Cys Glu Ala Val Ala Gly Cys Ser Ser Gly Leu Asp Ser Leu
305                 310                 315                 320

Val Pro Gly Asp Glu Asp Lys Pro Tyr Lys Cys Gln Leu Cys Arg Ser
                325                 330                 335

Ser Phe Arg Tyr Lys Gly Asn Leu Ala Ser His Arg Thr Val His Thr
            340                 345                 350

Gly Glu Lys Pro Tyr His Cys Ser Ile Cys Gly Ala Arg Phe Asn Arg
        355                 360                 365

Pro Ala Asn Leu Lys Thr His Ser Arg Ile His Ser Gly Glu Lys Pro
    370                 375                 380

Tyr Lys Cys Glu Thr Cys Gly Ser Arg Phe Val Gln Val Arg Ser Gln
385                 390                 395                 400

Pro Pro Ser Gly Phe Gln Gly Lys Pro Ala Arg Gly Gly Val Gly Gln
                405                 410                 415

Lys Gly Gly Phe Cys Ser Ser Gln Arg Gln Asp Leu Lys Ser Pro Pro
            420                 425                 430

Ser Gln Val Ala His Leu Arg Ala His Val Leu Ile His Thr Gly Glu
        435                 440                 445

Lys Pro Tyr Pro Cys Pro Thr Cys Gly Thr Arg Phe Arg His Leu Gln
    450                 455                 460

Thr Leu Lys Ser His Val Arg Ile His Thr Gly Glu Lys Pro Tyr His
465                 470                 475                 480

Cys Asp Pro Cys Gly Leu His Phe Arg His Lys Ser Gln Leu Arg Leu
                485                 490                 495

His Leu Arg Gln Lys His Gly Ala Ala Thr Asn Thr Lys Val His Tyr
            500                 505                 510

His Ile Leu Gly Gly Pro
        515
```

<210> 5
<211> 3501
<212> DNA
<213> Homo sapiens
<220>
<221> modified_base
<222> (3070)
<223> a, t, c, g, other or unknown
<220>

<221> modified_base
<222> (3087)
<223> a, t, c, g, other or unknown
<400> 5

```
tctttttcct cgcgtccttt gccccggaag tgctcttaca acattggctg ccggcgtgac 60
tttgaccgct tcccggtgcg ttaccggcag ctgaacccac ccggcgccac gggactttga 120
cgcgtgctct gcgcttgcca tgagactcct gggagctgca gccgtcgcgg ctctggggcg 180
cggaagggcc cccgcctccc taggctggca gaggaagcag gttaattgga aggcctgccg 240
atggtcttca tcaggggtga ttcctaatga aaaaatacga aatattggaa tctcagctca 300
cattgattct gggaaaacta cattaacaga acgagtcctt tactacactg gcagaattgc 360
aaagatgcat gaggtgaaag gtaaagatgg agttggtgct gtcatggatt ccatggaact 420
agagagacaa agaggaatca ctattcagtc agcagccact ttcaccatgt ggaaagatgt 480
caatattaac attatagata ctcctgggca tgtggacttc acaatagaag tggaaagggc 540
cctgagagtg ttggatggtg cagtccttgt tctctgtgct gttggagggg tacagtgcca 600
gaccatgact gtcaatcgtc agatgaagcg ctacaacgtt ccgtttctaa cttttattaa 660
caaattggac cgaatgggct ccaacccagc cagggccctg cagcaaatga ggtctaaact 720
aaatcataat acagcgttta tgcagatacc catgggtttg gagggtaatt ttaaaggtat 780
tgtagatctt attgaggaac gagccatcta ttttgatgga gactttagtc agattgttcg 840
atatggtgag attccagctg aattaagggc ggcggccact gaccaccggc aggagctaat 900
tgaatgtgtt gccaattcag atgaacagct tggtgagatg tttctggaag aaaaaatccc 960
ctcgatttct gatttaaagc tagcaattcg aagagctact ctgaaaagat catttactcc 1020
tgtatttttg ggaagcgcct tgaagaacaa aggagttcag cctcttttag atgctgtttt 1080
agaatacctc ccaaatccat ctgaagtcca gaactatgct attctcaata aaaaggatga 1140
ctcaaaagag aaaaccaaaa tcctaatgaa ctccagtaga cacaattccc acccatttgt 1200
aggcctggct ttcccctgg aggtaggtcg atttggacaa ttaacttatg ttcgcagtta 1260
tcagggagag ctaaagaagg gtgacaccat ctataacaca aggacaagaa agaaagtacg 1320
gttgcaacgg ctggctcgca tgcatgccga catgatggag gcaagtacag aggaagtata 1380
tgccggagac atctgtgcat tgtttggcat tgactgtgct agtggagaca cattcacaga 1440
caaagccaac agcggccttt ctatggagtc aattcatgtt cctgatcctg tcatttcaat 1500
agcaatgaag ccttctaaca agaacgatct ggaaaaattt tcaaaaggta ttggcaggtt 1560
tacaagagaa gatcccacat ttaaagtata ctttgacact gagaacaaag agacagttat 1620
atctggaatg ggagaattac acctggaaat ctatgctcag aggctggaaa gagagtatgg 1680
ctgtccttgt atcacaggaa agccaaaagt tgcctttcga gagaccatta ctgcccctgt 1740
cccgtttgac tttacacata aaaaacaatc aggtggtgca ggccagtatg gaaaagtaat 1800
aggtgtcctg gagcctctgg acccagagga ctacactaaa ttggaatttt cagatgaaac 1860
attcggatca aatattccaa agcagtttgt gcctgctgta gaaaaggggt ttttagatgc 1920
ctgcgagaag ggccctcttt ctggtcacaa gctctctggg ctccggtttg tcctgcaaga 1980
tggagcacac cacatggttg attctaatga aatctctttc atccgagcag gagaaggtgc 2040
tcttaaacaa gccttggcaa atgcaacatt atgtattctt gaacctatta tggctgtgga 2100
agttgtagct ccaaatgaat ttcagggaca agtaattgca ggaattaacc gacgccatgg 2160
ggtaatcact gggcaagatg gagttgagga ctattttaca ctgtatgcag atgtccctct 2220
aaatgatatg tttggttatt ccactgaact taggtcatgc acagagggaa agggagaata 2280
cacaatggag tatagcaggt atcagccatg tttaccatcc acacaagaag acgtcattaa 2340
taagtatttg gaagctacag gtcaacttcc tgttaaaaaa ggaaaagcca agaactaact 2400
ttgcttactg tgagttgact gactctaatt gaatctgcgt ggttttgata ctttgatgga 2460
ttccagtgga ataaattcag gctgctgaaa caagaaattc tgagcccagg aagcgggctc 2520
ttctttcttc aaaagaagcc cttcttgttc atattcagga gcttctgtta tattcaaagg 2580
taattctatg tctatctcaa ctctattgat tggttttata gttcattgaa aatcctcaaa 2640
taaaatataa ttattactga aatatgttta atatttaagg ggaaaagaga ctaatttcag 2700
ttatactttt aagcttagaa tgtatgttca tttccaaatt ttgtatcata agagttttca 2760
acatagagaa aagctgaaaa aatgcaaaga ataaccacat actttccatc taccttcctt 2820
tgttaacggg ttgtttatca tataataatt tgttttgtca tatttgcttt cactgtctat 2880
tatctgttta agtctcataa ctctattttt agtttgctga agacttgaaa gtgaatcgca 2940
tatatcatga cacttcttgg agtgtcatta atgggcaggc ttttctgttg aagagtggat 3000
```

```
tccgtatgtt cttcatagag agtgtttttc agattcttca ttgggatatt aaaatattag 3060
ccaaatttcn ctctgtttta tatatgncag tttatttcag tttgtggttt ctgcaaattt 3120
gtaactgcct ctgttttagg agtataagta ttacttcctt gtggtctatt gtgaagtaaa 3180
aagtagaccc ttgcatatac tattcttgtt tgtgttcatc ttaatgtttt tgtacagcta 3240
aatcaaatgt aatttataga gttagtttca tcaacctaat gaatgctagt taaatttgaa 3300
ttccttggaa tttatcgtat attgtattca ctgagattat gaagggacaa atgttaatct 3360
tttgtttcca gaaaaagttg ggctttccca agcagttcta ttacccggtt cagaattgct 3420
tcatccaaaa atcatctgat ggtatagatg gatcctagtc cttttcatta cctgatggta 3480
gaaataaaat aattgatttt a                                           3501
```

<210> 6
<211> 752
<212> PRT
<213> Homo sapiens
<400> 6

```
Met Arg Leu Leu Gly Ala Ala Ala Val Ala Ala Leu Gly Arg Gly Arg
 1               5                  10                  15

Ala Pro Ala Ser Leu Gly Trp Gln Arg Lys Gln Val Asn Trp Lys Ala
            20                  25                  30

Cys Arg Trp Ser Ser Ser Gly Val Ile Pro Asn Glu Lys Ile Arg Asn
        35                  40                  45

Ile Gly Ile Ser Ala His Ile Asp Ser Gly Lys Thr Thr Leu Thr Glu
    50                  55                  60

Arg Val Leu Tyr Tyr Thr Gly Arg Ile Ala Lys Met His Glu Val Lys
65                  70                  75                  80

Gly Lys Asp Gly Val Gly Ala Val Met Asp Ser Met Glu Leu Glu Arg
                85                  90                  95

Gln Arg Gly Ile Thr Ile Gln Ser Ala Ala Thr Phe Thr Met Trp Lys
            100                 105                 110

Asp Val Asn Ile Asn Ile Ile Asp Thr Pro Gly His Val Asp Phe Thr
        115                 120                 125

Ile Glu Val Glu Arg Ala Leu Arg Val Leu Asp Gly Ala Val Leu Val
    130                 135                 140

Leu Cys Ala Val Gly Gly Val Gln Cys Gln Thr Met Thr Val Asn Arg
145                 150                 155                 160

Gln Met Lys Arg Tyr Asn Val Pro Phe Leu Thr Phe Ile Asn Lys Leu
                165                 170                 175

Asp Arg Met Gly Ser Asn Pro Ala Arg Ala Leu Gln Gln Met Arg Ser
            180                 185                 190

Lys Leu Asn His Asn Thr Ala Phe Met Gln Ile Pro Met Gly Leu Glu
        195                 200                 205

Gly Asn Phe Lys Gly Ile Val Asp Leu Ile Glu Glu Arg Ala Ile Tyr
    210                 215                 220
```

```
Phe Asp Gly Asp Phe Ser Gln Ile Val Arg Tyr Gly Glu Ile Pro Ala
225                 230                 235                 240

Glu Leu Arg Ala Ala Ala Thr Asp His Arg Gln Glu Leu Ile Glu Cys
                245                 250                 255

Val Ala Asn Ser Asp Glu Gln Leu Gly Glu Met Phe Leu Glu Glu Lys
            260                 265                 270

Ile Pro Ser Ile Ser Asp Leu Lys Leu Ala Ile Arg Arg Ala Thr Leu
        275                 280                 285

Lys Arg Ser Phe Thr Pro Val Phe Leu Gly Ser Ala Leu Lys Asn Lys
    290                 295                 300

Gly Val Gln Pro Leu Leu Asp Ala Val Leu Glu Tyr Leu Pro Asn Pro
305                 310                 315                 320

Ser Glu Val Gln Asn Tyr Ala Ile Leu Asn Lys Lys Asp Asp Ser Lys
                325                 330                 335

Glu Lys Thr Lys Ile Leu Met Asn Ser Ser Arg His Asn Ser His Pro
            340                 345                 350

Phe Val Gly Leu Ala Phe Pro Leu Glu Val Gly Arg Phe Gly Gln Leu
        355                 360                 365

Thr Tyr Val Arg Ser Tyr Gln Gly Glu Leu Lys Lys Gly Asp Thr Ile
    370                 375                 380

Tyr Asn Thr Arg Thr Arg Lys Lys Val Arg Leu Gln Arg Leu Ala Arg
385                 390                 395                 400

Met His Ala Asp Met Met Glu Ala Ser Thr Glu Glu Val Tyr Ala Gly
                405                 410                 415

Asp Ile Cys Ala Leu Phe Gly Ile Asp Cys Ala Ser Gly Asp Thr Phe
            420                 425                 430

Thr Asp Lys Ala Asn Ser Gly Leu Ser Met Glu Ser Ile His Val Pro
        435                 440                 445

Asp Pro Val Ile Ser Ile Ala Met Lys Pro Ser Asn Lys Asn Asp Leu
    450                 455                 460

Glu Lys Phe Ser Lys Gly Ile Gly Arg Phe Thr Arg Glu Asp Pro Thr
465                 470                 475                 480

Phe Lys Val Tyr Phe Asp Thr Glu Asn Lys Glu Thr Val Ile Ser Gly
                485                 490                 495

Met Gly Glu Leu His Leu Glu Ile Tyr Ala Gln Arg Leu Glu Arg Glu
            500                 505                 510

Tyr Gly Cys Pro Cys Ile Thr Gly Lys Pro Lys Val Ala Phe Arg Glu
        515                 520                 525
```

```
Thr Ile Thr Ala Pro Val Pro Phe Asp Phe Thr His Lys Lys Gln Ser
    530                 535                 540

Gly Gly Ala Gly Gln Tyr Gly Lys Val Ile Gly Val Leu Glu Pro Leu
545                 550                 555                 560

Asp Pro Glu Asp Tyr Thr Lys Leu Glu Phe Ser Asp Glu Thr Phe Gly
                565                 570                 575

Ser Asn Ile Pro Lys Gln Phe Val Pro Ala Val Glu Lys Gly Phe Leu
            580                 585                 590

Asp Ala Cys Glu Lys Gly Pro Leu Ser Gly His Lys Leu Ser Gly Leu
        595                 600                 605

Arg Phe Val Leu Gln Asp Gly Ala His His Met Val Asp Ser Asn Glu
    610                 615                 620

Ile Ser Phe Ile Arg Ala Gly Glu Gly Ala Leu Lys Gln Ala Leu Ala
625                 630                 635                 640

Asn Ala Thr Leu Cys Ile Leu Glu Pro Ile Met Ala Val Glu Val Val
                645                 650                 655

Ala Pro Asn Glu Phe Gln Gly Gln Val Ile Ala Gly Ile Asn Arg Arg
            660                 665                 670

His Gly Val Ile Thr Gly Gln Asp Gly Val Glu Asp Tyr Phe Thr Leu
        675                 680                 685

Tyr Ala Asp Val Pro Leu Asn Asp Met Phe Gly Tyr Ser Thr Glu Leu
    690                 695                 700

Arg Ser Cys Thr Glu Gly Lys Gly Glu Tyr Thr Met Glu Tyr Ser Arg
705                 710                 715                 720

Tyr Gln Pro Cys Leu Pro Ser Thr Gln Glu Asp Val Ile Asn Lys Tyr
                725                 730                 735

Leu Glu Ala Thr Gly Gln Leu Pro Val Lys Lys Gly Lys Ala Lys Asn
            740                 745                 750
```

<210> 7
<211> 7506
<212> DNA
<213> Homo sapiens
<400> 7

```
gccgcgggag caggcggagg cggaggcggc gggggcagga ggatgtcgca gccgccgctg 60
ctccccgcct cggcggagac tcggaagttc acccgggcgc tgagtaagcc gggcacggcg 120
gccgagctgc ggcagagcgt gtctgaggtg gtgcgcggct ccgtgctcct ggcaaagcca 180
aagctaattg agccactcga ctatgaaaat gtcatcgtcc agaagaagac tcagatcctg 240
aacgactgtt tacgggagat gctgctcttc ccttacgatg actttcagac ggccatcctg 300
agacgacagg gtcgatacat atgctcaaca gtgcctgcga aggcggaaga ggaagcacag 360
agcttgtttg ttacagagtg catcaaaacc tataactctg actggcatct tgtgaactat 420
aaatatgaag attactcagg agagtttcga cagcttccga acaaagtggt caagttggat 480
aaacttccag ttcatgtcta tgaagttgac gaggaggtcg acaaagatga ggatgctgcc 540
```

tcccttggtt cccagaaagg tgggatcacc aagcatggct ggctgtacaa aggcaacatg 600
aacagtgcca tcagcgtgac catgaggtca tttaagagac gatttttcca cctgattcaa 660
cttggcgatg gatcctataa atttgaattt ttaaaagatc tccaaaagga accaaaagga 720
tcaatatttc tgggattcct gtatggggtg tcgttcagga acaacaaagt caggcgtttt 780
gcttttgagc tcaagatgca ggacaaaagt agttatctct tggcagcaga cagtgaagtg 840
gaaatggaag aatggatcac aattctaaat aagatcctcc agctcaactt tgaagctgca 900
atgcaagaaa agcgaaatgg cgactctcac gaagatgatg aacaaagcaa attggaaggt 960
tctggttccg gtttagatag ctacctgccg gaacttgcca agagtgcaag agaagcagaa 1020
atcaaactga aaagtgaaag cagagtcaaa ctttttttatt tggacccaga tgcccagaag 1080
cttgacttct catcagctga gccagaagtg aagtcatttg aagagaagtt tggaaaaagg 1140
atccttgtca agtgcaatga tttatctttc aatttgcaat gctgtgttgc cgaaaatgaa 1200
gaaggaccca ctacaaatgt tgaacctttc tttgttactc tatccctgtt tgacataaaa 1260
tacaaccgga agatttctgc cgatttccac gtagacctga accatttctc agtgaggcaa 1320
atgatcgcca ccacgtcccc ggcgctgatg aatggcagtg ggccggaaac ccaatctgcc 1380
ctcaggggca tccttcatga agccgccatg cagtatccga agcagggaat attttcagtc 1440
acttgtcctc atccagatat atttcttgtg gccagaattg aaaaagtcct tcaggggagc 1500
atcacacatt gcgctgagcc atatatgaaa agttcagact cttctaaggt ggcccagaag 1560
gtgctgaaga atgccaagca ggcatgccaa agactaggac agtatagaat gccatttgct 1620
tgggcagcaa ggacattgtt taaggatgca tctggaaatc ttgacaaaaa tgccagattt 1680
tctgccatct acaggcaaga cagcaataag ctatccaatg atgacatgct caagttactt 1740
gcagactttc ggaaacctga gaagatggct aagctcccag tgattttagg caatctagac 1800
attacaattg ataatgtttc ctcagacttc cctaattatg ttaattcatc atacattccc 1860
acaaaacaat ttgaaacctg cagtaaaact cccatcacgt ttgaagtgga ggaatttgtg 1920
ccctgcatac caaaacacac tcagccttac accatctaca ccaatcacct ttacgtttat 1980
cctaagtact tgaaatacga cagtcagaag tcttttgcca aggctagaaa tattgcgatt 2040
tgcattgaat tcaaagattc agatgaggaa gactctcagc cccttaagtg catttatggc 2100
agacctggtg ggccagtttt cacaagaagc gcctttgctg cagttttaca ccatcaccaa 2160
aacccagaat tttatgatga gattaaaata gagttgccca ctcagctgca tgaaaagcac 2220
cacctgttgc tcacattctt ccatgtcagc tgtgacaact caagtaaagg aagcacgaag 2280
aagagggatg tcgttgaaac ccaagttggc tactcctggc ttcccctcct gaaagacgga 2340
agggtggtga caagcgagca gcacatcccg gtctcggcga accttccttc gggctatctt 2400
ggctaccagg agcttgggat gggcaggcat tatggtccgg aaattaaatg ggtagatgga 2460
ggcaagccac tgctgaaaat ttccactcat ctggtttcta cagtgtatac tcaggatcag 2520
catttacata attttttcca gtactgtcag aaaaccgaat ctggagccca agccttagga 2580
aacgaacttg taaagtacct taagagtctg catgcgatgg aaggccacgt gatgatcgcc 2640
ttcttgccca ctatcctaaa ccagctgttc cgagtcctca ccagagccac acaggaagaa 2700
gtcgcggtta acgtgactcg ggtcattatt catgtggttg cccagtgcca tgaggaagga 2760
ttggagagcc acttgaggtc atatgttaag tacgcgtata aggctgagcc atatgttgcc 2820
tctgaataca agacagtgca tgaagaactg accaaatcca tgaccacgat tctcaagcct 2880
tctgccgatt tcctcaccag caacaaacta ctgaagtact catggttttt ctttgatgta 2940
ctgatcaaat ctatggctca gcatttgata gagaactcca aagttaagtt gctgcgaaac 3000
cagagatttc ctgcatccta tcatcatgca gtggaaaccg ttgtaaatat gctgatgcca 3060
cacatcactc agaagtttcg agataatcca gaggcatcta agaacgcgaa tcatagcctt 3120
gctgtcttca tcaagagatg tttcaccttc atggacaggg gctttgtctt caagcagatc 3180
aacaactaca ttagctgttt tgctcctgga gacccaaaga ccctctttga atacaagttt 3240
gaatttctcc gtgtagtgtg caaccatgaa cattatattc cgttgaactt accaatgcca 3300
tttggaaaag gcaggattca aagataccaa gacctccagc ttgactactc attaacagat 3360
gagttctgca gaaaccactt cttggtggga ctgttactga gggaggtggg gacagccctc 3420
caggagttcc gggaggtccg tctgatcgcc atcagtgtgc tcaagaacct gctgataaag 3480
cattcttttg atgacagata tgcttcaagg agccatcagg caaggatagc caccctctac 3540
ctgcctctgt ttggtctgct gattgaaaac gtccagcgga tcaatgtgag ggatgtgtca 3600
cccttccctg tgaacgcggg catgactgtg aaggatgaat ccctggctct accagctgtg 3660
aatccgctgg tgacgccgca gaagggaagc accctggaca acagcctgca caaggacctg 3720
ctgggcgcca tctccggcat tgcttctcca tatacaacct caactccaaa catcaacagt 3780
gtgagaaatg ctgattcgag aggatctctc ataagcacag attcgggtaa cagccttcca 3840
gaaaggaata gtgagaagag caattccctg gataagcacc aacaaagtag cacattggga 3900
aattccgtgg ttcgctgtga taaacttgac cagtctgaga ttaagagcct actgatgtgt 3960
ttcctctaca tcttaaagag catgtctgat gatgctttgt ttacatattg gaacaaggct 4020

tcaacatctg aacttatgga tttttttaca atatctgaag tctgcctgca ccagttccag 4080
tacatgggga agcgatacat agccagaaca ggaatgatgc atgccagatt gcagcagctg 4140
ggcagcctgg ataactctct cacttttaac cacagctatg gccactcgga cgcagatgtt 4200
ctgcaccagt cattacttga agccaacatt gctactgagg tttgcctgac agctctggac 4260
acgctttctc tatttacatt ggcgtttaag aaccagctcc tggccgacca tggacataat 4320
cctctcatga aaaaagtttt tgatgtctac ctgtgttttc ttcaaaaaca tcagtctgaa 4380
acggctttaa aaaatgtctt cactgcctta aggtccttaa tttataagtt tccctcaaca 4440
ttctatgaag ggagagcgga catgtgtgcg gctctgtgtt acgagattct caagtgctgt 4500
aactccaagc tgagctccat caggacggag gcctcccagc tgctctactt cctgatgagg 4560
aacaactttg attacactgg aaagaagtcc tttgtccgga cacatttgca agtcatcata 4620
tctgtcagcc agctgatagc agacgttgtt ggcattgggg gaaccagatt ccagcagtcc 4680
ctgtccatca tcaacaactg tgccaacagt gaccggctta ttaagcacac cagcttctcc 4740
tctgatgtga aggacttaac caaaaggata cgcacggtgc taatggccac cgcccagatg 4800
aaggagcatg agaacgaccc agagatgctg gtggacctcc agtacagcct ggccaaatcc 4860
tatgccagca cgcccgagct caggaagacg tggctcgaca gcatggccag gatccatgtc 4920
aaaaatggcg atctctcaga ggcagcaatg tgctatgtcc acgtaacagc cctagtggca 4980
gaatatctca cacggaaaga agcagtccag tgggagccgc cccttctccc ccacagccat 5040
agcgcctgcc tgaggaggag ccggggaggc gtgtttagac aaggatgcac cgccttcagg 5100
gtcattaccc caaacatcga cgaggaggcc tccatgatgg aagacgtggg gatgcaggat 5160
gtccatttca acgaggatgt gctgatggag ctccttgagc agtgcgcaga tggactctgg 5220
aaagccgagc gctacgagct cattgccgac atctacaaac ttatcatccc catttatgag 5280
aagcggaggg attttgagag gctggcccat ctgtatgaca cgctgcaccg ggcctacagc 5340
aaagtgaccg aggtcatgca ctcgggccgc aggcttctgg ggacctactt ccgggtagcc 5400
ttcttcgggc aggcagcgca ataccagttt acagacagtg aaacagatgt ggagggattc 5460
tttgaagatg aagatggaaa ggagtatatt tacaaggaac ccaaactcac accgctgtcg 5520
gaaatttctc agagactcct taaactgtac tcggataaat ttggttctga aaatgtcaaa 5580
atgatacagg attctggcaa ggtcaaccct aaggatctgg attctaagta tgcctacatc 5640
caggtgactc acgtcatccc cttctttgac gaaaaagagt tgcaagaaag gaaaacagag 5700
tttgagagat cccacaacat ccgccgcttc atgtttgaga tgccatttac gcagaccggg 5760
aagaggcagg gcggggtgga agagcagtgc aaacggcgca ccatcctgac agccatacac 5820
tgcttccctt atgtgaagaa gcgcatccct gtcatgtacc agcaccacac tgacctgaac 5880
cccatcgagg tggccattga cgagatgagt aagaaggtgg cggagctccg gcagctgtgc 5940
tcctcggccg aggtggacat gatcaaactg cagctcaaac tccagggcag cgtgagtgtt 6000
caggtcaatg ctggcccact agcatatgcg cgagctttct tagatgatac aaacacaaag 6060
cgatatcctg acaataaagt gaagctgctt aaggaagttt tcaggcaatt tgtggaagct 6120
tgcggtcaag ccttagcggt aaacgaacgt ctgattaaag aagaccagct cgagtatcag 6180
gaagaaatga aagccaacta cagggaaatg gcgaaggagc tttctgaaat catgcatgag 6240
cagatctgcc ccctggagga gaagacgagc gtcttaccga attcccttca catcttcaac 6300
gccatcagtg ggactccaac aagcacaatg gttcacggga tgaccagctc gtcttcggtc 6360
gtgtgattac atctcatggc ccgtgtgtgg ggacttgctt tgtcatttgc aaactcagga 6420
tgctttccaa agccaatcac tggggagacc gagcacaggg aggaccaagg ggaaggggag 6480
agaaaggaaa taaagaacaa cgttatttct taacagactt tctataggag ttgtaagaag 6540
gtgcacatat tttttaaat ctcactggca atattcaaag ttttcattgt gtcttaacaa 6600
aggtgtggta gacactcttg agctggactt agattttatt cttccttgca gagtagtgtt 6660
agaatagatg gcctacagaa aaaaaaggtt ctgggatcta catggcaggg agggctgcac 6720
tgacattgat gcctggggga ccttttgcct cgaggctgag ctggaaaatc ttgaaaatat 6780
tttttttttc ctgtggcaca ttcaggttga atacaagaac tatttttgtg actagttttt 6840
gatgacctaa gggaactgac cattgtaatt tttgtaccag tgaaccagga gatttagtgc 6900
ttttatattc atttccttgc atttaagaaa atatgaaagc ttaaggaatt atgtgagctt 6960
aaaactagtc aagcagttta gaaccaaagg cctatattaa taaccgcaac tatgctgaaa 7020
agtacaaagt agtacagtat attgttatgt acatatcatt gttaatacag tcctggcatt 7080
ctgtacatat atgtattaca tttctacatt tttaatactc acatgggctt atgcattaag 7140
tttaattgtg ataaatttgt gctgttccag tatatgcaat acactttaat gttttattct 7200
tgtacataaa aatgtgcaat atggagatgt atacagtctt tactatatta ggtttataaa 7260
cagttttaag aatttcatcc ttttgccaaa atggtggagt atgtaattgg taaatcataa 7320
atcctgtggt gaatggtggt gtactttaaa gctgtcacca tgttatattt tcttttaaga 7380
ctttaattta gtaattttat atttgggaaa ataaaggttt ttaattttat ttaactggaa 7440
tcactgccct gctgtaatta aacattctgt accacatctg tattaaaaag acattgctga 7500

ccatta 7506

<210> 8
<211> 2107
<212> PRT
<213> Homo sapiens
<400> 8

Met Ser Gln Pro Pro Leu Leu Pro Ala Ser Ala Glu Thr Arg Lys Phe
1 5 10 15

Thr Arg Ala Leu Ser Lys Pro Gly Thr Ala Ala Glu Leu Arg Gln Ser
20 25 30

Val Ser Glu Val Val Arg Gly Ser Val Leu Leu Ala Lys Pro Lys Leu
35 40 45

Ile Glu Pro Leu Asp Tyr Glu Asn Val Ile Val Gln Lys Lys Thr Gln
50 55 60

Ile Leu Asn Asp Cys Leu Arg Glu Met Leu Leu Phe Pro Tyr Asp Asp
65 70 75 80

Phe Gln Thr Ala Ile Leu Arg Arg Gln Gly Arg Tyr Ile Cys Ser Thr
85 90 95

Val Pro Ala Lys Ala Glu Glu Glu Ala Gln Ser Leu Phe Val Thr Glu
100 105 110

Cys Ile Lys Thr Tyr Asn Ser Asp Trp His Leu Val Asn Tyr Lys Tyr
115 120 125

Glu Asp Tyr Ser Gly Glu Phe Arg Gln Leu Pro Asn Lys Val Val Lys
130 135 140

Leu Asp Lys Leu Pro Val His Val Tyr Glu Val Asp Glu Glu Val Asp
145 150 155 160

Lys Asp Glu Asp Ala Ala Ser Leu Gly Ser Gln Lys Gly Gly Ile Thr
165 170 175

Lys His Gly Trp Leu Tyr Lys Gly Asn Met Asn Ser Ala Ile Ser Val
180 185 190

Thr Met Arg Ser Phe Lys Arg Arg Phe Phe His Leu Ile Gln Leu Gly
195 200 205

Asp Gly Ser Tyr Lys Phe Glu Phe Leu Lys Asp Leu Gln Lys Glu Pro
210 215 220

Lys Gly Ser Ile Phe Leu Gly Phe Leu Tyr Gly Val Ser Phe Arg Asn
225 230 235 240

Asn Lys Val Arg Arg Phe Ala Phe Glu Leu Lys Met Gln Asp Lys Ser
245 250 255

Ser Tyr Leu Leu Ala Ala Asp Ser Glu Val Glu Met Glu Glu Trp Ile
260 265 270

Thr Ile Leu Asn Lys Ile Leu Gln Leu Asn Phe Glu Ala Ala Met Gln
275 280 285

Glu Lys Arg Asn Gly Asp Ser His Glu Asp Asp Glu Gln Ser Lys Leu
290 295 300

Glu Gly Ser Gly Ser Gly Leu Asp Ser Tyr Leu Pro Glu Leu Ala Lys
305 310 315 320

Ser Ala Arg Glu Ala Glu Ile Lys Leu Lys Ser Glu Ser Arg Val Lys
325 330 335

Leu Phe Tyr Leu Asp Pro Asp Ala Gln Lys Leu Asp Phe Ser Ser Ala
340 345 350

Glu Pro Glu Val Lys Ser Phe Glu Glu Lys Phe Gly Lys Arg Ile Leu
355 360 365

Val Lys Cys Asn Asp Leu Ser Phe Asn Leu Gln Cys Cys Val Ala Glu
370 375 380

Asn Glu Glu Gly Pro Thr Thr Asn Val Glu Pro Phe Phe Val Thr Leu
385 390 395 400

Ser Leu Phe Asp Ile Lys Tyr Asn Arg Lys Ile Ser Ala Asp Phe His
405 410 415

Val Asp Leu Asn His Phe Ser Val Arg Gln Met Ile Ala Thr Thr Ser
420 425 430

Pro Ala Leu Met Asn Gly Ser Gly Pro Glu Thr Gln Ser Ala Leu Arg
435 440 445

Gly Ile Leu His Glu Ala Ala Met Gln Tyr Pro Lys Gln Gly Ile Phe
450 455 460

Ser Val Thr Cys Pro His Pro Asp Ile Phe Leu Val Ala Arg Ile Glu
465 470 475 480

Lys Val Leu Gln Gly Ser Ile Thr His Cys Ala Glu Pro Tyr Met Lys
485 490 495

Ser Ser Asp Ser Ser Lys Val Ala Gln Lys Val Leu Lys Asn Ala Lys
500 505 510

Gln Ala Cys Gln Arg Leu Gly Gln Tyr Arg Met Pro Phe Ala Trp Ala
515 520 525

Ala Arg Thr Leu Phe Lys Asp Ala Ser Gly Asn Leu Asp Lys Asn Ala
530 535 540

Arg Phe Ser Ala Ile Tyr Arg Gln Asp Ser Asn Lys Leu Ser Asn Asp
545 550 555 560

```
Asp Met Leu Lys Leu Leu Ala Asp Phe Arg Lys Pro Glu Lys Met Ala
                565                 570                 575

Lys Leu Pro Val Ile Leu Gly Asn Leu Asp Ile Thr Ile Asp Asn Val
            580                 585                 590

Ser Ser Asp Phe Pro Asn Tyr Val Asn Ser Ser Tyr Ile Pro Thr Lys
        595                 600                 605

Gln Phe Glu Thr Cys Ser Lys Thr Pro Ile Thr Phe Glu Val Glu Glu
    610                 615                 620

Phe Val Pro Cys Ile Pro Lys His Thr Gln Pro Tyr Thr Ile Tyr Thr
625                 630                 635                 640

Asn His Leu Tyr Val Tyr Pro Lys Tyr Leu Lys Tyr Asp Ser Gln Lys
                645                 650                 655

Ser Phe Ala Lys Ala Arg Asn Ile Ala Ile Cys Ile Glu Phe Lys Asp
            660                 665                 670

Ser Asp Glu Glu Asp Ser Gln Pro Leu Lys Cys Ile Tyr Gly Arg Pro
        675                 680                 685

Gly Gly Pro Val Phe Thr Arg Ser Ala Phe Ala Ala Val Leu His His
    690                 695                 700

His Gln Asn Pro Glu Phe Tyr Asp Glu Ile Lys Ile Glu Leu Pro Thr
705                 710                 715                 720

Gln Leu His Glu Lys His His Leu Leu Leu Thr Phe Phe His Val Ser
                725                 730                 735

Cys Asp Asn Ser Ser Lys Gly Ser Thr Lys Lys Arg Asp Val Val Glu
            740                 745                 750

Thr Gln Val Gly Tyr Ser Trp Leu Pro Leu Leu Lys Asp Gly Arg Val
        755                 760                 765

Val Thr Ser Glu Gln His Ile Pro Val Ser Ala Asn Leu Pro Ser Gly
    770                 775                 780

Tyr Leu Gly Tyr Gln Glu Leu Gly Met Gly Arg His Tyr Gly Pro Glu
785                 790                 795                 800

Ile Lys Trp Val Asp Gly Gly Lys Pro Leu Leu Lys Ile Ser Thr His
                805                 810                 815

Leu Val Ser Thr Val Tyr Thr Gln Asp Gln His Leu His Asn Phe Phe
            820                 825                 830

Gln Tyr Cys Gln Lys Thr Glu Ser Gly Ala Gln Ala Leu Gly Asn Glu
        835                 840                 845

Leu Val Lys Tyr Leu Lys Ser Leu His Ala Met Glu Gly His Val Met
    850                 855                 860
```

Ile Ala Phe Leu Pro Thr Ile Leu Asn Gln Leu Phe Arg Val Leu Thr
865 870 875 880

Arg Ala Thr Gln Glu Glu Val Ala Val Asn Val Thr Arg Val Ile Ile
885 890 895

His Val Val Ala Gln Cys His Glu Glu Gly Leu Glu Ser His Leu Arg
900 905 910

Ser Tyr Val Lys Tyr Ala Tyr Lys Ala Glu Pro Tyr Val Ala Ser Glu
915 920 925

Tyr Lys Thr Val His Glu Glu Leu Thr Lys Ser Met Thr Thr Ile Leu
930 935 940

Lys Pro Ser Ala Asp Phe Leu Thr Ser Asn Lys Leu Leu Lys Tyr Ser
945 950 955 960

Trp Phe Phe Phe Asp Val Leu Ile Lys Ser Met Ala Gln His Leu Ile
965 970 975

Glu Asn Ser Lys Val Lys Leu Leu Arg Asn Gln Arg Phe Pro Ala Ser
980 985 990

Tyr His His Ala Val Glu Thr Val Val Asn Met Leu Met Pro His Ile
995 1000 1005

Thr Gln Lys Phe Arg Asp Asn Pro Glu Ala Ser Lys Asn Ala Asn His
1010 1015 1020

Ser Leu Ala Val Phe Ile Lys Arg Cys Phe Thr Phe Met Asp Arg Gly
1025 1030 1035 1040

Phe Val Phe Lys Gln Ile Asn Asn Tyr Ile Ser Cys Phe Ala Pro Gly
1045 1050 1055

Asp Pro Lys Thr Leu Phe Glu Tyr Lys Phe Glu Phe Leu Arg Val Val
1060 1065 1070

Cys Asn His Glu His Tyr Ile Pro Leu Asn Leu Pro Met Pro Phe Gly
1075 1080 1085

Lys Gly Arg Ile Gln Arg Tyr Gln Asp Leu Gln Leu Asp Tyr Ser Leu
1090 1095 1100

Thr Asp Glu Phe Cys Arg Asn His Phe Leu Val Gly Leu Leu Leu Arg
1105 1110 1115 1120

Glu Val Gly Thr Ala Leu Gln Glu Phe Arg Glu Val Arg Leu Ile Ala
1125 1130 1135

Ile Ser Val Leu Lys Asn Leu Leu Ile Lys His Ser Phe Asp Asp Arg
1140 1145 1150

Tyr Ala Ser Arg Ser His Gln Ala Arg Ile Ala Thr Leu Tyr Leu Pro
1155 1160 1165

Leu Phe Gly Leu Leu Ile Glu Asn Val Gln Arg Ile Asn Val Arg Asp
1170 1175 1180

Val Ser Pro Phe Pro Val Asn Ala Gly Met Thr Val Lys Asp Glu Ser
1185 1190 1195 1200

Leu Ala Leu Pro Ala Val Asn Pro Leu Val Thr Pro Gln Lys Gly Ser
1205 1210 1215

Thr Leu Asp Asn Ser Leu His Lys Asp Leu Leu Gly Ala Ile Ser Gly
1220 1225 1230

Ile Ala Ser Pro Tyr Thr Thr Ser Thr Pro Asn Ile Asn Ser Val Arg
1235 1240 1245

Asn Ala Asp Ser Arg Gly Ser Leu Ile Ser Thr Asp Ser Gly Asn Ser
1250 1255 1260

Leu Pro Glu Arg Asn Ser Glu Lys Ser Asn Ser Leu Asp Lys His Gln
1265 1270 1275 1280

Gln Ser Ser Thr Leu Gly Asn Ser Val Val Arg Cys Asp Lys Leu Asp
1285 1290 1295

Gln Ser Glu Ile Lys Ser Leu Leu Met Cys Phe Leu Tyr Ile Leu Lys
1300 1305 1310

Ser Met Ser Asp Asp Ala Leu Phe Thr Tyr Trp Asn Lys Ala Ser Thr
1315 1320 1325

Ser Glu Leu Met Asp Phe Phe Thr Ile Ser Glu Val Cys Leu His Gln
1330 1335 1340

Phe Gln Tyr Met Gly Lys Arg Tyr Ile Ala Arg Thr Gly Met Met His
1345 1350 1355 1360

Ala Arg Leu Gln Gln Leu Gly Ser Leu Asp Asn Ser Leu Thr Phe Asn
1365 1370 1375

His Ser Tyr Gly His Ser Asp Ala Asp Val Leu His Gln Ser Leu Leu
1380 1385 1390

Glu Ala Asn Ile Ala Thr Glu Val Cys Leu Thr Ala Leu Asp Thr Leu
1395 1400 1405

Ser Leu Phe Thr Leu Ala Phe Lys Asn Gln Leu Leu Ala Asp His Gly
1410 1415 1420

His Asn Pro Leu Met Lys Lys Val Phe Asp Val Tyr Leu Cys Phe Leu
1425 1430 1435 1440

Gln Lys His Gln Ser Glu Thr Ala Leu Lys Asn Val Phe Thr Ala Leu
1445 1450 1455

Arg Ser Leu Ile Tyr Lys Phe Pro Ser Thr Phe Tyr Glu Gly Arg Ala
1460 1465 1470

Asp Met Cys Ala Ala Leu Cys Tyr Glu Ile Leu Lys Cys Cys Asn Ser
1475 1480 1485

Lys Leu Ser Ser Ile Arg Thr Glu Ala Ser Gln Leu Leu Tyr Phe Leu
1490 1495 1500

Met Arg Asn Asn Phe Asp Tyr Thr Gly Lys Lys Ser Phe Val Arg Thr
1505 1510 1515 1520

His Leu Gln Val Ile Ile Ser Val Ser Gln Leu Ile Ala Asp Val Val
1525 1530 1535

Gly Ile Gly Gly Thr Arg Phe Gln Gln Ser Leu Ser Ile Ile Asn Asn
1540 1545 1550

Cys Ala Asn Ser Asp Arg Leu Ile Lys His Thr Ser Phe Ser Ser Asp
1555 1560 1565

Val Lys Asp Leu Thr Lys Arg Ile Arg Thr Val Leu Met Ala Thr Ala
1570 1575 1580

Gln Met Lys Glu His Glu Asn Asp Pro Glu Met Leu Val Asp Leu Gln
1585 1590 1595 1600

Tyr Ser Leu Ala Lys Ser Tyr Ala Ser Thr Pro Glu Leu Arg Lys Thr
1605 1610 1615

Trp Leu Asp Ser Met Ala Arg Ile His Val Lys Asn Gly Asp Leu Ser
1620 1625 1630

Glu Ala Ala Met Cys Tyr Val His Val Thr Ala Leu Val Ala Glu Tyr
1635 1640 1645

Leu Thr Arg Lys Glu Ala Val Gln Trp Glu Pro Pro Leu Leu Pro His
1650 1655 1660

Ser His Ser Ala Cys Leu Arg Arg Ser Arg Gly Gly Val Phe Arg Gln
1665 1670 1675 1680

Gly Cys Thr Ala Phe Arg Val Ile Thr Pro Asn Ile Asp Glu Glu Ala
1685 1690 1695

Ser Met Met Glu Asp Val Gly Met Gln Asp Val His Phe Asn Glu Asp
1700 1705 1710

Val Leu Met Glu Leu Leu Glu Gln Cys Ala Asp Gly Leu Trp Lys Ala
1715 1720 1725

Glu Arg Tyr Glu Leu Ile Ala Asp Ile Tyr Lys Leu Ile Ile Pro Ile
1730 1735 1740

Tyr Glu Lys Arg Arg Asp Phe Glu Arg Leu Ala His Leu Tyr Asp Thr
1745 1750 1755 1760

Leu His Arg Ala Tyr Ser Lys Val Thr Glu Val Met His Ser Gly Arg
1765 1770 1775

Arg Leu Leu Gly Thr Tyr Phe Arg Val Ala Phe Phe Gly Gln Ala Ala
1780 1785 1790

Gln Tyr Gln Phe Thr Asp Ser Glu Thr Asp Val Glu Gly Phe Phe Glu
1795 1800 1805

Asp Glu Asp Gly Lys Glu Tyr Ile Tyr Lys Glu Pro Lys Leu Thr Pro
1810 1815 1820

Leu Ser Glu Ile Ser Gln Arg Leu Leu Lys Leu Tyr Ser Asp Lys Phe
1825 1830 1835 1840

Gly Ser Glu Asn Val Lys Met Ile Gln Asp Ser Gly Lys Val Asn Pro
1845 1850 1855

Lys Asp Leu Asp Ser Lys Tyr Ala Tyr Ile Gln Val Thr His Val Ile
1860 1865 1870

Pro Phe Phe Asp Glu Lys Glu Leu Gln Glu Arg Lys Thr Glu Phe Glu
1875 1880 1885

Arg Ser His Asn Ile Arg Arg Phe Met Phe Glu Met Pro Phe Thr Gln
1890 1895 1900

Thr Gly Lys Arg Gln Gly Gly Val Glu Glu Gln Cys Lys Arg Arg Thr
1905 1910 1915 1920

Ile Leu Thr Ala Ile His Cys Phe Pro Tyr Val Lys Lys Arg Ile Pro
1925 1930 1935

Val Met Tyr Gln His His Thr Asp Leu Asn Pro Ile Glu Val Ala Ile
1940 1945 1950

Asp Glu Met Ser Lys Lys Val Ala Glu Leu Arg Gln Leu Cys Ser Ser
1955 1960 1965

Ala Glu Val Asp Met Ile Lys Leu Gln Leu Lys Leu Gln Gly Ser Val
1970 1975 1980

Ser Val Gln Val Asn Ala Gly Pro Leu Ala Tyr Ala Arg Ala Phe Leu
1985 1990 1995 2000

Asp Asp Thr Asn Thr Lys Arg Tyr Pro Asp Asn Lys Val Lys Leu Leu
2005 2010 2015

Lys Glu Val Phe Arg Gln Phe Val Glu Ala Cys Gly Gln Ala Leu Ala
2020 2025 2030

Val Asn Glu Arg Leu Ile Lys Glu Asp Gln Leu Glu Tyr Gln Glu Glu
2035 2040 2045

Met Lys Ala Asn Tyr Arg Glu Met Ala Lys Glu Leu Ser Glu Ile Met
2050 2055 2060

His Glu Gln Ile Cys Pro Leu Glu Glu Lys Thr Ser Val Leu Pro Asn
2065 2070 2075 2080

```
Ser Leu His Ile Phe Asn Ala Ile Ser Gly Thr Pro Thr Ser Thr Met
                2085                2090                2095

Val His Gly Met Thr Ser Ser Ser Ser Val Val
            2100                2105
```

<210> 9
<211> 6171
<212> DNA
<213> Homo sapiens
<400> 9

```
ttttgtttac agggaacacg ggtctggctg agagaaaatg gccagcattt tccaagtact 60
gtaaattcct gtgcagaagg catcgtcgtc ttccggacag actatggtca ggtattcact 120
tacaagcaga gcacaattac ccaccagaag gtgactgcta tgcaccccac gaacgaggag 180
ggcgtggatg acatggcgtc cttgacagag ctccatggcg gctccatcat gtataactta 240
ttccagcggt ataagagaaa tcaaatatgg acctacatcg gctccatcct ggcctctgtg 300
aacccctacc agcccatcgc cgggctgtac gagcctgcca ccatggagca gtacagccgg 360
cgccacctgg gcgagctgcc cccgcacatc ttcgccatcg ccaacgagtg ctaccgctgc 420
ctgtggaagc gccacgacaa ccagtgcatc ctcatcaagg gtgaaagtgg ggcaggtaaa 480
accgaaagca ctaaattgat cctcaagttt ctgtcagtca tcagtcaaca gtctttggaa 540
ttgtccttaa aggagaagac atcctgtgtt gaacgagcta ttcttgaaag cagcccccatc 600
atggaagctt tcggcaatgc gaagaccgtg tacaacaaca actctagtcg ctttgggaag 660
tttgttcagc tgaacatctg tcagaaagga aatattcagg gcgggagaat tgtagattgt 720
atcctctctt cccagaaccg agtagtaagg caaaatcccg gggaaaggaa ttatcacata 780
ttttatgcac tgctggcagg gctggaacat gaagaaagag aagaatttta tttatctacg 840
ccagaaaact accactactt gaatcagtct ggatgtgtag aagacaagac aatcagtgac 900
caggaatcct ttagggaagt tattacggca atggacgtga tgcagttcag caaggaggaa 960
gttcgggaag tgtcgaggct gcttgctggt atactgcatc ttgggaacat agaatttatc 1020
actgctggtg gggcacaggt ttccttcaaa acagctttgg gcagatctgc ggagttactt 1080
gggctggacc caacacagct cacagatgct ttgacccaga gatcaatgtt cctcagggga 1140
gaagagatcc tcacgcctct caatgttcaa caggcagtag acagcaggga ctccctggcc 1200
atggctctgt atgcgtgctg ctttgagtgg gtaatcaaga agatcaacag caggatcaaa 1260
ggcaatgagg acttcaagtc tattggcatc ctcgacatct ttggatttga aaactttgag 1320
gttaatcact ttgaacagtt caatataaac tatgcaaacg agaaacttca ggagtacttc 1380
aacaagcata ttttttcttt agaacaacta gaatatagca gggaaggatt agtgtgggaa 1440
gatattgact ggatagacaa tggagaatgc ctggacttga ttgagaagaa acttggcctc 1500
ctagccctta tcaatgaaga aagccatttt cctcaagcca cagacagcac cttattggag 1560
aagctacaca gtcagcatgc gaataaccac ttttatgtga agcccagagt tgcagttaac 1620
aattttggag tgaagcacta tgctggagag gtgcaatatg atgtccgagg tatcttggag 1680
aagaacagag atacatttcg agatgacctt ctcaatttgc taagagaaag ccggtttgac 1740
tttatctacg atctttttga acatgtttca agccgcaaca accaggatac cttgaaatgt 1800
ggaagcaaac atcggcggcc tacagtcagc tcacagttca aggttgactc actgcattcc 1860
ttaatggcaa cgctaagctc ctctaatcct ttctttgttc gctgtatcaa gccaaacatg 1920
cagaagatgc cagaccagtt tgaccaggcg gttgtgctga accagctgcg gtactcaggg 1980
atgctggaga ctgtgagaat ccgcaaagct gggtatgcgg tccgaagacc ctttcaggac 2040
ttttacaaaa ggtataaagt gctgatgagg aatctggctc tgcctgagga cgtccgaggg 2100
aagtgcacga gcctgctgca gctctatgat gcctccaaca gcgagtggca gctggggaag 2160
accaaggtat ttcttcgaga atccttggaa cagaaactgg agaagcggag ggaagaggaa 2220
gtgagccacg cggccatggt gattcgggcc catgtcttgg gcttcttagc acggaaacaa 2280
tacagaaagg tcctttattg tgtggtgata atacagaaga attacagagc attccttctg 2340
aggaggagat tttgcacct gaaaaaggca gccatagttt tccagaagca actcagaggt 2400
cagattgctc ggagagttta cagacaattg ctggcagaga aaagggagca agaagaaaag 2460
aagaaacagg aagaggaaga aaagaagaaa cgggaggaag aagaaagaga aagagagaga 2520
gagcgaagag aagccgagct ccgcgcccag caggaagaag aaacgaggaa gcagcaagaa 2580
ctcgaagcct tgcagaagag ccagaaggaa gctgaactga cccgtgaact ggagaaacag 2640
aaggaaaata agcaggtgga agagatcctc cgtctggaga aagaaatcga ggacctgcag 2700
```

```
cgcatgaagg agcagcagga gctgtcgctg accgaggctt ccctgcagaa gctgcaggag 2760
cggcgggacc aggagctccg caggctggag gaggaagcgt gcagggcggc ccaggagttc 2820
ctcgagtccc tcaatttcga cgagatcgac gagtgtgtcc ggaatatcga gcggtccctg 2880
tcggggggaa gcgaattttc cagcgagctg gctgagagcg catgcgagga gaagcccaac 2940
ttcaacttca gccagcccta cccagaggag gaggtcgatg agggcttcga agccgacgac 3000
gacgccttca aggactcccc caaccccagc gagcacggcc actcagacca gcgaacaagt 3060
ggcatccgga ccagcgatga ctcttcagag gaggacccat acatgaacga cacggtggtg 3120
cccaccagcc ccagtgcgga cagcacggtg ctgctcgccc catcagtgca ggactccggg 3180
agcctacaca actcctccag cggcgagtcc acctactgca tgccccagaa cgctggggac 3240
ttgccctccc cagacggcga ctacgactac gaccaggatg actatgagga cggtgccatc 3300
acttccggca gcagcgtgac cttctccaac tcctacggca gccagtggtc cccgactac 3360
cgctgctctg tggggaccta caacagctcg ggtgcctacc ggttcagctc tgaggggcg 3420
cagtcctcgt ttgaagatag tgaagaggac tttgattcca ggtttgatac agatgatgag 3480
ctttcatacc ggcgtgactc tgtgtacagc tgtgtcactc tgccgtattt ccacagcttt 3540
ctgtacatga aaggtggcct gatgaactct tggaaacgcc gctggtgcgt cctcaaggat 3600
gaaaccttct tgtggttccg ctccaagcag gaggccctca agcaaggctg gctccacaaa 3660
aaagggggg gctcctccac gctgtccagg agaaattgga agaagcgctg gtttgtcctc 3720
cgccagtcca agctgatgta ctttgaaaac gacagcgagg agaagctcaa gggcaccgta 3780
gaagtgcgaa cggcaaaaga gatcatagat aacaccacca aggagaatgg gatcgacatc 3840
attatggccg ataggacttt ccacctgatt gcagagtccc cagaagatgc cagccagtgg 3900
ttcagcgtgc tgagtcaggt ccacgcgtcc acggaccagg agatccagga gatgcatgat 3960
gagcaggcaa acccacagaa tgctgtgggc accttggatg tggggctgat tgattctgtg 4020
tgtgcctctg acagccctga tagacccaac tcgtttgtga tcatcacggc caaccgggtg 4080
ctgcactgca acgccgacac gccggaggag atgcaccact ggataaccct gctgcagagg 4140
tccaaagggg acaccagagt ggagggccag gaattcatcg tgagaggatg gttgcacaaa 4200
gaggtgaaga acagtccaaa gatgtcttca ctgaaactga agaaacggtg gtttgtactc 4260
acccacaatt ccctggatta ctacaagagt tcagagaaga acgcgctcaa actggggacc 4320
ctggtcctca acagcctctg ctctgtcgtc cccccagatg agaagatatt caaagagaca 4380
ggctactgga acgtcaccgt gtacgggcgc aagcactgtt accggctcta caccaagctg 4440
ctcaacgagg ccacccggtg gtccagtgtc attcaaaacg tgactgacac caaggccccg 4500
atcgacaccc ccacccagca gctgattcaa gatatcaagg agaactgcct gaactcggat 4560
gtggtggaac agatttacaa gcggaacccg atccttcgat acacccatca ccccttgcac 4620
tccccgctcc tgccccttcc gtatggggac ataaatctca acttgctgaa agacaaaggc 4680
tataccaccc ttcaggatga ggccatcaag atattcaatt ccctgcagca actggagtcc 4740
atgtctgacc caattccaat aatccagggc atcctacaga cagggcatga cctgcgacct 4800
ctgcgggacg agctgtactg ccagcttatc aaacagacca acaaagtgcc ccacccccggc 4860
agtgtgggca acctgtacag ctggcagatc ctgacatgcc tgagctgcac cttcctgccg 4920
agtcgaggga ttctcaagta tctcaagttc catctgaaaa ggatacggga acagtttcca 4980
ggaaccgaga tggaaaaata cgctctcttc acttacgaat ctcttaagaa aaccaaatgc 5040
cgagagtttg tgccttcccg agatgaaata gaagctctga tccacaggca ggaaatgaca 5100
tccacggtct attgccatgg cggcggctcc tgcaagatca ccatcaactc ccacaccacc 5160
gctggggagg tggtggagaa gctgatccga ggcctggcca tggaggacag caggaacatg 5220
tttgctttgt ttgaatacaa cggccacgtc gacaaagcca ttgaaagtcg aaccgtcgta 5280
gctgatgtct tagccaagtt tgaaaagctg gctgccacat ccgaggttgg ggacctgcca 5340
tggaaattct acttcaaact ttactgcttc ctggacacag acaacgtgcc aaaagacagt 5400
gtggagtttg catttatgtt tgaacaggcc cacgaagcgg ttatccatgg ccaccatcca 5460
gccccggaag aaaacctcca ggttcttgct gccctgcgac tccagtatct gcagggggat 5520
tatactctgc acgctgccat cccacctctc gaagaggttt attccctgca gagactcaag 5580
gcccgcatca gccagtcaac caaaaccttc accccttgtg aacggctgga gaagaggcgg 5640
acgagcttcc tagaggggac cctgaggcgg agcttccgga caggatccgt ggtccggcag 5700
aaggtcgagg aggagcagat gctggacatg tggattaagg aagaagtctc ctctgctcga 5760
gccagtatca ttgacaagtg gaggaaattt cagggaatga accaggaaca ggccatggcc 5820
aagtacatgg ccttgatcaa ggagtggcct ggctatggct cgacgctgtt tgatgtggag 5880
tgcaaggaag gtggcttccc tcaggaactc tggttgggtg tcagcgcgga cgccgtctcc 5940
gtctacaagc gtggagaggg aagaccactg gaagtcttcc agtatgaaca catcctctct 6000
tttggggcac ccctggcgaa tacgtataag atcgtggtcg atgagaggga gctgctcttt 6060
gaaaccagtg aggtagtgga tgtggccaag ctcatgaaag cctacatcag catgatcgtg 6120
aagaagcgct acagcacgac acgctccgcc agcagccagg gcagctccag g          6171
```

<210> 10
<211> 2057
<212> PRT
<213> Homo sapiens
<400> 10

```
Phe Cys Leu Gln Gly Thr Arg Val Trp Leu Arg Glu Asn Gly Gln His
1               5                   10                  15

Phe Pro Ser Thr Val Asn Ser Cys Ala Glu Gly Ile Val Val Phe Arg
            20                  25                  30

Thr Asp Tyr Gly Gln Val Phe Thr Tyr Lys Gln Ser Thr Ile Thr His
        35                  40                  45

Gln Lys Val Thr Ala Met His Pro Thr Asn Glu Glu Gly Val Asp Asp
    50                  55                  60

Met Ala Ser Leu Thr Glu Leu His Gly Gly Ser Ile Met Tyr Asn Leu
65                  70                  75                  80

Phe Gln Arg Tyr Lys Arg Asn Gln Ile Trp Thr Tyr Ile Gly Ser Ile
                85                  90                  95

Leu Ala Ser Val Asn Pro Tyr Gln Pro Ile Ala Gly Leu Tyr Glu Pro
            100                 105                 110

Ala Thr Met Glu Gln Tyr Ser Arg Arg His Leu Gly Glu Leu Pro Pro
        115                 120                 125

His Ile Phe Ala Ile Ala Asn Glu Cys Tyr Arg Cys Leu Trp Lys Arg
    130                 135                 140

His Asp Asn Gln Cys Ile Leu Ile Lys Gly Glu Ser Gly Ala Gly Lys
145                 150                 155                 160

Thr Glu Ser Thr Lys Leu Ile Leu Lys Phe Leu Ser Val Ile Ser Gln
                165                 170                 175

Gln Ser Leu Glu Leu Ser Leu Lys Glu Lys Thr Ser Cys Val Glu Arg
            180                 185                 190

Ala Ile Leu Glu Ser Ser Pro Ile Met Glu Ala Phe Gly Asn Ala Lys
        195                 200                 205

Thr Val Tyr Asn Asn Asn Ser Ser Arg Phe Gly Lys Phe Val Gln Leu
    210                 215                 220

Asn Ile Cys Gln Lys Gly Asn Ile Gln Gly Gly Arg Ile Val Asp Cys
225                 230                 235                 240

Ile Leu Ser Ser Gln Asn Arg Val Val Arg Gln Asn Pro Gly Glu Arg
                245                 250                 255

Asn Tyr His Ile Phe Tyr Ala Leu Leu Ala Gly Leu Glu His Glu Glu
            260                 265                 270
```

```
Arg Glu Glu Phe Tyr Leu Ser Thr Pro Glu Asn Tyr His Tyr Leu Asn
        275                 280                 285

Gln Ser Gly Cys Val Glu Asp Lys Thr Ile Ser Asp Gln Glu Ser Phe
    290                 295                 300

Arg Glu Val Ile Thr Ala Met Asp Val Met Gln Phe Ser Lys Glu Glu
305                 310                 315                 320

Val Arg Glu Val Ser Arg Leu Leu Ala Gly Ile Leu His Leu Gly Asn
                325                 330                 335

Ile Glu Phe Ile Thr Ala Gly Gly Ala Gln Val Ser Phe Lys Thr Ala
            340                 345                 350

Leu Gly Arg Ser Ala Glu Leu Leu Gly Leu Asp Pro Thr Gln Leu Thr
        355                 360                 365

Asp Ala Leu Thr Gln Arg Ser Met Phe Leu Arg Gly Glu Glu Ile Leu
    370                 375                 380

Thr Pro Leu Asn Val Gln Gln Ala Val Asp Ser Arg Asp Ser Leu Ala
385                 390                 395                 400

Met Ala Leu Tyr Ala Cys Cys Phe Glu Trp Val Ile Lys Lys Ile Asn
                405                 410                 415

Ser Arg Ile Lys Gly Asn Glu Asp Phe Lys Ser Ile Gly Ile Leu Asp
            420                 425                 430

Ile Phe Gly Phe Glu Asn Phe Glu Val Asn His Phe Glu Gln Phe Asn
        435                 440                 445

Ile Asn Tyr Ala Asn Glu Lys Leu Gln Glu Tyr Phe Asn Lys His Ile
    450                 455                 460

Phe Ser Leu Glu Gln Leu Glu Tyr Ser Arg Glu Gly Leu Val Trp Glu
465                 470                 475                 480

Asp Ile Asp Trp Ile Asp Asn Gly Glu Cys Leu Asp Leu Ile Glu Lys
                485                 490                 495

Lys Leu Gly Leu Leu Ala Leu Ile Asn Glu Glu Ser His Phe Pro Gln
            500                 505                 510

Ala Thr Asp Ser Thr Leu Leu Glu Lys Leu His Ser Gln His Ala Asn
        515                 520                 525

Asn His Phe Tyr Val Lys Pro Arg Val Ala Val Asn Asn Phe Gly Val
    530                 535                 540

Lys His Tyr Ala Gly Glu Val Gln Tyr Asp Val Arg Gly Ile Leu Glu
545                 550                 555                 560

Lys Asn Arg Asp Thr Phe Arg Asp Asp Leu Leu Asn Leu Leu Arg Glu
                565                 570                 575
```

```
Ser Arg Phe Asp Phe Ile Tyr Asp Leu Phe Glu His Val Ser Ser Arg
            580                 585                 590

Asn Asn Gln Asp Thr Leu Lys Cys Gly Ser Lys His Arg Arg Pro Thr
        595                 600                 605

Val Ser Ser Gln Phe Lys Val Asp Ser Leu His Ser Leu Met Ala Thr
    610                 615                 620

Leu Ser Ser Ser Asn Pro Phe Phe Val Arg Cys Ile Lys Pro Asn Met
625                 630                 635                 640

Gln Lys Met Pro Asp Gln Phe Asp Gln Ala Val Val Leu Asn Gln Leu
                645                 650                 655

Arg Tyr Ser Gly Met Leu Glu Thr Val Arg Ile Arg Lys Ala Gly Tyr
            660                 665                 670

Ala Val Arg Arg Pro Phe Gln Asp Phe Tyr Lys Arg Tyr Lys Val Leu
        675                 680                 685

Met Arg Asn Leu Ala Leu Pro Glu Asp Val Arg Gly Lys Cys Thr Ser
    690                 695                 700

Leu Leu Gln Leu Tyr Asp Ala Ser Asn Ser Glu Trp Gln Leu Gly Lys
705                 710                 715                 720

Thr Lys Val Phe Leu Arg Glu Ser Leu Glu Gln Lys Leu Glu Lys Arg
                725                 730                 735

Arg Glu Glu Glu Val Ser His Ala Ala Met Val Ile Arg Ala His Val
            740                 745                 750

Leu Gly Phe Leu Ala Arg Lys Gln Tyr Arg Lys Val Leu Tyr Cys Val
        755                 760                 765

Val Ile Ile Gln Lys Asn Tyr Arg Ala Phe Leu Leu Arg Arg Arg Phe
    770                 775                 780

Leu His Leu Lys Lys Ala Ala Ile Val Phe Gln Lys Gln Leu Arg Gly
785                 790                 795                 800

Gln Ile Ala Arg Arg Val Tyr Arg Gln Leu Leu Ala Glu Lys Arg Glu
                805                 810                 815

Gln Glu Glu Lys Lys Lys Gln Glu Glu Glu Glu Lys Lys Lys Arg Glu
            820                 825                 830

Glu Glu Glu Arg Glu Arg Glu Arg Glu Arg Arg Glu Ala Glu Leu Arg
        835                 840                 845

Ala Gln Gln Glu Glu Glu Thr Arg Lys Gln Gln Glu Leu Glu Ala Leu
    850                 855                 860

Gln Lys Ser Gln Lys Glu Ala Glu Leu Thr Arg Glu Leu Glu Lys Gln
865                 870                 875                 880
```

Lys Glu Asn Lys Gln Val Glu Glu Ile Leu Arg Leu Glu Lys Glu Ile
885 890 895

Glu Asp Leu Gln Arg Met Lys Glu Gln Gln Glu Leu Ser Leu Thr Glu
900 905 910

Ala Ser Leu Gln Lys Leu Gln Glu Arg Arg Asp Gln Glu Leu Arg Arg
915 920 925

Leu Glu Glu Glu Ala Cys Arg Ala Ala Gln Glu Phe Leu Glu Ser Leu
930 935 940

Asn Phe Asp Glu Ile Asp Glu Cys Val Arg Asn Ile Glu Arg Ser Leu
945 950 955 960

Ser Gly Gly Ser Glu Phe Ser Ser Glu Leu Ala Glu Ser Ala Cys Glu
965 970 975

Glu Lys Pro Asn Phe Asn Phe Ser Gln Pro Tyr Pro Glu Glu Glu Val
980 985 990

Asp Glu Gly Phe Glu Ala Asp Asp Asp Ala Phe Lys Asp Ser Pro Asn
995 1000 1005

Pro Ser Glu His Gly His Ser Asp Gln Arg Thr Ser Gly Ile Arg Thr
1010 1015 1020

Ser Asp Asp Ser Ser Glu Glu Asp Pro Tyr Met Asn Asp Thr Val Val
1025 1030 1035 1040

Pro Thr Ser Pro Ser Ala Asp Ser Thr Val Leu Leu Ala Pro Ser Val
1045 1050 1055

Gln Asp Ser Gly Ser Leu His Asn Ser Ser Ser Gly Glu Ser Thr Tyr
1060 1065 1070

Cys Met Pro Gln Asn Ala Gly Asp Leu Pro Ser Pro Asp Gly Asp Tyr
1075 1080 1085

Asp Tyr Asp Gln Asp Asp Tyr Glu Asp Gly Ala Ile Thr Ser Gly Ser
1090 1095 1100

Ser Val Thr Phe Ser Asn Ser Tyr Gly Ser Gln Trp Ser Pro Asp Tyr
1105 1110 1115 1120

Arg Cys Ser Val Gly Thr Tyr Asn Ser Ser Gly Ala Tyr Arg Phe Ser
1125 1130 1135

Ser Glu Gly Ala Gln Ser Ser Phe Glu Asp Ser Glu Glu Asp Phe Asp
1140 1145 1150

Ser Arg Phe Asp Thr Asp Asp Glu Leu Ser Tyr Arg Arg Asp Ser Val
1155 1160 1165

Tyr Ser Cys Val Thr Leu Pro Tyr Phe His Ser Phe Leu Tyr Met Lys
1170 1175 1180

Gly Gly Leu Met Asn Ser Trp Lys Arg Arg Trp Cys Val Leu Lys Asp
1185 1190 1195 1200

Glu Thr Phe Leu Trp Phe Arg Ser Lys Gln Glu Ala Leu Lys Gln Gly
1205 1210 1215

Trp Leu His Lys Lys Gly Gly Gly Ser Ser Thr Leu Ser Arg Arg Asn
1220 1225 1230

Trp Lys Lys Arg Trp Phe Val Leu Arg Gln Ser Lys Leu Met Tyr Phe
1235 1240 1245

Glu Asn Asp Ser Glu Glu Lys Leu Lys Gly Thr Val Glu Val Arg Thr
1250 1255 1260

Ala Lys Glu Ile Ile Asp Asn Thr Thr Lys Glu Asn Gly Ile Asp Ile
1265 1270 1275 1280

Ile Met Ala Asp Arg Thr Phe His Leu Ile Ala Glu Ser Pro Glu Asp
1285 1290 1295

Ala Ser Gln Trp Phe Ser Val Leu Ser Gln Val His Ala Ser Thr Asp
1300 1305 1310

Gln Glu Ile Gln Glu Met His Asp Glu Gln Ala Asn Pro Gln Asn Ala
1315 1320 1325

Val Gly Thr Leu Asp Val Gly Leu Ile Asp Ser Val Cys Ala Ser Asp
1330 1335 1340

Ser Pro Asp Arg Pro Asn Ser Phe Val Ile Ile Thr Ala Asn Arg Val
1345 1350 1355 1360

Leu His Cys Asn Ala Asp Thr Pro Glu Glu Met His His Trp Ile Thr
1365 1370 1375

Leu Leu Gln Arg Ser Lys Gly Asp Thr Arg Val Glu Gly Gln Glu Phe
1380 1385 1390

Ile Val Arg Gly Trp Leu His Lys Glu Val Lys Asn Ser Pro Lys Met
1395 1400 1405

Ser Ser Leu Lys Leu Lys Lys Arg Trp Phe Val Leu Thr His Asn Ser
1410 1415 1420

Leu Asp Tyr Tyr Lys Ser Ser Glu Lys Asn Ala Leu Lys Leu Gly Thr
1425 1430 1435 1440

Leu Val Leu Asn Ser Leu Cys Ser Val Val Pro Pro Asp Glu Lys Ile
1445 1450 1455

Phe Lys Glu Thr Gly Tyr Trp Asn Val Thr Val Tyr Gly Arg Lys His
1460 1465 1470

Cys Tyr Arg Leu Tyr Thr Lys Leu Leu Asn Glu Ala Thr Arg Trp Ser
1475 1480 1485

```
Ser Val Ile Gln Asn Val Thr Asp Thr Lys Ala Pro Ile Asp Thr Pro
    1490                1495                1500

Thr Gln Gln Leu Ile Gln Asp Ile Lys Glu Asn Cys Leu Asn Ser Asp
1505                1510                1515                1520

Val Val Glu Gln Ile Tyr Lys Arg Asn Pro Ile Leu Arg Tyr Thr His
                1525                1530                1535

His Pro Leu His Ser Pro Leu Leu Pro Leu Pro Tyr Gly Asp Ile Asn
            1540                1545                1550

Leu Asn Leu Leu Lys Asp Lys Gly Tyr Thr Thr Leu Gln Asp Glu Ala
        1555                1560                1565

Ile Lys Ile Phe Asn Ser Leu Gln Gln Leu Glu Ser Met Ser Asp Pro
    1570                1575                1580

Ile Pro Ile Ile Gln Gly Ile Leu Gln Thr Gly His Asp Leu Arg Pro
1585                1590                1595                1600

Leu Arg Asp Glu Leu Tyr Cys Gln Leu Ile Lys Gln Thr Asn Lys Val
                1605                1610                1615

Pro His Pro Gly Ser Val Gly Asn Leu Tyr Ser Trp Gln Ile Leu Thr
            1620                1625                1630

Cys Leu Ser Cys Thr Phe Leu Pro Ser Arg Gly Ile Leu Lys Tyr Leu
        1635                1640                1645

Lys Phe His Leu Lys Arg Ile Arg Glu Gln Phe Pro Gly Thr Glu Met
    1650                1655                1660

Glu Lys Tyr Ala Leu Phe Thr Tyr Glu Ser Leu Lys Lys Thr Lys Cys
1665                1670                1675                1680

Arg Glu Phe Val Pro Ser Arg Asp Glu Ile Glu Ala Leu Ile His Arg
                1685                1690                1695

Gln Glu Met Thr Ser Thr Val Tyr Cys His Gly Gly Gly Ser Cys Lys
            1700                1705                1710

Ile Thr Ile Asn Ser His Thr Thr Ala Gly Glu Val Val Glu Lys Leu
        1715                1720                1725

Ile Arg Gly Leu Ala Met Glu Asp Ser Arg Asn Met Phe Ala Leu Phe
    1730                1735                1740

Glu Tyr Asn Gly His Val Asp Lys Ala Ile Glu Ser Arg Thr Val Val
1745                1750                1755                1760

Ala Asp Val Leu Ala Lys Phe Glu Lys Leu Ala Ala Thr Ser Glu Val
                1765                1770                1775

Gly Asp Leu Pro Trp Lys Phe Tyr Phe Lys Leu Tyr Cys Phe Leu Asp
            1780                1785                1790
```

```
Thr Asp Asn Val Pro Lys Asp Ser Val Glu Phe Ala Phe Met Phe Glu
        1795                1800                1805

Gln Ala His Glu Ala Val Ile His Gly His His Pro Ala Pro Glu Glu
    1810                1815                1820

Asn Leu Gln Val Leu Ala Ala Leu Arg Leu Gln Tyr Leu Gln Gly Asp
1825                1830                1835                1840

Tyr Thr Leu His Ala Ala Ile Pro Pro Leu Glu Glu Val Tyr Ser Leu
                1845                1850                1855

Gln Arg Leu Lys Ala Arg Ile Ser Gln Ser Thr Lys Thr Phe Thr Pro
            1860                1865                1870

Cys Glu Arg Leu Glu Lys Arg Arg Thr Ser Phe Leu Glu Gly Thr Leu
        1875                1880                1885

Arg Arg Ser Phe Arg Thr Gly Ser Val Val Arg Gln Lys Val Glu Glu
    1890                1895                1900

Glu Gln Met Leu Asp Met Trp Ile Lys Glu Glu Val Ser Ser Ala Arg
1905                1910                1915                1920

Ala Ser Ile Ile Asp Lys Trp Arg Lys Phe Gln Gly Met Asn Gln Glu
                1925                1930                1935

Gln Ala Met Ala Lys Tyr Met Ala Leu Ile Lys Glu Trp Pro Gly Tyr
            1940                1945                1950

Gly Ser Thr Leu Phe Asp Val Glu Cys Lys Glu Gly Gly Phe Pro Gln
        1955                1960                1965

Glu Leu Trp Leu Gly Val Ser Ala Asp Ala Val Ser Val Tyr Lys Arg
    1970                1975                1980

Gly Glu Gly Arg Pro Leu Glu Val Phe Gln Tyr Glu His Ile Leu Ser
1985                1990                1995                2000

Phe Gly Ala Pro Leu Ala Asn Thr Tyr Lys Ile Val Val Asp Glu Arg
                2005                2010                2015

Glu Leu Leu Phe Glu Thr Ser Glu Val Val Asp Val Ala Lys Leu Met
            2020                2025                2030

Lys Ala Tyr Ile Ser Met Ile Val Lys Lys Arg Tyr Ser Thr Thr Arg
        2035                2040                2045

Ser Ala Ser Ser Gln Gly Ser Ser Arg
    2050                2055
```

<210> 11
<211> 9621
<212> DNA
<213> Homo sapiens
<400> 11

```
agctagtatc ttttattgtc agaacttctg tgagccaaca aacagttttg catggttgta 60
cacaaaggga caaggcaaat ttcttttttc gtgtgggtag acttagttgg cccaagtcct 120
taaaactttt ccatataaaa ataaaaagtc caagaccaga ttatttttct tctggtcata 180
aatgctgatt tatttacagg tgccttgttc agaccaccat tataaacttg ggataaaata 240
tgtgtgtatt aaagcctcag catttaatgt cagggtcctt tgaagattca ctcaagtgtt 300
aagacgtttc tggaatgcag cgtctctccc ccatagtcaa catggttatt atatctgtaa 360
tctatccaga atgatagaag ctaaccttcc aagtaacact ttgtttttaa cttaaatctt 420
ttagacatga aagactccaa aatgacttca ttcttgttct aaaaccagca ctggagccag 480
ctgttgaaga gtggtttata aatacagtta tcttgtaggc tgcttatctg tttataatac 540
agcagacaca gatggcagac tttgctacat gtaaaacaat ggagtcaaca cgtgtttttc 600
aaaatacagc aaagacagga aaatccagga tttgggtttg ttaataaaac caccttataa 660
agtaacaatt gagactatag ctctgcatta ttaaaatata cagactgtgt acaccattac 720
acatcctttt tccctttgct ttttaatgct catgaaacca tgattaaggt gttgagttta 780
tgaacacatg cacgaacagg caagcacgta cacttaaaag atgaaacaaa gaaaaaagtt 840
gattcatgtc attccatgag aaaggctgcc cgcagcactc cagctcaaac acactgtccc 900
ctcgagctct ccatcccccct tcccactccc tcaccttccc tcagattcgg ggaaatcagg 960
ttgggaggtt agtgcatcat tgacagagaa tgccccccctt ccacgctctg ttaagtctcc 1020
cccagaaggg ggaaaggcag ttcccttcag tagcacagtt acggtcgatt agtgttggtt 1080
ccacaagtta aggcacttcc ggctgctttg gtggcagcgt ggttcctccc ctcctttttt 1140
aaggcatgtg tcctctaaga gtagtaaagc tttggaaact gtgcagactg ttaaagttga 1200
cagcttaata caggatcaat gaaggcggca ggcaaaagga tcctcggaga cacctccctc 1260
agaccagaag cttccagaaa gcctgggcag ctctgtgttt gttttggctg ggcatggcac 1320
actggagcca gcctaggcca gagggtggtg cgttcaggta gcaaagacag gtgggctctg 1380
tcccgccttc acctggagct gccctggctg ctggcggagc gtgtcgtgct gtagcgcttc 1440
ttcacgatca tgctgatgta ggctttcatg agcttggcca catccaccac ctcactggtt 1500
tcaaagagca gctccctctc atcgaccacg atcttatacg tattcgccag gggtgcccca 1560
aaagagagga tgtgttcata ctggaagact tccagtggtc ttccctctcc acgcttgtag 1620
acggagacgg cgtccgcgct gacacccaac cagagttcct gagggaagcc accttccttg 1680
cactggtggg caagagtcaa cagaagagtt aagtcatgaa gtggttggca acagaaagca 1740
tctaaaccat aagacaggct ttgagtgaag tcctctgtgc agaagattaa atatattcga 1800
tgtgcatgca tgcatggagg ggcctgaaat atgaaaaatg gcacctctct ggctatcttg 1860
atttctaact agttaatctc acgcttttgg gaaaacctca ctaactggca gagtctaaca 1920
tcttgctttg actctccact tctcagcatt attctactag ctgtttggat tagctacgtg 1980
gaagtggcct ggaaacgtac atgcttggcc gggggactta agaaagcttc cctgcaaccc 2040
aagccaagtc tactcttgta ttaatatctc cagttctgcc tccaatcctc tttgcggatg 2100
gttagtcttc aaatacaaaa tctaggatca cagaggaaaa ttctccaaat cacgcatgct 2160
gagcagttct ggctcctctt cacaaggagc agcaatggcc ttccatatgc agagtgggaa 2220
cagggacttt accagtttaa ctgtagactt tcctgtacag attggtggaa gaaaataaga 2280
ccccatatga aggggctaac aacacagggc tgatccaaac ctggacaagc aggagggcta 2340
taaattggag acgctgaaaa gagtctctag tttatatccc taataaccag acattctctg 2400
catcctccat gcaaaagcca gtagctttct ttttttcttt ttttttttga cggagtctca 2460
ctctgtcgcc caggctggag tgcagtggcg tgatcctggc tcactgcaac ctccacctcc 2520
tgagttcaag cgattctcct gtctcagcct ctcgagtagc tgggattaca ggtgcatgcc 2580
accacgccca gctaattttt tgcagagatg gggtttcacc gtgttagcca ggatggtctc 2640
gatctcctga cctcatgatc cgcccgcctt ggcctcccaa agcgctggga ttacaggcat 2700
gagccaccgc gcccggccaa gccagtagct ttctatgcta attcacagct cacgttttgc 2760
aggaagccaa gagtttaact gctattatct attccttgtc agggagaaat ggaattatgg 2820
ctttgtacaa agcacctgat ttttttatac ttaaaaacag gcataattga accaaccaaa 2880
ccaatcaaaa acatcaccta atgaaaagcc acccacggat tctagaattt ataatattta 2940
gaattttata cagcctcaat ataaagtcat cagatatacg ctgaattact gtgatcataa 3000
aaaatggaag ctaatctaga cgatgagctg gcacacttat ctgttaaggg ctgcatagta 3060
aagattttta gactttgtgg atcacatggt ctctgtcaca actactcaac tctgtacaaa 3120
aacagccagg gaatatatct aaaggaatga gcttggctgt gttccaataa aactttgttt 3180
agaaaaaaag gaggcaggca agatctgacc cacagaccag tttgccaaac tctcatctag 3240
acaattagta agatttcttt tcaataagcg gtctacttaa aacaaaacaa aaatcagtac 3300
tgggttgatg ccaatggcta aattccatta cgagatagac attcttcctt tcaaacagat 3360
ggctgtaaag aaaaaacaaa gtaaaatgca agtatatcca aagtttctaa tttgtatata 3420
```

cagctataac attttttaa atgtagattt ttatcagtgt ttaaaaaatt agatctatag 3480
cttccctaag gaagggtaga agaatagatg acatcttaat tttgcattca ttcctaatat 3540
tacagatgca tttactacac aggagaagag aaactgtgag gagaagggag gcgttaatgg 3600
tacaattttg gggctcgaa aaaaagaggt tgagagagca aaatgctcca tcttgtcttc 3660
tctccacatg aacttggccg tgatccatgt tctcagatgc cagcacccag cccaccccaa 3720
cacatggcag ccagttctca cctccacatc aaacagcgtc gagccatagc caggccactc 3780
cttgatcaag gccatgtact tggccatggc ctgttcctgg ttcattccct gaaatttcct 3840
ccacttgtca atgatactgg ctcgagcaga ggagacttct tccttaatcc acatgtccag 3900
catctgctcc tcctcgacct tctgccggac cacggatcct gtccggaagc tccgcctcag 3960
ggtcccctct aggaagctcg tccgcctctt ctccagccgt tcacaagggg tgaaggtttt 4020
ggttgactgg ctgatgcggg ccttgagtct ctgcagggaa taaacctctt cgagaggtgg 4080
gatggcagcg tgcagagtat aatccccctg cagatactgg agtcgcaggg cagcaagaac 4140
ctggaggttt tcttccgggg ctggatggtg gccatggata accgcttcgt gggcctgttc 4200
aaacataaat gcaaactcca cactgtcttt tggcacgttg tctgtgtcca ggaagcagta 4260
aagtttgaag tagaatttcc atggcaggtc cccaacctcg gatgtggcag ccagcttttc 4320
aaacttggct aagacatcag ctacgacggt tcgactttca atggctttgt cgacgtggcc 4380
gttgtattca aacaaagcaa acatgttcct gctgtcctcc atggccaggc ctcggatcag 4440
cttctccacc acctccccag cggtggtgtg ggagttgatg gtgatcttgc aggagccgcc 4500
gccatggcaa tagaccgtgg atgtcatttc ctgcctgtgg atcagagctt ctatttcatc 4560
tcgggaaggc acaaactctc ggcatttggt tttcttaaga gattcgtaag tgaagagagc 4620
gtatttttcc atctcggttc ctggaaactg ttcccgtatc cttttcagat ggaacttgag 4680
atacttgaga atccctcgac tcggcaggaa ggtgcagctc aggcatgtca ggatctgcca 4740
gctgtacagg ttgcccacac tgccggggtg gggcactttg ttggtctgtt tgataagctg 4800
gcagtacagc tcgtcccgca gaggtcgcag gtcatgccct gtctgtagga tgccctggat 4860
tattggaatt gggtcagaca tggactccag ttgctgcagg gaattgaata tcttgatggc 4920
ctcatcctga agggtggtat agcctttgtc tttcagcaag ttgagattta tgtccccata 4980
cggaaggggc aggagcgggg agtgcaaggg gtgatgggtg tatcgaagga tcgggttccg 5040
cttgtaaatc tgttccacca catccgagtt caggcagttc tccttgatat cttgaatcag 5100
ctgctgggtg gggtgtcgga tcggggcctt ggtgtcagtc acgttttgaa tgacactgga 5160
ccaccgggtg gcctcgttga gcagcttggt gtagagccgg taacagtgct tgcgcccgta 5220
cacggtgacg ttccagtagc ctgtctcttt gaatatcttc tcatctgggg ggacgacaga 5280
gcagaggctg ttgaggacca gggtccccag tttgagcgcg ttcttctctg aactcttgta 5340
gtaatccagg gaattgtggg tgagtacaaa ccaccgtttc ttcagtttca gtgaagacat 5400
ctttggactg ttcttcacct ctttgtgcaa ccatcctctc acgatgaatt cctggccctc 5460
cactctggtg tcccctttgg acctctgcag cagggttatc cagtggtgca tctcctccgg 5520
cgtgtcggcg ttgcagtgca gcacccggtt ggccgtgatg atcacaaacg agttgggtct 5580
atcagggctg tcagaggcac acacagaatc aatcagcccc acatccaagg tgcccacagc 5640
attctgtggg tttgcctgct catcatgcat ctcctggatc tcctggtccg tggacgcgtg 5700
gacctgactc agcacgctga accactggct ggcatcttct ggggactctg caatcaggtg 5760
gaaagtccta tcggccataa tgatgtcgat cccattctcc ttggtggtgt tatctatgat 5820
ctcttttgcc gttcgcactt ctacggtgcc cttgagcttc tcctcgctgt cgttttcaaa 5880
gtacatcagc ttggactggc ggaggacaaa ccagcgcttc ttccaatttc tcctggacag 5940
cgtggaggag cccccccctt ttttgtggag ccagccttgc ttgagggcct cctgcttgga 6000
gcggaaccac aagaaggttt catccttgag gacgcaccag cggcgtttcc aagagttcat 6060
caggccacct ttcatgtaca gaaagctgtg gaaatacggc agagtgacac agctgtacac 6120
agagtcacgc cggtatgaaa gctcatcatc tgtatcaaac ctggaatcaa agtcctcttc 6180
actatcttca aacgaggact gcgccccctc agagctgaac cggtaggcac ccgagctgtt 6240
gtaggtcccc acagagcagc ggtagtcggg ggaccactgg ctgccgtagg agttggagaa 6300
ggtcacgctg ctgccggaag tgatggcacc gtcctcatag tcatcctggt cgtagtcgta 6360
gtcgccgtct ggggagggca agtccccagc gttctggggc atgcagtagg tggactcgcc 6420
gctggaggag ttgtgtaggc tcccggagtc ctgcactgat ggggcgagca gcaccgtgct 6480
gtccgcactg ggctggtggg gcaccaccgt gtcgttcatg tatgggtcct cctctgaaga 6540
gtcatcgctg gtccggatgc cacttgttcg ctggtctgag tggccgtgct cgctggggtt 6600
gggggagtcc ttgaaggcgt cgtcgtcggc ttcgaagccc tcatcgacct cctcctctgg 6660
gtagggctgg ctgaagttga agttgggctt ctcctcgcat gcgctctcag ccagctcgct 6720
ggaaaattcg cttccccccg acaggaccg ctcgatattc cggacacact cgtcgatctc 6780
gtcgaaattg agggactcga ggaactcctg ggccgccctg cacgcttcct cctccagcct 6840
gcggagctcc tggtcccgcc gctcctgcag cttctgcagg gaagcctcgg tcagcgacag 6900

```
ctcctgctgc tccttcatgc gctgcaggtc ctcgatttct ttctccagac ggaggatctc 6960
ttccacctgc ttattttcct tctgtttctc cagttcacgg gtcagttcag cttccttctg 7020
gctcttctgc aaggcttcga gttcttgctg cttcctcgtt tcttcttcct gctgggcgcg 7080
gagctcggct tctcttcgct ctctctctct ttctctttct tcttcctccc gtttcttctt 7140
ttcttcctct tcctgtttct tcttttcttc ttgctccctt ttctctgcca gcaattgtct 7200
gtaaactctc cgagcaatct gacctctgag ttgcttctgg aaaactatgg ctgccttttt 7260
caggtgcaaa aatctcctcc tcagaaggaa tgctctgtaa ttcttctgta ttatcaccac 7320
acaataaagg acctttctgt attgtttccg tgctaagaag cccaagacat gggcccgaat 7380
caccatggcc gcgtggctca cttcctcttc cctccgcttc tccagtttct gttccaagga 7440
ttctcgaaga aataccttgg tcttccccag ctgccactcg ctgttggagg catcatagag 7500
ctgcagcagg ctcgtgcact tccctcggac gtcctcaggc agagccagat tcctcatcag 7560
cactttatac cttttgtaaa agtcctgaaa gggtcttcgg accgcatacc cagctttgcg 7620
gattctcaca gtctccagca tccctgagta ccgcagctgg ttcagcacaa ccgcctggtc 7680
aaactggtct ggcatcttct gcatgtttgg cttgatacag cgaacaaaga aaggattaga 7740
ggagcttagc gttgccatta aggaatgcag tgagtcaacc ttgaactgtg agctgactgt 7800
aggccgccga tgtttgcttc cacatttcaa ggtatcctgg ttgttgcggc ttgaaacatg 7860
ttcaaaaaga tcgtagataa agtcaaaccg gctttctctt agcaaattga gaaggtcatc 7920
tcgaaatgta tctctgttct tctccaagat acctcggaca tcatattgca cctctccagc 7980
atagtgcttc actccaaaat tgttaactgc aactctgggc ttcacataaa agtggttatt 8040
cgcatgctga ctgtgtagct tctccaataa ggtgctgtct gtggcttgag gaaaatggct 8100
ttcttcattg ataagggcta ggaggccaag tttcttctca atcaagtcca ggcattctcc 8160
attgtctatc cagtcaatat cttcccacac taatccttcc ctgctatatt ctagttgttc 8220
taaagaaaaa atatgcttgt tgaagtactc ctgaagtttc tcgtttgcat agtttatatt 8280
gaactgttca aagtgattaa cctcaaagtt ttcaaatcca aagatgtcga ggatgccaat 8340
agacttgaag tcctcattgc ctttgatcct gctgttgatc ttcttgatta cccactcaaa 8400
gcagcacgca tacagagcca tggccaggga gtccctgctg tctactgcct gttgaacatt 8460
gagaggcgtg aggatctctt ctcccctgag gaacattgat ctctgggtca aagcatctgt 8520
gagctgtgtt gggtccagcc caagtaactc cgcagatctg cccaaagctg ttttgaagga 8580
aacctgtgcc ccaccagcag tgataaattc tatgttccca agatgcagta taccagcaag 8640
cagcctcgac acttcccgaa cttcctcctt gctgaactgc atcacgtcca ttgccgtaat 8700
aacttcccta aaggattcct ggtcactgat tgtcttgtct tctacacatc cagactgatt 8760
caagtagtgg tagttttctg gcgtagataa ataaaattct tctctttctt catgttccag 8820
ccctgccagc agtgcataaa atatgtgata attcctttcc ccgggatttt gccttactac 8880
tcggttctgg gaagagagga tacaatctac aattctcccg ccctgaatat ttcctttctg 8940
acagatgttc agctgaacaa acttcccaaa gcgactagag ttgttgttgt acacggtctt 9000
cgcattgccg aaagcttcca tgatggggct gctttcaaga atagctcgtt caacacagga 9060
tgtcttctcc tttaaggaca attccaaaga ctgttgactg atgactgaca gaaacttgag 9120
gatcaattta gtgctttcgg ttttacctgc cccactttca cccttgatga ggatgcactg 9180
gttgtcgtgg cgcttccaca ggcagcggta gcactcgttg gcgatggcga agatgtgcgg 9240
gggcagctcg cccaggtggc gccggctgta ctgctccatg gtggcaggct cgtacagccc 9300
ggcgatgggc tggtaggggt tcacagaggc caggatggag ccgatgtagg tccatatttg 9360
atttctctta taccgctgga ataagttata catgatggag ccgccatgga gctctgtcaa 9420
ggacgccatg tcatccacgc cctcctcgtt cgtggggtgc atagcagtca ccttctggtg 9480
ggtaattgtg ctctgcttgt aagtgaatac ctgaccatag tctgtccgga agacgacgat 9540
gccttctgca caggaattta cagtacttgg aaaatgctgg ccatttctc tcagccagac 9600
ccgtgttccc tgtaaacaaa a                                          9621
```

<210> 12
<211> 2048
<212> PRT
<213> Homo sapiens
<400> 12

```
Phe Cys Leu Gln Gly Thr Arg Val Trp Leu Arg Glu Asn Gly Gln His
 1               5                  10                  15
```

```
Phe Pro Ser Thr Val Asn Ser Cys Ala Glu Gly Ile Val Val Phe Arg
            20                  25                  30

Thr Asp Tyr Gly Gln Val Phe Thr Tyr Lys Gln Ser Thr Ile Thr His
        35                  40                  45

Gln Lys Val Thr Ala Met His Pro Thr Asn Glu Glu Gly Val Asp Asp
    50                  55                  60

Met Ala Ser Leu Thr Glu Leu His Gly Gly Ser Ile Met Tyr Asn Leu
65                  70                  75                  80

Phe Gln Arg Tyr Lys Arg Asn Gln Ile Trp Thr Tyr Ile Gly Ser Ile
                85                  90                  95

Leu Ala Ser Val Asn Pro Tyr Gln Pro Ile Ala Gly Leu Tyr Glu Pro
            100                 105                 110

Ala Thr Met Glu Gln Tyr Ser Arg Arg His Leu Gly Glu Leu Pro Pro
        115                 120                 125

His Ile Phe Ala Ile Ala Asn Glu Cys Tyr Arg Cys Leu Trp Lys Arg
    130                 135                 140

His Asp Asn Gln Cys Ile Leu Ile Lys Gly Glu Ser Gly Ala Gly Lys
145                 150                 155                 160

Thr Glu Ser Thr Lys Leu Ile Leu Lys Phe Leu Ser Val Ile Ser Gln
                165                 170                 175

Gln Ser Leu Glu Leu Ser Leu Lys Glu Lys Thr Ser Cys Val Glu Arg
            180                 185                 190

Ala Ile Leu Glu Ser Ser Pro Ile Met Glu Ala Phe Gly Asn Ala Lys
        195                 200                 205

Thr Val Tyr Asn Asn Asn Ser Ser Arg Phe Gly Lys Phe Val Gln Leu
    210                 215                 220

Asn Ile Cys Gln Lys Gly Asn Ile Gln Gly Gly Arg Ile Val Asp Cys
225                 230                 235                 240

Ile Leu Ser Ser Gln Asn Arg Val Val Arg Gln Asn Pro Gly Glu Arg
                245                 250                 255

Asn Tyr His Ile Phe Tyr Ala Leu Leu Ala Gly Leu Glu His Glu Glu
            260                 265                 270

Arg Glu Glu Phe Tyr Leu Ser Thr Pro Glu Asn Tyr His Tyr Leu Asn
        275                 280                 285

Gln Ser Gly Cys Val Glu Asp Lys Thr Ile Ser Asp Gln Glu Ser Phe
    290                 295                 300

Arg Glu Val Ile Thr Ala Met Asp Val Met Gln Phe Ser Lys Glu Glu
305                 310                 315                 320
```

```
Val Arg Glu Val Ser Arg Leu Leu Ala Gly Ile Leu His Leu Gly Asn
                325                 330                 335

Ile Glu Phe Ile Thr Ala Gly Gly Ala Gln Val Ser Phe Lys Thr Ala
            340                 345                 350

Leu Gly Arg Ser Ala Glu Leu Leu Gly Leu Asp Pro Thr Gln Leu Thr
        355                 360                 365

Asp Ala Leu Thr Gln Arg Ser Met Phe Leu Arg Gly Glu Glu Ile Leu
    370                 375                 380

Thr Pro Leu Asn Val Gln Gln Ala Val Asp Ser Arg Asp Ser Leu Ala
385                 390                 395                 400

Met Ala Leu Tyr Ala Cys Cys Phe Glu Trp Val Ile Lys Lys Ile Asn
                405                 410                 415

Ser Arg Ile Lys Gly Asn Glu Asp Phe Lys Ser Ile Gly Ile Leu Asp
            420                 425                 430

Ile Phe Gly Phe Glu Asn Phe Glu Val Asn His Phe Glu Gln Phe Asn
        435                 440                 445

Ile Asn Tyr Ala Asn Glu Lys Leu Gln Glu Tyr Phe Asn Lys His Ile
    450                 455                 460

Phe Ser Leu Glu Gln Leu Glu Tyr Ser Arg Glu Gly Leu Val Trp Glu
465                 470                 475                 480

Asp Ile Asp Trp Ile Asp Asn Gly Glu Cys Leu Asp Leu Ile Glu Lys
                485                 490                 495

Lys Leu Gly Leu Leu Ala Leu Ile Asn Glu Glu Ser His Phe Pro Gln
            500                 505                 510

Ala Thr Asp Ser Thr Leu Leu Glu Lys Leu His Ser Gln His Ala Asn
        515                 520                 525

Asn His Phe Tyr Val Lys Pro Arg Val Ala Val Asn Asn Phe Gly Val
    530                 535                 540

Lys His Tyr Ala Gly Glu Val Gln Tyr Asp Val Arg Gly Ile Leu Glu
545                 550                 555                 560

Lys Asn Arg Asp Thr Phe Arg Asp Asp Leu Leu Asn Leu Leu Arg Glu
                565                 570                 575

Ser Arg Phe Asp Phe Ile Tyr Asp Leu Phe Glu His Val Ser Ser Arg
            580                 585                 590

Asn Asn Gln Asp Thr Leu Lys Cys Gly Ser Lys His Arg Arg Pro Thr
        595                 600                 605

Val Ser Ser Gln Phe Lys Val Asp Ser Leu His Ser Leu Met Ala Thr
    610                 615                 620
```

Leu Ser Ser Ser Asn Pro Phe Phe Val Arg Cys Ile Lys Pro Asn Met
625 630 635 640

Gln Lys Met Pro Asp Gln Phe Asp Gln Ala Val Val Leu Asn Gln Leu
645 650 655

Arg Tyr Ser Gly Met Leu Glu Thr Val Arg Ile Arg Lys Ala Gly Tyr
660 665 670

Ala Val Arg Arg Pro Phe Gln Asp Phe Tyr Lys Arg Tyr Lys Val Leu
675 680 685

Met Arg Asn Leu Ala Leu Pro Glu Asp Val Arg Gly Lys Cys Thr Ser
690 695 700

Leu Leu Gln Leu Tyr Asp Ala Ser Asn Ser Glu Trp Gln Leu Gly Lys
705 710 715 720

Thr Lys Val Phe Leu Arg Glu Ser Leu Glu Gln Lys Leu Glu Lys Arg
725 730 735

Arg Glu Glu Glu Val Ser His Ala Ala Met Val Ile Arg Ala His Val
740 745 750

Leu Gly Phe Leu Ala Arg Lys Gln Tyr Arg Lys Val Leu Tyr Cys Val
755 760 765

Val Ile Ile Gln Lys Asn Tyr Arg Ala Phe Leu Leu Arg Arg Arg Phe
770 775 780

Leu His Leu Lys Lys Ala Ala Ile Val Phe Gln Lys Gln Leu Arg Gly
785 790 795 800

Gln Ile Ala Arg Arg Val Tyr Arg Gln Leu Leu Ala Glu Lys Arg Glu
805 810 815

Gln Glu Glu Lys Lys Lys Gln Glu Glu Glu Glu Lys Lys Lys Arg Glu
820 825 830

Glu Glu Glu Arg Glu Arg Glu Arg Glu Arg Arg Glu Ala Glu Leu Arg
835 840 845

Ala Gln Gln Glu Glu Glu Thr Arg Lys Gln Gln Glu Leu Glu Ala Leu
850 855 860

Gln Lys Ser Gln Lys Glu Ala Glu Leu Thr Arg Glu Leu Glu Lys Gln
865 870 875 880

Lys Glu Asn Lys Gln Val Glu Glu Ile Leu Arg Leu Glu Lys Glu Ile
885 890 895

Glu Asp Leu Gln Arg Met Lys Glu Gln Gln Glu Leu Ser Leu Thr Glu
900 905 910

Ala Ser Leu Gln Lys Leu Gln Glu Arg Arg Asp Gln Glu Leu Arg Arg
915 920 925

Leu Glu Glu Glu Ala Cys Arg Ala Ala Gln Glu Phe Leu Glu Ser Leu
930 935 940

Asn Phe Asp Glu Ile Asp Glu Cys Val Arg Asn Ile Glu Arg Ser Leu
945 950 955 960

Ser Gly Gly Ser Glu Phe Ser Ser Glu Leu Ala Glu Ser Ala Cys Glu
965 970 975

Glu Lys Pro Asn Phe Asn Phe Ser Gln Pro Tyr Pro Glu Glu Glu Val
980 985 990

Asp Glu Gly Phe Glu Ala Asp Asp Asp Ala Phe Lys Asp Ser Pro Asn
995 1000 1005

Pro Ser Glu His Gly His Ser Asp Gln Arg Thr Ser Gly Ile Arg Thr
1010 1015 1020

Ser Asp Asp Ser Ser Glu Glu Asp Pro Tyr Met Asn Asp Thr Val Val
1025 1030 1035 1040

Pro Thr Ser Pro Ser Ala Asp Ser Thr Val Leu Leu Ala Pro Ser Val
1045 1050 1055

Gln Asp Ser Gly Ser Leu His Asn Ser Ser Ser Gly Glu Ser Thr Tyr
1060 1065 1070

Cys Met Pro Gln Asn Ala Gly Asp Leu Pro Ser Pro Asp Gly Asp Tyr
1075 1080 1085

Asp Tyr Asp Gln Asp Asp Tyr Glu Asp Gly Ala Ile Thr Ser Gly Ser
1090 1095 1100

Ser Val Thr Phe Ser Asn Ser Tyr Gly Ser Gln Trp Ser Pro Asp Tyr
1105 1110 1115 1120

Arg Cys Ser Val Gly Thr Tyr Asn Ser Ser Gly Ala Tyr Arg Phe Ser
1125 1130 1135

Ser Glu Gly Ala Gln Ser Ser Phe Glu Asp Ser Glu Glu Asp Phe Asp
1140 1145 1150

Ser Arg Phe Asp Thr Asp Asp Glu Leu Ser Tyr Arg Arg Asp Ser Val
1155 1160 1165

Tyr Ser Cys Val Thr Leu Pro Tyr Phe His Ser Phe Leu Tyr Met Lys
1170 1175 1180

Gly Gly Leu Met Asn Ser Trp Lys Arg Arg Trp Cys Val Leu Lys Asp
1185 1190 1195 1200

Glu Thr Phe Leu Trp Phe Arg Ser Lys Gln Glu Ala Leu Lys Gln Gly
1205 1210 1215

Trp Leu His Lys Lys Gly Gly Gly Ser Ser Thr Leu Ser Arg Arg Asn
1220 1225 1230

Trp Lys Lys Arg Trp Phe Val Leu Arg Gln Ser Lys Leu Met Tyr Phe
1235 1240 1245

Glu Asn Asp Ser Glu Glu Lys Leu Lys Gly Thr Val Glu Val Arg Thr
1250 1255 1260

Ala Lys Glu Ile Ile Asp Asn Thr Thr Lys Glu Asn Gly Ile Asp Ile
1265 1270 1275 1280

Ile Met Ala Asp Arg Thr Phe His Leu Ile Ala Glu Ser Pro Glu Asp
1285 1290 1295

Ala Ser Gln Trp Phe Ser Val Leu Ser Gln Val His Ala Ser Thr Asp
1300 1305 1310

Gln Glu Ile Gln Glu Met His Asp Glu Gln Ala Asn Pro Gln Asn Ala
1315 1320 1325

Val Gly Thr Leu Asp Val Gly Leu Ile Asp Ser Val Cys Ala Ser Asp
1330 1335 1340

Ser Pro Asp Arg Pro Asn Ser Phe Val Ile Ile Thr Ala Asn Arg Val
1345 1350 1355 1360

Leu His Cys Asn Ala Asp Thr Pro Glu Glu Met His His Trp Ile Thr
1365 1370 1375

Leu Leu Gln Arg Ser Lys Gly Asp Thr Arg Val Glu Gly Gln Glu Phe
1380 1385 1390

Ile Val Arg Gly Trp Leu His Lys Glu Val Lys Asn Ser Pro Lys Met
1395 1400 1405

Ser Ser Leu Lys Leu Lys Lys Arg Trp Phe Val Leu Thr His Asn Ser
1410 1415 1420

Leu Asp Tyr Tyr Lys Ser Ser Glu Lys Asn Ala Leu Lys Leu Gly Thr
1425 1430 1435 1440

Leu Val Leu Asn Ser Leu Cys Ser Val Val Pro Pro Asp Glu Lys Ile
1445 1450 1455

Phe Lys Glu Thr Gly Tyr Trp Asn Val Thr Val Tyr Gly Arg Lys His
1460 1465 1470

Cys Tyr Arg Leu Tyr Thr Lys Leu Leu Asn Glu Ala Thr Arg Trp Ser
1475 1480 1485

Ser Val Ile Gln Asn Val Thr Asp Thr Lys Ala Pro Ile Asp Thr Pro
1490 1495 1500

Thr Gln Gln Leu Ile Gln Asp Ile Lys Glu Asn Cys Leu Asn Ser Asp
1505 1510 1515 1520

Val Val Glu Gln Ile Tyr Lys Arg Asn Pro Ile Leu Arg Tyr Thr His
1525 1530 1535

His Pro Leu His Ser Pro Leu Leu Pro Leu Pro Tyr Gly Asp Ile Asn
1540 1545 1550

Leu Asn Leu Leu Lys Asp Lys Gly Tyr Thr Thr Leu Gln Asp Glu Ala
1555 1560 1565

Ile Lys Ile Phe Asn Ser Leu Gln Gln Leu Glu Ser Met Ser Asp Pro
1570 1575 1580

Ile Pro Ile Ile Gln Gly Ile Leu Gln Thr Gly His Asp Leu Arg Pro
1585 1590 1595 1600

Leu Arg Asp Glu Leu Tyr Cys Gln Leu Ile Lys Gln Thr Asn Lys Val
1605 1610 1615

Pro His Pro Gly Ser Val Gly Asn Leu Tyr Ser Trp Gln Ile Leu Thr
1620 1625 1630

Cys Leu Ser Cys Thr Phe Leu Pro Ser Arg Gly Ile Leu Lys Tyr Leu
1635 1640 1645

Lys Phe His Leu Lys Arg Ile Arg Glu Gln Phe Pro Gly Thr Glu Met
1650 1655 1660

Glu Lys Tyr Ala Leu Phe Thr Tyr Glu Ser Leu Lys Lys Thr Lys Cys
1665 1670 1675 1680

Arg Glu Phe Val Pro Ser Arg Asp Glu Ile Glu Ala Leu Ile His Arg
1685 1690 1695

Gln Glu Met Thr Ser Thr Val Tyr Cys His Gly Gly Gly Ser Cys Lys
1700 1705 1710

Ile Thr Ile Asn Ser His Thr Thr Ala Gly Glu Val Val Glu Lys Leu
1715 1720 1725

Ile Arg Gly Leu Ala Met Glu Asp Ser Arg Asn Met Phe Ala Leu Phe
1730 1735 1740

Glu Tyr Asn Gly His Val Asp Lys Ala Ile Glu Ser Arg Thr Val Val
1745 1750 1755 1760

Ala Asp Val Leu Ala Lys Phe Glu Lys Leu Ala Ala Thr Ser Glu Val
1765 1770 1775

Gly Asp Leu Pro Trp Lys Phe Tyr Phe Lys Leu Tyr Cys Phe Leu Asp
1780 1785 1790

Thr Asp Asn Val Pro Lys Asp Ser Val Glu Phe Ala Phe Met Phe Glu
1795 1800 1805

Gln Ala His Glu Ala Val Ile His Gly His His Pro Ala Pro Glu Glu
1810 1815 1820

Asn Leu Gln Val Leu Ala Ala Leu Arg Leu Gln Tyr Leu Gln Gly Asp
1825 1830 1835 1840

```
Tyr Thr Leu His Ala Ala Ile Pro Pro Leu Glu Glu Val Tyr Ser Leu
                1845                1850                1855

Gln Arg Leu Lys Ala Arg Ile Ser Gln Ser Thr Lys Thr Phe Thr Pro
            1860                1865                1870

Cys Glu Arg Leu Glu Lys Arg Arg Thr Ser Phe Leu Glu Gly Thr Leu
        1875                1880                1885

Arg Arg Ser Phe Arg Thr Gly Ser Val Val Arg Gln Lys Val Glu Glu
    1890                1895                1900

Glu Gln Met Leu Asp Met Trp Ile Lys Glu Glu Val Ser Ser Ala Arg
1905                1910                1915                1920

Ala Ser Ile Ile Asp Lys Trp Arg Lys Phe Gln Gly Met Asn Gln Glu
                1925                1930                1935

Gln Ala Met Ala Lys Tyr Met Ala Leu Ile Lys Glu Trp Pro Gly Tyr
            1940                1945                1950

Gly Ser Thr Leu Phe Asp Val Glu Val Arg Thr Gly Cys His Val Leu
        1955                1960                1965

Gly Trp Ala Gly Cys Trp His Leu Arg Thr Trp Ile Thr Ala Lys Phe
    1970                1975                1980

Met Trp Arg Glu Asp Lys Met Glu His Phe Ala Leu Ser Thr Ser Phe
1985                1990                1995                2000

Phe Arg Ala Pro Lys Ile Val Pro Leu Thr Pro Pro Phe Ser Ser Gln
                2005                2010                2015

Phe Leu Phe Ser Cys Val Val Asn Ala Ser Val Ile Leu Gly Met Asn
            2020                2025                2030

Ala Lys Leu Arg Cys His Leu Phe Phe Tyr Pro Ser Leu Gly Lys Leu
        2035                2040                2045
```

<210> 13
<211> 1288
<212> DNA
<213> Homo sapiens
<400> 13

```
ccaacttttg cagctccacc caggatgtgg cctcgctcca ccccagctgt gcgcctctct 60
ccacccctag gcgaaggcac tagaatttcc caaattaaga acgaagagga agtttggacc 120
ttttcggcca ccgctcgctt caatatggct gcccccaggg agagacgagg ctaccatgaa 180
ggagccgagc gcagaccctg agtccgtcac ccatggatcg cagcgcggag ttcaggaaat 240
ggaaggcgca atgtttgagc aaagcggacc tcagccggaa gggcagtgtt gacgaggatg 300
tggtagagct tgtgcagttt ctgaacatgc gagatcagtt tttcaccacc agctccttcg 360
ctggccgcat cctactcctt gaccggggta taaatggttt tgaggttcag aaacaaaact 420
gttgctggct actggttaca cacaaacttt gtgtaaaaga tgatgtgatt gtagctctga 480
agaaagcaaa tggtgatgcc actttgaaat ttgaaccatt tgttcttcat gtgcagtgtc 540
gacaattgca ggatgcacag attctgcatt ccatggcaat agattctggt ttcaggaact 600
ctggcataac ggtgggaaag agaggaaaaa ctatgttggc tgtccggagt acacatggct 660
tagaagttcc attaagccat aagggaaaac tgatggtgac agaggaatat attgacttcc 720
```

```
tgttaaatgt ggcaaatcaa aaaatggagg aaaacaagaa aagaattgag aggttttaca 780
actgcctaca gcatgctttg gaaagggaaa cgatgactaa cttacatccc aagatcaaag 840
agaaaaataa ctcatcatat attcataaga aaaaaagaaa cccagaaaaa acacgtgccc 900
agtgtattac taaagaaagt gatgaagaac ttgaaaatga tgatgatgat gatctaggaa 960
tcaatgttac catcttccct gaagattact aagctttggt tctgatgtgt cttggccgta 1020
atgtttctag taggttttat aaagctgctc ttcataagag tattttagtt tgttgagtgt 1080
atcagccatt cataagccag taatgacaag tgcagagctt caaactataa ctttgttgcc 1140
cagaggatgt gcagttgtca tctaagctct cagcagtacc cggcttatcc tacgacttca 1200
cctgaaatgc tatagttatc cctacttttt taccagtttc tcccagaagc acctgcttaa 1260
taaatcaaag atgtttgaaa aaaaaaaa                                    1288
```

<210> 14
<211> 259
<212> PRT
<213> Homo sapiens
<400> 14

```
Met Asp Arg Ser Ala Glu Phe Arg Lys Trp Lys Ala Gln Cys Leu Ser
 1               5                  10                  15

Lys Ala Asp Leu Ser Arg Lys Gly Ser Val Asp Glu Asp Val Val Glu
            20                  25                  30

Leu Val Gln Phe Leu Asn Met Arg Asp Gln Phe Phe Thr Thr Ser Ser
        35                  40                  45

Phe Ala Gly Arg Ile Leu Leu Leu Asp Arg Gly Ile Asn Gly Phe Glu
    50                  55                  60

Val Gln Lys Gln Asn Cys Cys Trp Leu Leu Val Thr His Lys Leu Cys
65                  70                  75                  80

Val Lys Asp Asp Val Ile Val Ala Leu Lys Lys Ala Asn Gly Asp Ala
                85                  90                  95

Thr Leu Lys Phe Glu Pro Phe Val Leu His Val Gln Cys Arg Gln Leu
            100                 105                 110

Gln Asp Ala Gln Ile Leu His Ser Met Ala Ile Asp Ser Gly Phe Arg
        115                 120                 125

Asn Ser Gly Ile Thr Val Gly Lys Arg Gly Lys Thr Met Leu Ala Val
    130                 135                 140

Arg Ser Thr His Gly Leu Glu Val Pro Leu Ser His Lys Gly Lys Leu
145                 150                 155                 160

Met Val Thr Glu Glu Tyr Ile Asp Phe Leu Leu Asn Val Ala Asn Gln
                165                 170                 175

Lys Met Glu Glu Asn Lys Lys Arg Ile Glu Arg Phe Tyr Asn Cys Leu
            180                 185                 190

Gln His Ala Leu Glu Arg Glu Thr Met Thr Asn Leu His Pro Lys Ile
        195                 200                 205

Lys Glu Lys Asn Asn Ser Ser Tyr Ile His Lys Lys Lys Arg Asn Pro
    210                 215                 220

Glu Lys Thr Arg Ala Gln Cys Ile Thr Lys Glu Ser Asp Glu Glu Leu
225                 230                 235                 240

Glu Asn Asp Asp Asp Asp Asp Leu Gly Ile Asn Val Thr Ile Phe Pro
                245                 250                 255

Glu Asp Tyr
```

<210> 15

<211> 2352
<212> DNA
<213> Homo sapiens
<220>
<221> modified_base
<222> (699)
<223> a, t, c, g, other or unknown
<400> 15

```
acgcgtgcag gtggcgtggc gccagggatt tgaaccgcgc tgacgaagtt tggtgatcca 60
tcttccgagt atcgccggga tttcgaatcg cgatgatcat cccctctcta gaggagctgg 120
actccctcaa gtacagtgac ctgcagaact tagccaagag tctgggtctc cgggccaacc 180
tgagggcaac caagttgtta aaagccttga aaggctacat taaacatgag gcaagaaaag 240
gaaatgagaa tcaggatgaa agtcaaactt ctgcatcctc ttgtgatgag actgagatac 300
agatcagcaa ccaggaagag ctgagagaca gccacttggc catgtcacca aaacaaggag 360
aaggtgcaag actgtccgtg tggaccctga ctcacagaga atcattcaga gataaaaata 420
agtaatccca ctgaattcca gaatcatgaa aagcaggaaa gccaggatct cagagcactg 480
caaaagttcc ttctccacca gacgagcacc aagaagctga gaatgctgtt tcctcaggta 540
acagagattc aaaggtacct tcagaaggaa agaaatctct ctacacagat gagtcatcca 600
aacctggaaa aaataaaaga actgcaatca ctactccaaa ctttaagaag cttcatgaag 660
ctcattttaa ggaaatggag tccattgatc caatatatng aggagaaaaa aagaaacatt 720
ttgaagaaca caattccatg aatgaactga agcagccgcc catcaataag ggagggtca 780
ggactccagt acctccaaga ggaagactct ctgtggcttc tactcccatc agccaacgac 840
gctcgcaagg ccggtcttgt ggccctgcaa gtcagagtac cttgggtctg aaggggtcac 900
tcaagcgctc tgctatctct gcagctaaaa cgggtgtcag gttttcagct gctactaaag 960
ataatgagca taagcgttca ctgaccaaga ctccagccag aaagtctgca catgtgaccg 1020
tgtctggggg cacccaaaaa ggcgaggctg tgcttgggac acacaaatta aagaccatca 1080
cggggaattc tgctgctgtt attaccccat tcaagttgac aactgaggca acgcagactc 1140
cagtctccaa taagaaacca gtgtttgatc ttaaagcaag tttgtctcgt cccctcaact 1200
atgaaccaca caaaggaaag ctaaaaccat gggggcaatc taaagaaaat aattatctaa 1260
atcaacatgt caacaaatta acttctacaa gaaaacttac aaacaacccc atctccagac 1320
aaaggaagag caacggaaga aacgcgagca agaagaaagg agaagaaagc aaaggttttg 1380
ggaatgcgaa ggggcctcat tttggctgaa gattaataat tttttaacat cttgtaaata 1440
ttcctgtatt ctcaactttt ttcctttttgt aaattttttt tttttgctgt catccccact 1500
ttagtcacga gatctttttc tgctaactgt tcatagtctg tgtagtgtcc atgggttctt 1560
catgtgctat gatctctgaa aagacgttat caccttaaag ctcaaattct ttgggatggt 1620
ttttacttaa gtccattaac aattcaggtt tctaacgaga cccatcctaa aattctcttt 1680
ctagtttttt aatgtcacca tcccaaactc ccgtttctgg atttttaatc cccagctccc 1740
cagttccctc ttatcgtact aatattaaca gaactgcagt cttctgctag ccaatagcat 1800
ttacctgatg gcagctagtt atgcaagctt caggagaatt tgaacaataa caagaatagg 1860
gtaagctggg atagaaaggc cacctcttca ctctctatag aatatagtaa cctttatgaa 1920
acggggccat atagtttggt tatgacatca atattttacc taggtgaaat tgtttaggct 1980
tatgtacctt cgttcaaata tcctcatgta attgccatct gtcactcact atattcacaa 2040

aaataaaact ctacaactca ttctaacatt gcttacttaa aagctacata gccctatcga 2100
aatgcgagga ttaatgcttt aatgctttta gagacagggt ctcactgtgt tgcccaggct 2160
ggtctcaaac tccaccaaat gtacttctta ttcattttat ggaaaagact aggctttgct 2220
tagtatcatg tccatgtttc cttcacctca gtggagcttc tgagttttat actgctcaag 2280
atcgtcataa ataaaatttt ttctcattgt caaaaaaaaa aaaaaaaaaa aaaaaaaaaa 2340
aaaaaaaaaa aa 2352
```

<210> 16
<211> 442

<212> PRT
<213> Homo sapiens
<220>
<221> MOD_RES
<222> (204)
<223> Any, other or unknown amino acid
<400> 16

```
Met Ile Ile Pro Ser Leu Glu Glu Leu Asp Ser Leu Lys Tyr Ser Asp
 1               5                  10                  15

Leu Gln Asn Leu Ala Lys Ser Leu Gly Leu Arg Ala Asn Leu Arg Ala
            20                  25                  30

Thr Lys Leu Leu Lys Ala Leu Lys Gly Tyr Ile Lys His Glu Ala Arg
        35                  40                  45

Lys Gly Asn Glu Asn Gln Asp Glu Ser Gln Thr Ser Ala Ser Ser Cys
    50                  55                  60

Asp Glu Thr Glu Ile Gln Ile Ser Asn Gln Glu Glu Ala Glu Arg Gln
 65                  70                  75                  80

Pro Leu Gly His Val Thr Lys Thr Arg Arg Arg Cys Lys Thr Val Arg
                85                  90                  95

Val Asp Pro Asp Ser Gln Gln Asn His Ser Glu Ile Lys Ile Ser Asn
            100                 105                 110

Pro Thr Glu Phe Gln Asn His Glu Lys Gln Glu Ser Gln Asp Leu Arg
        115                 120                 125

Ala Thr Ala Lys Val Pro Ser Pro Pro Asp Glu His Gln Glu Ala Glu
    130                 135                 140

Asn Ala Val Ser Ser Gly Asn Arg Asp Ser Lys Val Pro Ser Glu Gly
145                 150                 155                 160

Lys Lys Ser Leu Tyr Thr Asp Glu Ser Ser Lys Pro Gly Lys Asn Lys
                165                 170                 175

Arg Thr Ala Ile Thr Thr Pro Asn Phe Lys Lys Leu His Glu Ala His
            180                 185                 190

Phe Lys Glu Met Glu Ser Ile Asp Pro Ile Tyr Xaa Gly Glu Lys Lys
        195                 200                 205
```

Lys His Phe Glu Glu His Asn Ser Met Asn Glu Leu Lys Gln Pro Pro
210 215 220

Ile Asn Lys Gly Gly Val Arg Thr Pro Val Pro Pro Arg Gly Arg Leu
225 230 235 240

Ser Val Ala Ser Thr Pro Ile Ser Gln Arg Arg Ser Gln Gly Arg Ser
245 250 255

Cys Gly Pro Ala Ser Gln Ser Thr Leu Gly Leu Lys Gly Ser Leu Lys
260 265 270

Arg Ser Ala Ile Ser Ala Ala Lys Thr Gly Val Arg Phe Ser Ala Ala
275 280 285

Thr Lys Asp Asn Glu His Lys Arg Ser Leu Thr Lys Thr Pro Ala Arg
290 295 300

Lys Ser Ala His Val Thr Val Ser Gly Gly Thr Gln Lys Gly Glu Ala
305 310 315 320

Val Leu Gly Thr His Lys Leu Lys Thr Ile Thr Gly Asn Ser Ala Ala
325 330 335

Val Ile Thr Pro Phe Lys Leu Thr Thr Glu Ala Thr Gln Thr Pro Val
340 345 350

Ser Asn Lys Lys Pro Val Phe Asp Leu Lys Ala Ser Leu Ser Arg Pro
355 360 365

Leu Asn Tyr Glu Pro His Lys Gly Lys Leu Lys Pro Trp Gly Gln Ser
370 375 380

Lys Glu Asn Asn Tyr Leu Asn Gln His Val Asn Arg Ile Asn Phe Tyr
385 390 395 400

Lys Lys Thr Tyr Lys Gln Pro His Leu Gln Thr Lys Glu Glu Gln Arg
405 410 415

Lys Lys Arg Glu Gln Glu Arg Lys Glu Lys Lys Ala Lys Val Leu Gly
420 425 430

Met Arg Arg Gly Leu Ile Leu Ala Glu Asp
435 440

<210> 17
<211> 2058
<212> PRT
<213> Homo sapiens
<400> 17

Met Asp Asn Phe Phe Thr Glu Gly Thr Arg Val Trp Leu Arg Glu Asn
1 5 10 15

Gly Gln His Phe Pro Ser Thr Val Asn Ser Cys Ala Glu Gly Ile Val
20 25 30

```
Val Phe Arg Thr Asp Tyr Gly Gln Val Phe Thr Tyr Lys Gln Ser Thr
        35                  40                  45

Ile Thr His Gln Lys Val Thr Ala Met His Pro Thr Asn Glu Glu Gly
    50                  55                  60

Val Asp Asp Met Ala Ser Leu Thr Glu Leu His Gly Gly Ser Ile Met
65                  70                  75                  80

Tyr Asn Leu Phe Gln Arg Tyr Lys Arg Asn Gln Ile Tyr Thr Tyr Ile
                85                  90                  95

Gly Ser Ile Leu Ala Ser Val Asn Pro Tyr Gln Pro Ile Ala Gly Leu
            100                 105                 110

Tyr Glu Pro Ala Thr Met Glu Gln Tyr Ser Arg Arg His Leu Gly Glu
        115                 120                 125

Leu Pro Pro His Ile Phe Ala Ile Ala Asn Glu Cys Tyr Arg Cys Leu
    130                 135                 140

Trp Lys Arg Tyr Asp Asn Gln Cys Ile Leu Ile Ser Gly Glu Ser Gly
145                 150                 155                 160

Ala Gly Lys Thr Glu Ser Thr Lys Leu Ile Leu Lys Phe Leu Ser Val
                165                 170                 175

Ile Ser Gln Gln Ser Leu Glu Leu Ser Leu Lys Glu Lys Thr Ser Cys
            180                 185                 190

Val Glu Arg Ala Ile Leu Glu Ser Ser Pro Ile Met Glu Ala Phe Gly
        195                 200                 205

Asn Ala Lys Thr Val Tyr Asn Asn Asn Ser Ser Arg Phe Gly Lys Phe
    210                 215                 220

Val Gln Leu Asn Ile Cys Gln Lys Gly Asn Ile Gln Gly Gly Arg Ile
225                 230                 235                 240

Val Asp Tyr Leu Leu Glu Lys Asn Arg Val Val Arg Gln Asn Pro Gly
                245                 250                 255

Glu Arg Asn Tyr His Ile Phe Tyr Ala Leu Leu Ala Gly Leu Glu His
            260                 265                 270

Glu Glu Arg Glu Glu Phe Tyr Leu Ser Thr Pro Glu Asn Tyr His Tyr
        275                 280                 285

Leu Asn Gln Ser Gly Cys Val Glu Asp Lys Thr Ile Ser Asp Gln Glu
    290                 295                 300

Ser Phe Arg Glu Val Ile Thr Ala Met Asp Val Met Gln Phe Ser Lys
305                 310                 315                 320

Glu Glu Val Arg Glu Val Ser Arg Leu Leu Ala Gly Ile Leu His Leu
                325                 330                 335
```

Gly Asn Ile Glu Phe Ile Thr Ala Gly Gly Ala Gln Val Ser Phe Lys
340 345 350

Thr Ala Leu Gly Arg Ser Ala Glu Leu Leu Gly Leu Asp Pro Thr Gln
355 360 365

Leu Thr Asp Ala Leu Thr Gln Arg Ser Met Phe Leu Arg Gly Glu Glu
370 375 380

Ile Leu Thr Pro Leu Asn Val Gln Gln Ala Val Asp Ser Arg Asp Ser
385 390 395 400

Leu Ala Met Ala Leu Tyr Ala Cys Cys Phe Glu Trp Val Ile Lys Lys
405 410 415

Ile Asn Ser Arg Ile Lys Gly Asn Glu Asp Phe Lys Ser Ile Gly Ile
420 425 430

Leu Asp Ile Phe Gly Phe Glu Asn Phe Glu Val Asn His Phe Glu Gln
435 440 445

Phe Asn Ile Asn Tyr Ala Asn Glu Lys Leu Gln Glu Tyr Phe Asn Lys
450 455 460

His Ile Phe Ser Leu Glu Gln Leu Glu Tyr Ser Arg Glu Gly Leu Val
465 470 475 480

Trp Glu Asp Ile Asp Trp Ile Asp Asn Gly Glu Cys Leu Asp Leu Ile
485 490 495

Glu Lys Lys Leu Gly Leu Leu Ala Leu Ile Asn Glu Glu Ser His Phe
500 505 510

Pro Gln Ala Thr Asp Ser Thr Leu Leu Glu Lys Leu His Ser Gln His
515 520 525

Ala Asn Asn His Phe Tyr Val Lys Pro Arg Val Ala Val Asn Asn Phe
530 535 540

Gly Val Lys His Tyr Ala Gly Glu Val Gln Tyr Asp Val Arg Gly Ile
545 550 555 560

Leu Glu Lys Asn Arg Asp Thr Phe Arg Asp Asp Leu Leu Asn Leu Leu
565 570 575

Arg Glu Ser Arg Phe Asp Phe Ile Tyr Asp Leu Phe Glu His Val Ser
580 585 590

Ser Arg Asn Asn Gln Asp Thr Leu Lys Cys Gly Ser Lys His Arg Arg
595 600 605

Pro Thr Val Ser Ser Gln Phe Lys Asp Ser Leu His Ser Leu Met Ala
610 615 620

Thr Leu Ser Ser Ser Asn Pro Phe Phe Val Arg Cys Ile Lys Pro Asn
625 630 635 640

Met Gln Lys Met Pro Asp Gln Phe Asp Gln Ala Val Val Leu Asn Gln
645 650 655

Leu Arg Tyr Ser Gly Met Leu Glu Thr Val Arg Ile Arg Lys Ala Gly
660 665 670

Tyr Ala Val Arg Arg Pro Phe Gln Asp Phe Tyr Lys Arg Tyr Lys Val
675 680 685

Leu Met Arg Asn Leu Ala Leu Pro Glu Asp Val Arg Gly Lys Cys Thr
690 695 700

Ser Leu Leu Gln Leu Tyr Asp Ala Ser Asn Ser Glu Trp Gln Leu Gly
705 710 715 720

Lys Thr Lys Val Phe Leu Arg Glu Ser Leu Glu Gln Lys Leu Glu Lys
725 730 735

Arg Arg Glu Glu Glu Val Ser His Ala Ala Met Val Ile Arg Ala His
740 745 750

Val Leu Gly Phe Leu Ala Arg Lys Gln Tyr Arg Lys Val Leu Tyr Cys
755 760 765

Val Val Ile Ile Gln Lys Asn Tyr Arg Ala Phe Leu Leu Arg Arg Arg
770 775 780

Phe Leu His Leu Lys Lys Ala Ala Ile Val Phe Gln Lys Gln Leu Arg
785 790 795 800

Gly Gln Ile Ala Arg Arg Val Tyr Arg Gln Leu Leu Ala Glu Lys Arg
805 810 815

Glu Gln Glu Glu Lys Lys Lys Gln Glu Glu Glu Glu Lys Lys Lys Arg
820 825 830

Glu Glu Glu Glu Arg Glu Arg Glu Arg Glu Arg Arg Glu Ala Glu Leu
835 840 845

Arg Ala Gln Gln Glu Glu Glu Thr Arg Lys Gln Gln Glu Leu Glu Ala
850 855 860

Leu Gln Lys Ser Gln Lys Glu Ala Glu Leu Thr Arg Glu Leu Glu Lys
865 870 875 880

Gln Lys Glu Asn Lys Gln Val Glu Glu Ile Leu Arg Leu Glu Lys Glu
885 890 895

Ile Glu Asp Leu Gln Arg Met Lys Glu Gln Gln Glu Leu Ser Leu Thr
900 905 910

Glu Ala Ser Leu Gln Lys Leu Gln Glu Arg Arg Asp Gln Glu Leu Arg
915 920 925

Arg Leu Glu Glu Glu Ala Cys Arg Ala Ala Gln Glu Phe Leu Glu Ser
930 935 940

Leu Asn Phe Asp Glu Ile Asp Glu Cys Val Arg Asn Ile Glu Arg Ser
945 950 955 960

Leu Ser Val Gly Ser Glu Phe Ser Ser Glu Leu Ala Glu Ser Ala Cys
965 970 975

Glu Glu Lys Pro Asn Phe Asn Phe Ser Gln Pro Tyr Pro Glu Glu Glu
980 985 990

Val Asp Glu Gly Phe Glu Ala Asp Asp Asp Ala Phe Lys Asp Ser Pro
995 1000 1005

Asn Pro Ser Glu His Gly His Ser Asp Gln Arg Thr Ser Gly Ile Arg
1010 1015 1020

Thr Ser Asp Asp Ser Ser Glu Glu Asp Pro Tyr Met Asn Asp Thr Val
1025 1030 1035 1040

Val Pro Thr Ser Pro Ser Ala Asp Ser Thr Val Leu Leu Ala Pro Ser
1045 1050 1055

Val Gln Asp Ser Gly Ser Leu His Asn Ser Ser Ser Gly Glu Ser Thr
1060 1065 1070

Tyr Cys Met Pro Gln Asn Ala Gly Asp Leu Pro Ser Pro Asp Gly Asp
1075 1080 1085

Tyr Asp Tyr Asp Gln Asp Asp Tyr Glu Asp Gly Ala Ile Thr Ser Gly
1090 1095 1100

Ser Ser Val Thr Phe Ser Asn Ser Tyr Gly Ser Gln Trp Ser Pro Asp
1105 1110 1115 1120

Tyr Arg Cys Ser Val Gly Thr Tyr Asn Ser Ser Gly Ala Tyr Arg Phe
1125 1130 1135

Ser Ser Glu Gly Ala Gln Ser Ser Phe Glu Asp Ser Glu Glu Asp Phe
1140 1145 1150

Asp Ser Arg Phe Asp Thr Asp Asp Glu Leu Ser Tyr Arg Arg Asp Ser
1155 1160 1165

Val Tyr Ser Cys Val Thr Leu Pro Tyr Phe His Ser Phe Leu Tyr Met
1170 1175 1180

Lys Gly Gly Leu Met Asn Ser Trp Lys Arg Arg Trp Cys Val Leu Lys
1185 1190 1195 1200

Asp Glu Thr Phe Leu Trp Phe Arg Ser Lys Gln Glu Ala Leu Lys Gln
1205 1210 1215

Gly Trp Leu His Lys Lys Gly Gly Gly Ser Ser Thr Leu Ser Arg Arg
1220 1225 1230

Asn Trp Lys Lys Arg Trp Phe Val Leu Arg Gln Ser Lys Leu Met Tyr
1235 1240 1245

```
Phe Glu Asn Asp Ser Glu Glu Lys Leu Lys Gly Thr Val Glu Val Arg
   1250                1255                1260

Thr Ala Lys Glu Ile Ile Asp Asn Thr Thr Lys Glu Asn Gly Ile Asp
1265                1270                1275                1280

Ile Ile Met Ala Asp Arg Thr Phe His Leu Ile Ala Glu Ser Pro Glu
               1285                1290                1295

Asp Ala Ser Gln Trp Phe Ser Val Leu Ser Gln Val His Ala Ser Thr
           1300                1305                1310

Asp Gln Glu Ile Gln Glu Met His Asp Glu Gln Ala Asn Pro Gln Asn
       1315                1320                1325

Ala Val Gly Thr Leu Asp Val Gly Leu Ile Asp Ser Val Cys Ala Ser
   1330                1335                1340

Asp Ser Pro Asp Arg Pro Asn Ser Phe Val Ile Ile Thr Ala Asn Arg
1345                1350                1355                1360

Val Leu His Cys Asn Ala Asp Thr Pro Glu Glu Met His His Trp Ile
               1365                1370                1375

Thr Leu Leu Gln Arg Ser Lys Gly Asp Thr Arg Val Glu Gly Gln Glu
           1380                1385                1390

Phe Ile Val Arg Gly Trp Leu His Lys Glu Val Lys Asn Ser Pro Lys
       1395                1400                1405

Met Ser Ser Leu Lys Leu Lys Lys Arg Trp Phe Val Leu Thr His Asn
   1410                1415                1420

Ser Leu Asp Tyr Tyr Lys Ser Ser Glu Lys Asn Ala Leu Lys Leu Gly
1425                1430                1435                1440

Thr Leu Val Leu Asn Ser Leu Cys Ser Val Val Pro Pro Asp Glu Lys
               1445                1450                1455

Ile Phe Lys Glu Thr Gly Tyr Trp Asn Val Thr Val Tyr Gly Arg Lys
           1460                1465                1470

His Cys Tyr Arg Leu Tyr Thr Lys Leu Leu Asn Glu Ala Thr Arg Trp
       1475                1480                1485

Ser Ser Ala Ile Gln Asn Val Thr Asp Thr Lys Ala Pro Ile Asp Thr
   1490                1495                1500

Pro Thr Gln Gln Leu Ile Gln Asp Ile Lys Glu Asn Cys Leu Asn Ser
1505                1510                1515                1520

Asp Val Val Glu Gln Ile Tyr Lys Arg Asn Pro Ile Leu Arg Tyr Thr
               1525                1530                1535

His His Pro Leu His Ser Pro Leu Leu Pro Leu Pro Tyr Gly Asp Ile
           1540                1545                1550
```

Asn Leu Asn Leu Leu Lys Asp Lys Gly Tyr Thr Thr Leu Gln Asp Glu
1555 1560 1565

Ala Ile Lys Ile Phe Asn Ser Leu Gln Gln Leu Glu Ser Met Ser Asp
1570 1575 1580

Pro Ile Pro Ile Ile Gln Gly Ile Leu Gln Thr Gly His Asp Leu Arg
1585 1590 1595 1600

Pro Leu Arg Asp Glu Leu Tyr Cys Gln Leu Ile Lys Gln Thr Asn Lys
1605 1610 1615

Val Pro His Pro Gly Ser Val Gly Asn Leu Tyr Ser Trp Gln Ile Leu
1620 1625 1630

Thr Cys Leu Ser Cys Thr Phe Leu Pro Ser Arg Gly Ile Leu Lys Tyr
1635 1640 1645

Leu Lys Phe His Leu Lys Arg Ile Arg Glu Gln Phe Pro Gly Thr Glu
1650 1655 1660

Met Glu Lys Tyr Ala Leu Phe Thr Tyr Glu Ser Leu Lys Lys Thr Lys
1665 1670 1675 1680

Cys Arg Glu Phe Val Pro Ser Arg Asp Glu Ile Glu Ala Leu Ile His
1685 1690 1695

Arg Gln Glu Met Thr Ser Thr Val Tyr Cys His Gly Gly Gly Ser Cys
1700 1705 1710

Lys Ile Thr Ile Asn Ser His Thr Thr Ala Gly Glu Val Val Glu Lys
1715 1720 1725

Leu Ile Arg Gly Leu Ala Met Glu Asp Ser Arg Asn Met Phe Ala Leu
1730 1735 1740

Phe Glu Tyr Asn Gly His Val Asp Lys Ala Ile Glu Ser Arg Thr Val
1745 1750 1755 1760

Val Ala Asp Val Leu Ala Lys Phe Glu Lys Leu Ala Ala Thr Ser Glu
1765 1770 1775

Val Gly Asp Leu Pro Trp Lys Phe Tyr Phe Lys Leu Tyr Cys Phe Leu
1780 1785 1790

Asp Thr Asp Asn Val Pro Lys Asp Ser Val Glu Phe Ala Phe Met Phe
1795 1800 1805

Glu Gln Ala His Glu Ala Val Ile His Gly His His Pro Ala Pro Glu
1810 1815 1820

Glu Asn Leu Gln Val Leu Ala Ala Leu Arg Leu Gln Tyr Leu Gln Gly
1825 1830 1835 1840

Asp Tyr Thr Leu His Ala Ala Ile Pro Pro Leu Glu Glu Val Tyr Ser
1845 1850 1855

```
Leu Gln Arg Leu Lys Ala Arg Ile Ser Gln Ser Thr Lys Thr Phe Thr
            1860                1865                1870

Pro Cys Glu Arg Leu Glu Lys Arg Arg Thr Ser Phe Leu Glu Gly Thr
        1875                1880                1885

Leu Arg Arg Ser Phe Arg Thr Gly Ser Val Val Arg Gln Lys Val Glu
    1890                1895                1900

Glu Glu Gln Met Leu Asp Met Trp Ile Lys Glu Glu Val Ser Ser Ala
1905                1910                1915                1920

Arg Ala Ser Ile Ile Asp Lys Trp Arg Lys Phe Gln Gly Met Asn Gln
                1925                1930                1935

Glu Gln Ala Met Ala Lys Tyr Met Ala Leu Ile Lys Glu Trp Pro Gly
            1940                1945                1950

Tyr Gly Ser Thr Leu Phe Asp Val Glu Cys Lys Glu Gly Gly Phe Pro
        1955                1960                1965

Gln Glu Leu Trp Leu Gly Val Ser Ala Asp Ala Val Ser Val Tyr Lys
    1970                1975                1980

Arg Gly Glu Gly Arg Pro Leu Glu Val Phe Gln Tyr Glu His Ile Leu
1985                1990                1995                2000

Ser Phe Gly Ala Pro Leu Ala Asn Thr Tyr Lys Ile Val Val Asp Glu
                2005                2010                2015

Arg Glu Leu Leu Phe Glu Thr Ser Glu Val Val Asp Val Ala Lys Leu
            2020                2025                2030

Met Lys Ala Tyr Ile Ser Met Ile Val Lys Lys Arg Tyr Ser Thr Thr
        2035                2040                2045

Arg Ser Ala Ser Ser Gln Gly Ser Ser Arg
    2050                2055
```

## Description

Cities have roads and alleys, plants have xylem and phloem, and people have arteries, veins and lymphatics. Without these byways, the vertebrate animal cells would starve or drown in their metabolic refuse. Not only do blood vessels deliver food and oxygen and carry away metabolic wastes, but they also transport signaling substances that apprise cells of situations remote to them but to which they need to respond. Hormonal messages are a common signal.

All blood vessels are ensheathed by a basal lamina and a delicate monolayer of remarkably plastic endothelial cells lining the luminal walls. Depending on location and function, smooth muscle and connective tissue may also be present

Not only do healthy cells depend on the blood resources transported by the circulatory system, but so, too, unwanted cells: tumorigenic and malignant cells. These cells colonize and proliferate if they are able to divert blood resources to themselves. Angiogenesis, the type of blood vessel formation where new vessels emerge from the proliferation of preexisting vessels (Risau, 1995; Risau and Flamme, 1995), is exploited not only by usual processes, such as in wound healing or myocardial infarction repair, but also by tumors themselves and in cancers, diabetic retinopathy, macular degeneration, psoriasis, and rheumatoid arthritis. Regardless of the process, whether pathological or usual physiological, endothelial cells mediate angiogenesis in a multi-step fashion: (1) endothelia receive an extracellular cue, (2) the signaled cells breach the basal lamina sheath, abetted by proteases they secrete, (3) the cells then migrate to the signal and proliferate, and finally, (4) the cells form a tube, a morphogenic event (Alberts *et al.,* 1994). The complexity of this process indicates complex changes in cellular physiology and morphology, gene expression, and signaling. Angiogenic accomplices that are cues include basic fibroblast growth factors (bFGF), angiopoietins (such as ANG1) and various forms of vascular endothelial growth factor (VEGF).

The molecular events and the order in which they occur and the pathways that are required for this process are of fundamental importance to understand angiogenesis. *In vitro* models are useful for identifying alterations in gene expression that occur during angiogenesis. A particularly fruitful model systems involves the supspension in a three-dimensional type I collagen gel and various stimuli, such as phorbol myristate acetate (PMA), basic fibroblast growth factor (bFGF), and VEGF. The combination of the stimuli and the collagen gel results in the formation of a three-dimensional tubular network of endothelial cells with interconnecting lumenal structures. In this model, endothelial differentiation into tubelike structures is completely blocked by inhibitors of new mRNA or protein synthesis. Furthermore, the cells progress through differentiation in a coordinated and synchronized manner, thus optimizing the profile of gene expression (Kahn *et al.*, 2000; Yang *et al.*, 1999).

Tumor cells exploit angiogenesis to facilitate tumor growth. Controlling angiogenesis, by controlling the activity or expression of genes and proteins associated with angiogenesis, provides a way to prevent tumor growth, or even destoy tumors. Database EMBL (Online) 3 July 1998, Okabe, S. *et al.* Acc. No. AB011665; and Okabe, S. *et al.* Molecular and Cellular Biology, Vol. 18, No. 7, July 1998, pages 4235-4244, describes murine BAZF and its function as a transcriptional repressor.

The present invention provides an isolated polypeptide comprising an amino acid sequence having at least 90% sequence identity to the sequence of SEQ ID NO: 4, wherein expression of the polypeptide is upregulated during angiogenesis.

The present invention also provides an isolated polynucleotide comprising a nucleotide sequence having at least 80% sequence identity to the sequence of SEQ ID NO: 3, or a complement of said polynucleotide, wherein expression of the polynucleotide is upregulated during angiogenesis.

The invention is based in part upon the discovery of novel nucleic acid sequences encoding novel polypeptides. Nucleic acids encoding the polypeptides disclosed in the invention, and derivatives and fragments thereof, will hereinafter be collectively designated as "AAP" nucleic acid or polypeptide sequences. AAP, or angiogenesis associated polypeptides (AAP) comprise human ortholog of mouse BAZF (hBAZF).

In a first aspect, the present invention is an isolated polypeptide having at least 90% sequence identity to the sequence SEQ ID NO : 4. polynucleotides encoding the same, and antibodies that specifically bind the same.

In a second aspect, the present invention is an isolated polynucleotide having at least 80% sequence identity to the sequence SEQ ID NO: 3, or a complement thereof.

In a third aspect, the present invention is a transgenic non-human animal, having a disrupted *AAP* gene or a transgenic non-human animal expressing an exogenous polynucleotide having at least 80% sequence identity to the sequence SEQ ID NO : 3, or a complement of said polynucleotide.

In an eighth aspect, the present invention is a method of measuring a *AAP* transcriptional and translational up-regulation or down-regulation activity of a compound.

In a ninth aspect, the invention is a method of screening a tissue sample for tumorigenic potential.

In a tenth aspect, the invention is a method of determining the clinical stage of tumor which compares the expression of at least one AAP in a sample with expression of said at least one gene in control samples.

Although methods and materials similar or equivalent to those described herein can be used in the practice or testing of the present invention, suitable methods and materials are described below. In addition, the materials, methods, and examples are illustrative only and not intended to be limiting.

A model of angiogenesis-the suspension of endothelial cells in type I collagen gels with various stimuli-was used to identify a molecular fingerprint or transcriptional profile of endothelial differentiation into tubelike structures, using amplification and an imaging approach called GeneCalling (Shimkets *et al.,* 1999). This method was previously shown to provide a comprehensive sampling of cDNA populations in conjunction with the sensitive detection of quantitative differences in mRNA abundance for both known and novel genes. Many differentially expressed cDNA fragments were found. The identification and differential expression of these gens was confirmed by a second independent method employing real-time quantitative polymerase chain reaction (PCR). Although some of the identified cDNA fragments were genes known to play some role in angiogenesis, many other differentially expressed genes were unexpected. The inventors have identified the unexpected genes and polypeptides that are expressed in response to this model of angiogenesis, collectively refered to as angiogenesis associated polypeptides (AAP). AAP is human ortholog of mouse BAZF (hBAZF).

Unless defined otherwise, all technical and scientific terms have the same meaning as is commonly understood by one of skill in the art to which this invention belongs. The definitions below are presented for clarity.

The recommendations of (Demerec *et al.*, 1966) where these are relevant to genetics are adapted herein. To distinguish between genes (and related nucleic acids) and the proteins that they encode, the abbreviations for genes are indicated by *italicized* (or <u>underlined)</u> text while abbreviations for the proteins start with a capital letter and are not italicized. Thus, *AAP* or <u>AAP</u> refers to the nucleotide sequence that encodes AAP.

"Isolated," when referred to a molecule, refers to a molecule that has been identified and separated and/or recovered from a component of its natural environment Contaminant components of its natural environment are materials that interfere with diagnostic or therapeutic use.

"Container" is used broadly to mean any receptacle for holding material or reagent. Containers may be fabricated of glass, plastic, ceramic, metal, or any other material that can hold reagents. Acceptable materials will not react adversely with the contents.

*1. Nucleic acid-related definitions*

(a) *control sequences*

Control sequences are DNA sequences that enable the expression of an operably-linked coding sequence in a particular host organism. Prokaryotic control sequences include promoters, operator sequences, and ribosome binding sites. Eukaryotic cells utilize promoters, polyadenylation signals, and enhancers.

(b) *operably-linked*

Nucleic acid is operably-linked when it is placed into a functional relationship with another nucleic acid sequence. For example, a promoter or enhancer is operably-linked to a coding sequence if it affects the transcription of the sequence, or a ribosome-binding site is operably-linked to a coding sequence if positioned to facilitate translation. Generally, "operably-linked" means that the DNA sequences being linked are contiguous, and, in the case of a secretory leader, contiguous and in reading phase. However, enhancers do not have to be contiguous. Linking is accomplished by conventional recombinant DNA methods.

(c) *isolated nucleic acids*

An isolated nucleic acid molecule is purified from the setting in which it is found in nature and is separated from at least one contaminant nucleic acid molecule. Isolated *AAP* molecules are distinguished from the specific *AAP* molecule, as it exists in cells. However, an isolated *AAP* molecule includes *AAP* molecules contained in cells that ordinarily express an AAP where, for example, the nucleic acid molecule is in a chromosomal location different from that of natural cells.

2. *Protein-related definitions*

(a) *purified polypeptide*

When the molecule is a purified polypeptide, the polypeptide will be purified (1) to obtain at least 15 residues of N-terminal or internal amino acid sequence using a sequenator, or (2) to homogeneity by SDS-PAGE under non-reducing or reducing conditions using Coomassie blue or silver stain. Isolated polypeptides include those expressed heterologously in genetically-engineered cells or expressed *in vitro*, since at least one component of an AAP natural environment will not be present. Ordinarily, isolated polypeptides are prepared by at least one purification step.

(b) *active polypeptide*

An active AAP or AAP fragment retains a biological and/or an immunological activity of the native or naturally-occurring AAP. Immunological activity refers to the ability to induce the production of an antibody against an antigenic epitope possessed by a native AAP; biological activity refers to a function, either inhibitory or stimulatory, caused by a native AAP that excludes immunological activity. A biological activity of AAP includes, for example, modulating angiogenesis.

(c) *Abs*

Antibody may be single anti-AAP monoclonal Abs (including agonist, antagonist, and neutralizing Abs), anti-AAP antibody compositions with polyepitopic specificity, single chain anti-AAP Abs, and fragments of anti-AAP Abs. A "monoclonal antibody" refers to an antibody obtained from a population of substantially homogeneous Abs, *i.e.,* the individual Abs comprising the population are identical except for naturally-occurring mutations that may be present in minor amounts

(d) *epitope tags*

An epitope tagged polypeptide refers to a chimeric polypeptide fused to a "tag polypeptide". Such tags provide epitopes against which Abs can be made or are available, but do not interfere with polypeptide activity. To reduce anti-tag antibody reactivity with endogenous epitopes, the tag polypeptide is preferably unique. Suitable tag polypeptides generally have at least six amino acid residues and usually between about 8 and 50 amino acid residues, preferably between 8 and 20 amino acid residues). Examples of epitope tag sequences include HA from *Influenza* A virus and FLAG.

The novel *AAP* of the invention include the nucleic acid whose sequence is provided in Table 3. The invention also includes a mutant or variant *AAP,* any of whose bases may be changed from the corresponding base shown in Table 3, while still showing at least 80% identity with the polynucleotide provided in Table 3 and encoding a protein that maintains the activities and physiological functions of the AAP fragment. The invention further includes nucleic acids whose sequences are complementary to those just described. The invention additionally includes nucleic acids or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as anti-sense binding nucleic acids in therapeutic applications in a subject. In the mutant or variant nucleic acids, and their complements, up to 20% or more of the bases may be so changed.

The novel AAP of the invention include the protein fragment whose sequence is provided in Table 4. The invention also includes an AAP mutant or variant protein, any of whose residues may be changed from the corresponding residue shown in Table 4, while still showing at least 90% identity with the polypeptide of Table 4 while still encoding a protein that maintains its native activities and physiological functions. The invention further encompasses Abs and antibody fragments, such as $F_{ab}$ or $(F_{ab})'_2$, that bind immunospecifically to any of the AAP of the invention.

The AAP nucleic acid is shown in Table 3, and the corresponding polypeptides is shown in Table 4, respectively. Start and stop codons in the polynucleotide sequences are indicated in boldface and with underlining. SEQ ID NO:3 lacks a stop codon. For any lacking polynucleotide sequence, one of skill in the art may retrieve the full length sequence by, for example, probing cDNA or genomic libraries with probes designed according to the sequences of the instant invention.

**Table 3** **hBAZF nucleotide sequence (SEQ ID NO:3)**

| | | | | | | |
|---|---|---|---|---|---|---|
| caagggagcg | agggtgtcgt | agagggcaga | atgaacaaga | agaattagga | gggaggctgc | 60 |
| gtgtgccggg | gctaggggct | ggaagtcctg | gctctagttg | cacctcggaa | ggaaaaggca | 120 |
| aacagaggag | ggaaggcgtc | ttaggactgc | ctggatccag | agcactttcc | tcggcctcta | 180 |
| caggcctgtg | tcgctatggg | ttcccccgcc | gccccggagg | gagcgctggg | ctacgtccgc | 240 |
| gagttcactc | gccactcctc | cgacgtgctg | ggcaacctca | acgagctgcg | cctgcgcggg | 300 |
| atcctcactg | acgtcacgct | gctggttggc | gggcaacccc | tcagagcaca | caaggcagtt | 360 |
| ctcatcgcct | gcagtggctt | cttctattca | attttccggg | gccgtgcggg | agtcggggtg | 420 |
| gacgtgctct | ctctgcccgg | gggtcccgaa | gcgagaggct | tcgcccctct | attggacttc | 480 |
| atgtacactt | cgcgcctgcg | cctctctcca | gccactgcac | cagcagtcct | agcggccgcc | 540 |
| acctatttgc | agatggagca | cgtggtccag | gcatgccacc | gcttcatcca | ggccagctat | 600 |
| gaacctctgg | gcatctccct | gcgccccctg | gaagcagaac | cccaacacc | cccaacggcc | 660 |
| cctccaccag | gtagtcccag | gcgctccgaa | ggacacccag | acccacctac | tgaatctcga | 720 |
| agctgcagtc | aaggcccccc | cagtccagcc | agccctgacc | ccaaggcctg | caactggaaa | 780 |
| aagtacaagt | acatcgtgct | aaactctcag | gcctcccaag | cagggagcct | ggtcggggag | 840 |
| agaagttctg | gtcaaccttg | cccccaagcc | aggctcccca | gtggagacga | ggcctccagc | 900 |
| agcagcagca | gcagcagcag | cagcagcagt | gaagaaggac | ccattcctgg | tccccagagc | 960 |
| aggctctctc | caactgctgc | cactgtgcag | ttcaaatgtg | gggctccagc | cagtaccccc | 1020 |
| tacctcctca | catcccaggc | tcaagacacc | tctggatcac | cctctgaacg | ggctcgtcca | 1080 |
| ctacccggga | gtgaattttt | cagctgccag | aactgtgagg | ctgtggcagg | gtgctcatcg | 1140 |
| gggctggact | ccttggttcc | tggggacgaa | gacaaaccct | ataagtgtca | gctgtgccgg | 1200 |
| tcttcgttcc | gctacaaggg | caaccttgcc | agtcaccgta | cagtgcacac | aggggaaaag | 1260 |
| ccttaccact | gctcaatctg | cggagcccgt | tttaaccggc | cagcaaacct | gaaaacgcac | 1320 |
| agccgcatcc | attcgggaga | gaagccgtat | aagtgtgaga | cgtgcggctc | gcgctttgta | 1380 |

| | | | | | | |
|---|---|---|---|---|---|---|
| caggtacgga | gccagcctcc | aagtggcttc | caaggcaaac | ctgcaagagg | tggggtgggc | 1440 |
| caaaagggag | ggttctgttc | ctcccagagg | caggacttga | agtctcctcc | ctcccaggtg | 1500 |
| gcacatctgc | gggcgcacgt | gctgatccac | accggggaga | agccctaccc | ttgccctacc | 1560 |
| tgcggaaccc | gcttccgcca | cctgcagacc | ctcaagagcc | acgttcgcat | ccacaccgga | 1620 |
| gagaagcctt | accactgcga | cccctgtggc | ctgcatttcc | ggcacaagag | tcaactgcgg | 1680 |
| ctgcatctgc | gccagaaaca | cggagctgct | accaacacca | aagtgcacta | ccacattctc | 1740 |
| gggggggccc | | | | | | 1749 |

**Table 4** hBAZF polypeptide sequence (SEQ ID NO:4)

| | | | | | | | | | | | | | | | |
|---|---|---|---|---|---|---|---|---|---|---|---|---|---|---|---|
| Met | Gly | Ser | Pro | Ala | Ala | Pro | Glu | Gly | Ala | Leu | Gly | Tyr | Val | Arg | Glu |
| 1 | | | | 5 | | | | | 10 | | | | | 15 | |
| Phe | Thr | Arg | His | Ser | Ser | Asp | Val | Leu | Gly | Asn | Leu | Asn | Glu | Leu | Arg |
| | | | 20 | | | | | 25 | | | | | 30 | | |
| Leu | Arg | Gly | Ile | Leu | Thr | Asp | Val | Thr | Leu | Leu | Val | Gly | Gly | Gln | Pro |
| | | 35 | | | | | 40 | | | | | 45 | | | |
| Leu | Arg | Ala | His | Lys | Ala | Val | Leu | Ile | Ala | Cys | Ser | Gly | Phe | Phe | Tyr |
| | 50 | | | | | 55 | | | | | 60 | | | | |
| Ser | Ile | Phe | Arg | Gly | Arg | Ala | Gly | Val | Gly | Val | Asp | Val | Leu | Ser | Leu |
| 65 | | | | | 70 | | | | | 75 | | | | | 80 |
| Pro | Gly | Gly | Pro | Glu | Ala | Arg | Gly | Phe | Ala | Pro | Leu | Leu | Asp | Phe | Met |
| | | | | 85 | | | | | 90 | | | | | 95 | |
| Tyr | Thr | Ser | Arg | Leu | Arg | Leu | Ser | Pro | Ala | Thr | Ala | Pro | Ala | Val | Leu |
| | | | 100 | | | | | 105 | | | | | 110 | | |
| Ala | Ala | Ala | Thr | Tyr | Leu | Gln | Met | Glu | His | Val | Val | Gln | Ala | Cys | His |
| | | 115 | | | | | 120 | | | | | 125 | | | |
| Arg | Phe | Ile | Gln | Ala | Ser | Tyr | Glu | Pro | Leu | Gly | Ile | Ser | Leu | Arg | Pro |
| | 130 | | | | | 135 | | | | | 140 | | | | |
| Leu | Glu | Ala | Glu | Pro | Pro | Thr | Pro | Pro | Thr | Ala | Pro | Pro | Pro | Gly | Ser |
| 145 | | | | | 150 | | | | | 155 | | | | | 160 |
| Pro | Arg | Arg | Ser | Glu | Gly | His | Pro | Asp | Pro | Pro | Thr | Glu | Ser | Arg | Ser |
| | | | | 165 | | | | | 170 | | | | | 175 | |
| Cys | Ser | Gln | Gly | Pro | Pro | Ser | Pro | Ala | Ser | Pro | Asp | Pro | Lys | Ala | Cys |
| | | | 180 | | | | | 185 | | | | | 190 | | |
| Asn | Trp | Lys | Lys | Tyr | Lys | Tyr | Ile | Val | Leu | Asn | Ser | Gln | Ala | Ser | Gln |
| | | 195 | | | | | 200 | | | | | 205 | | | |
| Ala | Gly | Ser | Leu | Val | Gly | Glu | Arg | Ser | Ser | Gly | Gln | Pro | Cys | Pro | Gln |
| | 210 | | | | | 215 | | | | | 220 | | | | |

```
Ala Arg Leu Pro Ser Gly Asp Glu Ala Ser Ser Ser Ser Ser Ser Ser
225                 230                 235                 240

Ser Ser Ser Ser Ser Glu Glu Gly Pro Ile Pro Gly Pro Gln Ser Arg
                245                 250                 255

Leu Ser Pro Thr Ala Ala Thr Val Gln Phe Lys Cys Gly Ala Pro Ala
            260                 265                 270

Ser Thr Pro Tyr Leu Leu Thr Ser Gln Ala Gln Asp Thr Ser Gly Ser
        275                 280                 285

Pro Ser Glu Arg Ala Arg Pro Leu Pro Gly Ser Glu Phe Phe Ser Cys
    290                 295                 300

Gln Asn Cys Glu Ala Val Ala Gly Cys Ser Ser Gly Leu Asp Ser Leu
305                 310                 315                 320

Val Pro Gly Asp Glu Asp Lys Pro Tyr Lys Cys Gln Leu Cys Arg Ser
                325                 330                 335

Ser Phe Arg Tyr Lys Gly Asn Leu Ala Ser His Arg Thr Val His Thr
            340                 345                 350

Gly Glu Lys Pro Tyr His Cys Ser Ile Cys Gly Ala Arg Phe Asn Arg
        355                 360                 365

Pro Ala Asn Leu Lys Thr His Ser Arg Ile His Ser Gly Glu Lys Pro
    370                 375                 380

Tyr Lys Cys Glu Thr Cys Gly Ser Arg Phe Val Gln Val Arg Ser Gln
385                 390                 395                 400

Pro Pro Ser Gly Phe Gln Gly Lys Pro Ala Arg Gly Gly Val Gly Gln
                405                 410                 415

Lys Gly Gly Phe Cys Ser Ser Gln Arg Gln Asp Leu Lys Ser Pro Pro
            420                 425                 430

Ser Gln Val Ala His Leu Arg Ala His Val Leu Ile His Thr Gly Glu
        435                 440                 445

Lys Pro Tyr Pro Cys Pro Thr Cys Gly Thr Arg Phe Arg His Leu Gln
    450                 455                 460

Thr Leu Lys Ser His Val Arg Ile His Thr Gly Glu Lys Pro Tyr His
465                 470                 475                 480

Cys Asp Pro Cys Gly Leu His Phe Arg His Lys Ser Gln Leu Arg Leu
                485                 490                 495

His Leu Arg Gln Lys His Gly Ala Ala Thr Asn Thr Lys Val His Tyr
            500                 505                 510

His Ile Leu Gly Gly Pro
        515
```

The invention also includes polypeptides having 90, 91, 92, 93, 94, 95, 96, 97, 98 and 99%, sequence identity to SEQ ID NOS: 4, as well as nucleotides encoding any of these polypeptides, and compliments of any of these nucleotides. In an alternative embodiment, polypeptides and/or nucleotides (and compliments thereof) identical to any one of, or more than one of, SEQ ID NOS: 3 or 4 are excluded. In yet another embodiment, polypeptides and/or nucleotides (and compliments thereof) having 81-100% identical, including 82, 83, 84, 85, 86, 87, 88, 89, 90, 91, 92, 93, 94, 95, 96, 97, 98 and 99%, sequence identity to SEQ ID NOS: 3-4 are excluded.

The nucleic acids and proteins of the invention are potentially useful in promoting wound healing, for example after organ transplantation, or in the treatment of myocardial infarction, but also in treating tumors, and in cancers, diabetic retinopathy, macular degeneration, psoriasis, and rheumatoid arthritis. For example, a cDNA encoding AAP may be useful in gene therapy, and AAP proteins may be useful when administered to a subject in need thereof The novel nucleic acid encoding AAP, and the AAP proteins of the invention, or fragments thereof, may further be useful in diagnostic applications, wherein the presence or amount of the nucleic acid or the protein are to be assessed. These materials are further useful in the generation of Abs that bind immunospecifically to the novel substances of the invention for use in therapeutic or diagnostic methods.

*Human ortholog of mouse BAZF (hBAZP)*

hBAZF (SEQ ID NO:4) is the human ortholog of mouse BAZF (GenBank AB011665; SEQ ID NO:20), BAZF is a Bcl-6 (LAZ3) homolog, a transcription repressor that controls germinal center formation and the T cell-dependent immune response. Expression of Bcl-6 negatively correlates with cellular proliferation: Bcl-6 suppresses growth associated with impaired mitotic S phase progression and apoptosis (Albagli *et al.,* 1999).

BAZF contains a BTB/POZ domain and five repeats of the Kruppel-like zinc finger motifs, instead of 6 in Bcl-6 (Okabe *et al.*, 1998). Expression of *BAZF* mRNA is relegated to heart and lung, unlike *Bcl-6* mRNA, but is induced in activated lymphocytes as an immediate-early gene, like *Bcl-6* (Okabe *et al.,* 1998).

The hBAZF sequence was derived by using tblastn (protein query -translated database) (Altschul *et al.*, 1997), with the mouse protein sequence (GenBank O88282; SEQ ID NO:21) that has homology to GenBank AC015918 (SEQ ID NO:22), a clone of *Homo sapiens* chromosome 17. Human BAZF contains five Kruppel-like zinc finger motif repeats and a BTB/POZ domain.

The peptide sequence, "RSQ...PQV" that is present in the human sequence, might represent an alternative spliced form of the gene. Aligment with mouse BAZF, and aligment with mouse and human Bcl-6 demonstrates that the four proteins are almost identical in this region, but only human BAZF has this inserted sequence.

hBAZF is upregulated in HUVE cells grown embedded in collagen gels but not as a monolayer grown on collagen. When HUVE cells are suspended in collagen, they do not proliferate. Analagous to the role of mBAZF plays a role in regulating cell proliferation (Okabe *et al.,* 1998), hBAZF plays a roll in cell proliferation in HUVE suspended in collagen. Because of its high expression during vessel morphogenesis, hBAZF represents an excellent molecular marker, as well as an attractive target for various therapies to inhibit angiogenesis.

AAP *polynucleotides*

One aspect of the invention pertains to isolated nucleic acid molecules that encode AAP or biologically-active portions thereof. Also included in the invention are nucleic acid fragments sufficient for use as hybridization probes to identify AAP-encoding nucleic acids *(e.g., AAP* mRNAs) and fragments for use as polymerase chain reaction (PCR) primers for the amplification and/or mutation of *AAP* molecules. A "nucleic acid molecule" includes DNA molecules (*e.g.*, cDNA or genomic DNA), RNA molecules (*e.g.*, mRNA), analogs of the DNA or RNA generated using nucleotide analogs, and derivatives, fragments and homologs. The nucleic acid molecule may be single-stranded or double-stranded, but preferably comprises double-stranded DNA.

1. *probes*

Probes are nucleic acid sequences of variable length, preferably between at least about 10 nucleotides (nt), 100 nt, or many (*e.g.*, 6,000 nt) depending on the specific use. Probes are used to detect identical, similar, or complementary nucleic acid sequences. Longer length probes can be obtained from a natural or recombinant source, are highly specific, and much slower to hybridize than shorter-length oligomer probes. Probes may be single- or double-stranded and designed to have specificity in PCR, membrane-based hybridization technologies, or ELISA-like technologies. Probes are substantially purified oligonucleotides that will hybridize under stringent conditions to at least optimally 12, 25, 50, 100, 150, 200, 250, 300, 350 or 400 consecutive sense strand nucleotide sequence of SEQ ID NO: 3 or an anti-sense strand nucleotide sequence of these sequences; or of a naturally occurring mutant of these sequences.

The full- or partial length native sequence *AAP* may be used to "pull out" similar (homologous) sequences (Ausubel *et al.,* 1987; Sambrook, 1989), such as: (1) full-length or fragments of AAP cDNA from a cDNA library from any species (*e.g.* human, murine, feline, canine, bacterial, viral, retroviral, yeast), (2) from cells or tissues, (3) variants within a species, and (4) homologues and variants from other species. To find related sequences that may encode related genes, the probe may be designed to encode unique sequences or degenerate sequences. Sequences may also be genomic sequences including promoters, enhancer elements and introns of native sequence *AAP.*

For example, an *AAP* coding region in another species may be isolated using such probes. A probe of about 40 bases is designed, based on an *AAP,* and made. To detect hybridizations, probes are labeled using, for example, radionuclides such as $^{32}P$ or $^{35}S$, or enzymatic labels such as alkaline phosphatase coupled to the probe via avidin-biotin systems. Labeled probes are used to detect nucleic acids having a complementary sequence to that of an *AAP* in libraries of cDNA, genomic DNA or mRNA of a desired species.

Such probes can be used as a part of a diagnostic test kit for identifying cells or tissues which mis-express an AAP, such as by measuring a level of an *AAP* in a sample of cells from a subject *e.g.,* detecting *AAP* mRNA levels or determining whether a genomic *AAP* has been mutated or deleted.

2. *isolated nucleic acid*

An isolated nucleic acid molecule is separated from other nucleic acid molecules that are present in the natural source of the nucleic acid. Preferably, an isolated nucleic acid is free of sequences that naturally flank the nucleic acid (*i.e.*, sequences located at the 5'- and 3'-termini of the nucleic acid) in the genomic DNA of the organism from which the nucleic acid is derived. For example, in various embodiments, isolated *AAP* molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (*e.g.,* brain, heart, liver, spleen, *etc.*). Moreover, an isolated nucleic acid molecule, such as a cDNA molecule, can be substantially free of other cellular material or culture medium when produced by recombinant techniques, or of chemical precursors or other chemicals when chemically synthesized.

A nucleic acid molecule of the invention, *e.g.*, a nucleic acid molecule having the nucleotide sequence of SEQ ID NO: 3, or a complement of this aforementioned nucleotide sequence, can be isolated using standard molecular biology techniques and the provided sequence information. Using all or a portion of the nucleic acid sequence of SEQ ID NO : 3, as a hybridization probe, *AAP* molecules can be isolated using standard hybridization and cloning techniques (Ausubel *et al.*, 1987; Sambrook, 1989).

PCR amplification techniques can be used to amplify *AAP* using cDNA, mRNA or alternatively, genomic DNA, as a template and appropriate oligonucleotide primers. Such nucleic acids can be cloned into an appropriate vector and characterized by DNA sequence analysis. Furthermore, oligonucleotides corresponding to *AAP* sequences can be prepared by standard synthetic techniques, *e.g.,* an automated DNA synthesizer.

3. *oligonucleotide*

An oligonucleotide comprises a series of linked nucleotide residues, which oligonucleotide has a sufficient number of nucleotide bases to be used in a PCR reaction or other application. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment of the invention, an oligonucleotide comprising a nucleic acid molecule less than 100 nt in length would further comprise at least 6 contiguous nucleotides of SEQ ID NO : 3, or a complement thereof. Oligonucleotides may be chemically synthesized and may also be used as probes.

4. *complementary nucleic acid sequences; binding.*

In another embodiment, an isolated nucleic acid molecule of the invention comprises a nucleic acid molecule that is a complement of the nucleotide sequence shown in SEQ ID NO: 3, or a portion of this nucleotide sequence (*e.g.*, a fragment that can be used as a probe or primer or a fragment encoding a biologically-active portion of an AAP). A nucleic acid molecule that is complementary to the nucleotide sequence shown in SEQ ID NO : 3, is one that is sufficiently complementary to the nucleotide sequence shown in SEQ ID NO: 3, that it can hydrogen bond with little or no mismatches to the nucleotide sequence shown in SEQ ID NO : 3, thereby forming a stable duplex.

"Complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof Binding includes ionic, non-ionic, van der Waals, hydrophobic interactions, and the like. A physical interaction can be either direct or indirect Indirect interactions may be through or due to the effects of another polypeptide or compound. Direct binding refers to interactions that do not take place through, or due to, the effect of another polypeptide or compound, but instead are without other substantial chemical intermediates.

Nucleic acid fragments are at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full-length sequence. Fragments may be derived from any contiguous portion of a nucleic acid or amino acid sequence of choice.

5. *derivatives, and analogs*

Derivatives are nucleic acid sequences or amino acid sequences formed from the native compounds either directly or by modification or partial substitution. Analogs are nucleic acid sequences or amino acid sequences that have a structure similar to, but not identical to, the native compound but differ from it in respect to certain components or side chains. Analogs may be synthetic or from a different evolutionary origin and may have a similar or opposite metabolic activity compared to wild type. Homologs are nucleic acid sequences or amino acid sequences of a particular gene that are derived from different species.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions (Ausubel *et al.,* 1987).

6. *homology*

A "homologous nucleic acid sequence" or "homologous amino acid sequence," or variations thereof, refer to sequences characterized by homology at the nucleotide level or amino acid level as discussed above. Homologous nucleotide sequences encode those sequences coding for isoforms of AAP. Isoforms can be expressed in different tissues of the same organism as a result of, for example, alternative splicing of RNA. Alternatively, different genes can encode isoforms. In the invention, homologous nucleotide sequences include nucleotide sequences encoding for an AAP of species other than humans, including, but not limited to: vertebrates, and thus can include, *e.g.*, frog, mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the exact nucleotide sequence encoding human AAP. Homologous nucleic acid sequences include those nucleic acid sequences that encode conservative amino acid substitutions (see below) in SEQ ID NO: 4, as well as a polypeptide possessing AAP biological activity. Various biological activities of the AAP are described below.

7. *open reading frames*

The open reading frame (ORF) of an *AAP* gene encodes an AAP. An ORF is a nucleotide sequence that has a start codon (ATG) and terminates with one of the three "stop" codons (TAA, TAG, or TGA). In this invention, however, an ORF may be any part of a coding sequence that-may or may not comprise a start codon and a stop codon. To achieve a unique sequence, preferable *AAP* ORFs encode at least 50 amino acids.

*AAP polypeptides*

1. *mature*

An *AAP* can encode a mature AAP. A "mature" form of a polypeptide or protein disclosed in the present invention is the product of a naturally occurring polypeptide or precursor form or proprotein. The naturally occurring polypeptide, precursor or proprotein includes, by way of nonlimiting example, the full-length gene product, encoded by the corresponding gene. Alternatively, it may be defined as the polypeptide, precursor or proprotein encoded by an open reading frame described herein. The product -"mature" form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the N-terminal methionine residue encoded by the initiation codon of an open reading frame, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristoylation or phosphorylation. In general, a mature polypeptide or protein may result from the operation of only one of these processes, or a combination of any of them.

2. *active*

An active AAP polypeptide or AAP polypeptide fragment retains a biological and/or an immunological activity similar, but not necessarily identical, to an activity of a naturally-occuring (wild-type) AAP polypeptide of the invention, including mature forms. A particular biological assay, with or without dose dependency, can be used to determine AAP activity. A nucleic acid fragment encoding a biologically-active portion of AAP can be prepared by isolating a portion of SEQ ID NO: 3 that encodes a polypeptide having an AAP biological activity (the biological activities of the AAP are described below), expressing the encoded portion of AAP (*e.g.*, by recombinant expression *in vitro)* and assessing the activity of the encoded portion of AAP. Immunological activity refers to the ability to induce the production of an antibody against an antigenic epitope possessed by a native AAP; biological activity refers to a function, either inhibitory or stimulatory, caused by a native AAP that excludes immunological activity.

AAP *nucleic acid variants and hybridization*

1. *variant polynucleotides, genes and recombinant genes* The invention further encompasses nucleic acid molecules that differ from the nucleotide sequences shown in SEQ ID NO: 3, due to degeneracy of the genetic code and thus encode the same AAP as that encoded by the nucleotide sequences shown in SEQ ID NO NO:3. An isolated nucleic acid molecule of the invention has a nucleotide sequence encoding a protein having an amino acid sequence shown in SEQ ID NO : 4.

In addition to the *AAP* sequences shown in SEQ ID NO: 3, DNA sequence polymorphisms that change the amino acid sequences of the AAP may exist within a population. For example, allelic variation among individuals will exhibit genetic polymorphism in an *AAP.* The terms "gene" and "recombinant gene" refer to nucleic acid molecules comprising an open reading frame (ORF) encoding an AAP, preferably a vertebrate AAP. Such natural allelic variations can typically result in 1-5% variance in an *AAP.* Any and all such nucleotide variations and resulting amino acid polymorphisms in an AAP, which are the result of natural allelic variation and that do not alter the functional activity of an AAP are within the scope of the invention.

Moreover, *AAP* from other species that have a nucleotide sequence that differs from the human sequence of SEQ ID NO: 3, are contemplated. Nucleic acid molecules corresponding to natural allelic variants and homologues of an *AAP* cDNAs of the invention can be isolated based on their homology to an *AAP* of SEQ ID NO: 3 using cDNA-derived probes to hybridize to homologous *AAP* sequences under stringent conditions.

"AAP variant polynucleotide" or "AAP variant nucleic acid sequence" means a nucleic acid molecule which encodes an active AAP that (1) has at least about 80% nucleic acid sequence identity with a nucleotide acid sequence encoding a full-length native AAP, (2) a full-length native AAP lacking the signal peptide, (3) an extracellular domain of an AAP, with or without the signal peptide, or (4) any other fragment of a full-length AAP. Ordinarily, an AAP variant polynucleotide will have at least about 80% nucleic acid sequence identity, more preferably at least about 81%, 82%, 83%, 84%, 85%, 86%, 87%, 88%, 89%, 90%, 91%, 92%, 93%, 94%, 95%, 96%, 97%, 98% nucleic acid sequence identity and yet more preferably at least about 99% nucleic acid sequence identity with the nucleic acid sequence encoding a full-length native AAP. An AAP variant polynucleotide may encode a full-length native AAP lacking the signal peptide, an extracellular domain of an AAP, with or without the signal sequence, or any other fragment of a full-length AAP. Variants do not encompass the native nucleotide sequence.

Ordinarily, AAP variant polynucleotides are at least about 30 nucleotides in length, often at least about 60, 90,120, 150, 180, 210, 240, 270, 300, 450, 600 nucleotides in length, more often at least about 900 nucleotides in length, or more.

"Percent (%) nucleic acid sequence identity" with respect to AAP-encoding nucleic acid sequences identified herein is defined as the percentage of nucleotides in a candidate sequence that are identical with the nucleotides in the *AAP* sequence of interest, after aligning the sequences and introducing gaps, if necessary, to achieve the maximum percent sequence identity. Alignment for purposes of determining % nucleic acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

When nucleotide sequences are aligned, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) can be calculated as follows:

$$\%\text{nucleic acid sequence identity} = W/Z \cdot 100$$

where

W is the number of nucleotides cored as identical matches by the sequence alignment program's or algorithm's alignment of C and D

and

Z is the total number of nucleotides in D.

When the length of nucleic acid sequence C is not equal to the length of nucleic acid sequence D, the % nucleic acid sequence identity of C to D will not equal the % nucleic acid sequence identity ofD to C.

2. *Stringency*

Homologs *(i.e.,* nucleic acids encoding an AAP derived from species other than human) or other related sequences (*e.g.*, paralogs) can be obtained by low, moderate or high stringency hybridization with all or a portion of the particular human sequence as a probe using methods well known in the art for nucleic acid hybridization and cloning.

The specificity of single stranded DNA to hybridize complementary fragments is determined by the "stringency" of the reaction conditions. Hybridization stringency increases as the propensity to form DNA duplexes decreases. In nucleic acid hybridization reactions, the stringency can be chosen to either favor specific hybridizations (high stringency), which can be used to identify, for example, full-length clones from a library. Less-specific hybridizations (low stringency) can be used to identify related, but not exact, DNA molecules (homologous, but not identical) or segments.

DNA duplexes are stabilized by: (1) the number of complementary base pairs, (2) the type of base pairs, (3) salt concentration (ionic strength) of the reaction mixture, (4) the temperature of the reaction, and (5) the presence of certain organic solvents, such as formamide which decreases DNA duplex stability. In general, the longer the probe, the higher the temperature required for proper annealing. A common approach is to vary the temperature: higher relative temperatures result in more stringent reaction conditions. (Ausubel *et al.,* 1987) provide an excellent explanation of stringency of hybridization reactions.

To hybridize under "stringent conditions" describes hybridization protocols in which nucleotide sequences at least 60% homologous to each other remain hybridized. Generally, stringent conditions are selected to be about 5°C lower than the thermal melting point (Tm) for the specific sequence at a defined ionic strength and *p*H. The Tm is the temperature (under defined ionic strength, *p*H and nucleic acid concentration) at which 50% of the probes complementary to the target sequence hybridize to the target sequence at equilibrium. Since the target sequences are generally present at excess, at Tm, 50% of the probes are occupied at equilibrium.

(a) *high stringency*

"Stringent hybridization conditions" conditions enable a probe, primer or oligonucleotide to hybridize only to its target sequence. Stringent conditions are sequence-dependent and will differ. Stringent conditions comprise: (1) low ionic strength and high temperature washes (*e.g.* 15 mM sodium chloride, 1.5 mM sodium citrate, 0.1 % sodium dodecyl sulfate at 50°C); (2) a denaturing agent during hybridization (*e.g.* 50% (v/v) formamide, 0.1% bovine serum albumin, 0.1% Ficoll, 0.1% polyvinylpyrrolidone, 50mM sodium phosphate buffer (pH 6.5; 750 mM sodium chloride, 75 mM sodium citrate at 42°C); or (3) 50% formamide. Washes typically also comprise 5X SSC (0.75 M NaCl, 75 mM sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C. Preferably, the conditions are such that sequences at least about 65%, 70%, 75%, 85%, 90%, 95%, 98%, or 99% homologous to each other typically remain hybridized to each other. These conditions are presented as examples and are not meant to be limiting.

(b) *moderate stringency*

"Moderately stringent conditions" use washing solutions and hybridization conditions that are less stringent (Sambrook, 1989), such that a polynucleotide will hybridize to the entire, fragments, derivatives or analogs of SEQ ID NO: 3. One example comprises hybridization in 6X SSC, 5X Denhardt's solution, 0.5% SDS and 100 mg/ml denatured salmon sperm DNA at 55°C, followed by one or more washes in 1X SSC, 0.1% SDS at 37°C. The temperature, ionic strength, *etc*., can be adjusted to accommodate experimental factors such as probe length Other moderate stringency conditions are described in (Ausubel *et al,* 1987; Kriegler, 1990).

(c) *low stringency*

"Low stringent conditions" use washing solutions and hybridization conditions that are less stringent than those for moderate stringency (Sambrook, 1989), such that a polynucleotide will hybridize to the entire, fragments, derivatives or analogs of SEQ ID NO : 3. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10%,(wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency, such as those for cross-species hybridizations are described in (Ausubel *et al.,* 1987; Kriegler, 1990; Shilo and Weinberg, 1981).

*3. Conservative mutations*

In addition to naturally-occurring allelic variants of *AAP,* changes can be introduced by mutation into SEQ ID NO NO: 3 sequences that incur alterations in the amino acid sequences of the encoded AAP that do not alter the AAP function For example, nucleotide substitutions leading to amino acid substitutions at "non-essential" amino acid residues can be made in the sequence of SEQ ID NO: 4. A "non-essential" amino acid residue is a residue that can be altered from the wild-type sequences of the AAP without altering their biological activity, whereas an "essential" amino acid residue is required for such biological activity. For example, amino acid residues that are conserved among the AAP of the invention are predicted to be particularly non-amenable to alteration. Amino acids for which conservative substitutions can be made are well-known in the art.

Useful conservative substitutions are shown in Table A, "Preferred substitutions." Conservative substitutions whereby an amino acid of one class is replaced with another amino acid of the same type fall within the scope of the subject invention so long as the substitution does not materially alter the biological activity of the compound. If such substitutions result in a change in biological activity, then more substantial changes, indicated in Table B as exemplary are introduced and the products screened for an AAP polypeptide's biological activity.

Table A Preferred substitutions

| Original residue | Exemplary substitutions | Preferred substitutions |
|---|---|---|
| Ala (A) | Val, Leu, Ile | Val |
| Arg (R) | Lys, Gln, Asn | Lys |
| Asn (N) | Gln, His, Lys, Arg | Gln |
| Asp (D) | Glu | Glu |
| Cys (C) | Ser | Ser |
| Gln (Q) | Asn | Asn |
| Glu (E) | Asp | Asp |
| Gly (G) | Pro, Ala | Ala |
| His (H) | Asn, Gln, Lys, Arg | Arg |
| Ile (I) | Leu, Val, Met, Ala, Phe, Norleucine | Leu |
| Leu (L) | Norleucine, Ile, Val, Met, Ala, Phe | Ile |
| Lys (K) | Arg, Gln, Asn | Arg |
| Met (M) | Leu, Phe, Ile | Leu |
| Phe (F) | Leu, Val, Ile, Ala, Tyr | Leu |

(continued)

| Original residue | Exemplary substitutions | Preferred substitutions |
|---|---|---|
| Pro (P) | Ala | Ala |
| Ser (S) | Thr | Thr |
| Thr (T) | Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Norleucine | Leu |

Non-conservative substitutions that effect (1) the structure of the polypeptide backbone, such as a β-sheet or α-helical conformation, (2) the charge or (3) hydrophobicity, or (4) the bulk of the side chain of the target site can modify an AAP polypeptide's function or immunological identity. Residues are divided into groups based on common side-chain properties as denoted in Table B. Non-conservative substitutions entail exchanging a member of one of these classes for another class. Substitutions may be introduced into conservative substitution sites or more preferably into non-conserved sites.

Table B Amino acid classes

| Class | Amino acids |
|---|---|
| hydrophobic | Norleucine, Met, Ala, Val, Leu, Ile |
| neutral hydrophilic | Cys, Ser, Thr |
| acidic | Asp, Glu |
| basic | Asn, Gln, His, Lys, Arg |
| disrupt chain conformation | Gly, Pro |
| aromatic | Trp, Tyr, Phe |

The variant polypeptides can be made using methods known in the art such as oligonucleotide-mediated (site-directed) mutagenesis, alanine scanning, and PCR mutagenesis. Site-directed mutagenesis (Carter, 1986; Zoller and Smith, 1987), cassette mutagenesis, restriction selection mutagenesis (Wells *et al.,* 1985) or other known techniques can be performed on the cloned DNA to produce the AAP variant DNA (Ausubel *et al.,* 1987; Sambrook, 1989).

In one embodiment, the isolated nucleic acid molecule comprises a nucleotide sequence encoding a protein, wherein the protein comprises an amino acid sequence at least about 45%, preferably 60%, more preferably 70%, 80%, 90%, and most preferably about 95% homologous to SEQ ID NO: 3.

A mutant AAP can be assayed for blocking angiogenesis *in vitro.*

4. *Anti-sense nucleic acids*

Using antisense and sense AAP oligonucleotides can prevent AAP polypeptide expression. These oligonucleotides bind to target nucleic acid sequences, forming duplexes that block transcription or translation of the target sequence by enhancing degradation of the duplexes, terminating prematurely transcription or translation, or by other means.

Antisense or sense oligonucleotides are singe-stranded nucleic acids, either RNA or DNA, which can bind a target *AAP* mRNA (sense) or an *AAP* DNA (antisense) sequences. Anti-sense nucleic acids can be designed according to Watson and Crick or Hoogsteen base pairing rules. The anti-sense nucleic acid molecule can be complementary to the entire coding region of an *AAP* mRNA, but more preferably, to only a portion of the coding or noncoding region of an *AAP* mRNA. For example, the anti-sense oligonucleotide can be complementary to the region surrounding the translation start site of an *AAP* mRNA. Antisense or sense oligonucleotides may comprise a fragment of the AAP DNA coding region of at least about 14 nucleotides, preferably from about 14 to 30 nucleotides. In general, antisense RNA or DNA molecules can comprise at least 5, 10, 15, 20, 25, 30, 35, 40, 45, 50, 55, 60, 65, 70, 75, 80, 85, 90, 95,100 bases in length or more. Among others, (Stein and Cohen, 1988; van der Krol *et al.,* 1988a) describe methods to derive antisense or a sense oligonucleotides from a given cDNA sequence.

Examples of modified nucleotides that can be used to generate the anti-sense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine; 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the anti-sense nucleic acid can be produced biologically using an expression vector into which a nucleic acid has been subcloned in an anti-sense orientation such that the transcribed RNA will be complementary to a target nucleic acid of interest.

To introduce antisense or sense oligonucleotides into target cells (cells containing the target nucleic acid sequence), any gene transfer method may be used. Examples of gene transfer methods include (1) biological, such as gene transfer vectors like Epstein-Barr virus or conjugating the exogenous DNA to a ligand-binding molecule, (2) physical, such as electroporation and injection, and (3) chemical, such as $CaPO_4$ precipitation and oligonucleotide-lipid complexes.

An antisense or sense oligonucleotide is inserted into a suitable gene transfer retroviral vector. A cell containing the target nucleic acid sequence is contacted with the recombinant retroviral vector, either *in vivo* or *ex vivo*. Examples of suitable retroviral vectors include those derived from the murine retrovirus M-MuLV, N2 (a retrovirus derived from M-MuLV), or the double copy vectors designated DCT5A, DCT5B and DCT5C (WO 90/13641,1990). To achieve sufficient nucleic acid molecule transcription, vector constructs in which the transcription of the anti-sense nucleic acid molecule is controlled by a strong pol II or pol III promoter are preferred.

To specify target cells in a mixed population of cells cell surface receptors that are specific to the target cells can be exploited. Antisense and sense oligonucleotides are conjugated to a ligand-binding molecule, as described in (WO 91/04753, 1991). Ligands are chosen for receptors that are specific to the target cells. Examples of suitable ligand-binding molecules include cell surface receptors, growth factors, cytokines, or other ligands that bind to cell surface receptors or molecules. Preferably, conjugation of the ligand-binding molecule does not substantially interfere with the ability of the receptors or molecule to bind the ligand-binding molecule conjugate, or block entry of the sense or antisense oligonucleotide or its conjugated version into the cell.

Liposomes efficiently transfer sense or an antisense oligonucleotide to cells (WO 90/10448, 1990). The sense or antisense oligonucleotide-lipid complex is preferably dissociated within the cell by an endogenous lipase.

The anti-sense nucleic acid molecule of the invention may be an $\alpha$-anomeric nucleic acid molecule. An $\alpha$-anomeric nucleic acid molecule forms specific double-stranded hybrids with complementary RNA in which, contrary to the usual $\alpha$-units, the strands run parallel to each other (Gautier *et al.*, 1987). The anti-sense nucleic acid molecule can also comprise a 2'-o-methylribonucleotide (Inoue *et al.,* 1987a) or a chimeric RNA-DNA analogue (Inoue *et al.,* 1987b).

In one embodiment, an anti-sense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes, such as hammerhead ribozymes (Haseloff and Gerlach, 1988) can be used to catalytically cleave *AAP* mRNA transcripts and thus inhibit translation. A ribozyme specific for an AAP-encoding nucleic acid can be designed based on the nucleotide sequence of an *AAP* cDNA (*i.e.*, SEQ ID NO: 3). For example, a derivative of a *Tetrahymena* L-19 IVS RNA can be constructed in which the nucleotide sequence of the active site is complementary to the nucleotide sequence to be cleaved in an AAP-encoding mRNA (Cech *et al.,* U.S. Patent No. 5,116,742, 1992; Cech *et al.,* U.S. Patent No. 4,987,071, 1991). An *AAP* mRNA can also be used to select a catalytic RNA having a specific ribonuclease activity from a pool of RNA molecules (Bartel and Szostak, 1993).

Alternatively, *AAP* expression can be inhibited by targeting nucleotide sequences complementary to the regulatory region of an *AAP* (*e.g.*, an *AAP* promoter and/or enhancers) to form triple helical structures that prevent transcription of an *AAP* in target cells (Helene, 1991; Helene *et al.,* 1992; Maher, 1992).

Modifications of antisense and sense oligonucleotides can augment their effectiveness. Modified sugar-phosphodiester bonds or other sugar linkages (WO 91/06629, 1991), increase *in vivo* stability by conferring resistance to endogenous nucleases without disrupting binding specificity to target sequences. Other modifications can increase the affinities of the oligonucleotides for their targets, such as covalently linked organic moieties (WO 90/10448, 1990) or poly-(L)-lysine. Other attachments modify binding specificities of the oligonucleotides for their targets, including metal complexes or intercalating (*e.g.* ellipticine) and alkylating agents.

For example, the deoxyribose phosphate backbone of the nucleic acids can be modified to generate peptide nucleic acids (Hyrup and Nielsen, 1996). "Peptide nucleic acids" or "PNAs" refer to nucleic acid mimics (*e.g.*, DNA mimics) in that the deoxyribose phosphate backbone is replaced by a pseudopeptide backbone and only the four natural nucleobases are retained The neutral backbone of PNAs allows for specific hybridization to DNA and RNA under conditions of low ionic strength The synthesis of PNA oligomers can be performed using standard solid phase peptide synthesis protocols (Hyrup and Nielsen, 1996; Perry-O'Keefe *et al.* 1996).

PNAs of an AAP can be used in therapeutic and diagnostic applications. For example, PNAs can be used as anti-sense or antigene agents for sequence-specific modulation of gene expression by inducing transcription or translation arrest or inhibiting replication. AAP PNAs may also be used in the analysis of single base pair mutations (*e.g.*, PNA directed PCR clamping; as artificial restriction enzymes when used in combination with other enzymes, *e.g.*, $S_1$ nucleases (Hyrup and Nielsen, 1996); or as probes or primers for DNA sequence and hybridization (Hyrup and Nielsen, 1996; Perry-O'Keefe *et al.,* 1996).

PNAs of an AAP can be modified to enhance their stability or cellular uptake. Lipophilic or other helper groups may be attached to PNAs, PNA-DNA dimmers formed, or the use of liposomes or other drug delivery techniques. For example, PNA-DNA chimeras can be generated that may combine the advantageous properties of PNA and DNA. Such chimeras allow DNA recognition enzymes *(e.g.,* RNase H and DNA polymerases) to interact with the DNA portion while the PNA portion provides high binding affinity and specificity. PNA-DNA chimeras can be linked using linkers of appropriate lengths selected in terms of base stacking, number of bonds between the nucleobases, and orientation (Hyrup and Nielsen, 1996). The synthesis of PNA-DNA chimeras can be performed (Finn *et al.,* 1996; Hyrup and Nielsen, 1996). For example, a DNA chain can be synthesized on a solid support using standard phosphoramidite coupling chemistry, and modified nucleoside analogs, *e.g.,* 5'-(4-methoxytrityl)amino-5'-deoxy-thymidine phosphoramidite, can be used between the PNA and the 5' end of DNA (Finn *et al.,* 1996; Hyrup and Nielsen, 1996). PNA monomers are then coupled in a stepwise manner to produce a chimeric molecule with a 5' PNA segment and a 3' DNA segment (Finn *et al.,* 1996). Alternatively, chimeric molecules can be synthesized with a 5' DNA segment and a 3' PNA segment (Petersen *et al.*,1976).

The oligonucleotide may include other appended groups such as peptides (*e.g.*, for targeting host cell receptors *in vivo),* or agents facilitating transport across the cell membrane (Lemaitre *et al.,* 1987; Letsinger *et al.,* 1989) or PCT Publication No. WO88/09810) or the blood-brain barrier (*e.g.*, PCT Publication No. WO 89/10134). In addition, oligonucleotides can be modified with hybridization-triggered cleavage agents (van der Krol *et al.,* 1988b) or intercalating agents (Zon, 1988). The oligonucleotide may be conjugated to another molecule, *e.g.*, a peptide, a hybridization triggered cross-linking agent, a transport agent, a hybridization-triggered cleavage agent, and the like.

*AAP polypeptides*

One aspect of the invention pertains to isolated AAP, and biologically-active portions derivatives, fragments, analogs or homologs thereof. Also provided are polypeptide fragments suitable for use as immunogens to raise anti-AAP Abs. In one embodiment, a native AAP can be isolated from cells or tissue sources by an appropriate purification scheme using standard protein purification techniques. In another embodiment, AAP are produced by recombinant DNA techniques. Alternative to recombinant expression, an AAP can be synthesized chemically using standard peptide synthesis techniques.

1. *Polypeptides*

An AAP polypeptide includes the amino acid sequence of an AAP whose sequences are provided in SEQ ID NO: 4. The invention also includes a mutant or variant protein any of whose residues may be changed from the corresponding residues shown in SEQ ID NO: 4, while still encoding a protein that maintains its AAP activities and physiological functions, or a functional fragment thereof

*2. Variant AAP polypeptides*

In general, an AAP variant that preserves an AAP-like function and includes any variant in which residues at a particular position in the sequence have been substituted by other amino acids, and further includes the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

90% "AAP polypeptide variant" means an active AAP polypeptide having at least: (1) about amino acid sequence identity with a full-length native sequence AAP polypeptide sequence, (2) an AAP polypeptide sequence lacking the signal peptide, (3) an extracellular domain of an AAP polypeptide, with or without the signal peptide, or (4) any other fragment of a full-length AAP polypeptide sequence. For example, AAP polypeptide variants include AAP polypeptides wherein one or more amino acid residues are added or deleted at the N- or C- terminus of the full-length native amino acid sequence. An AAP polypeptide variant will have at least about 90% amino acid sequence identity, preferably at least 91%, 92%, 93%, 94%, 95%, 96%, 97%, 98% amino acid sequence identity and most preferably at least about 99% amino acid sequence identity with a full-length native sequence AAP polypeptide sequence. An AAP polypeptide variant may have a sequence lacking the signal peptide, an extracellular domain of an AAP polypeptide, with or without the signal peptide, or any other fragment of a full-length AAP polypeptide sequence. Ordinarily, AAP variant polypeptides are at least about 10 amino acids in length, often at least about 20 amino acids in length, more often at least about 30, 40, 50, 60, 70, 80, 90,100, 150, 200, or 300 amino acids in length, or more.

"Percent (%) amino acid sequence identity" is defined as the percentage of amino acid residues that are identical with amino acid residues in a disclosed AAP polypeptide sequence in a candidate sequence when the two sequences are aligned. To determine % amino acid identity, sequences are aligned and if necessary, gaps are introduced to achieve the maximum % sequence identity, conservative substitutions are not considered as part of the sequence identity. Amino acid sequence alignment procedures to determine percent identity are well known to those of skill in the art. Often publicly available computer software such as BLAST, BLAST2, ALIGN2 or Megalign (DNASTAR) software is used to align peptide sequences. Those skilled in the art can determine appropriate parameters for measuring alignment, including any algorithms needed to achieve maximal alignment over the full length of the sequences being compared.

When amino acid sequences are aligned, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) can be calculated as:

$$\%\text{amino acid sequence identity} = X/Y \cdot 100$$

where
X is the number of amino acid residues scored as identical matches by the sequence alignment program's or algorithm's alignment of A and B
and
Y is the total number of amino acid residues in B.

If the length of amino acid sequence A is not equal to the length of amino acid sequence B, the % amino acid sequence identity of A to B will not equal the % amino acid sequence identity of B to A.

*3. Isolated/purified polypeptides*

An "isolated" or "purified" polypeptide, protein or biologically active fragment is separated and/or recovered from a component of its natural environment. Contaminant components include materials that would typically interfere with diagnostic or therapeutic uses for the polypeptide, and may include enzymes, hormones, and other proteinaceous or non-proteinaceous materials. Preferably, the polypeptide is purified to a sufficient degree to obtain at least 15 residues of N-terminal or internal amino acid sequence. To be substantially isolated, preparations having less than 30% by dry weight of non-AAP contaminating material (contaminants), more preferably less than 20%, 10% and most preferably less than 5% contaminants. An isolated, recombinantly-produced AAP or biologically active portion is preferably substantially free of culture medium, *i.e.,* culture medium represents less than 20%, more preferably less than about 10%, and most preferably less than about 5% of the volume of the AAP preparation. Examples of contaminants include cell debris, culture media, and substances used and produced during *in vitro* synthesis of an AAP.

*4. Biologically active*

Biologically active portions of an AAP include peptides comprising amino acid sequences sufficiently homologous to or derived from the amino acid sequences of an AAP (SEQ ID NO: 4) that include fewer amino acids than a full-length AAP, and exhibit at least one activity of an AAP. Biologically active portions comprise a domain or motif with at least one activity of a native AAP. A biologically active portion of an AAP can be a polypeptide that is, for example, 10, 25, 50, 100 or more amino acid residues in length. Other biologically active portions, in which other regions of the protein are deleted, can be prepared by recombinant techniques and evaluated for one or more of the functional activities of a native AAP.

Biologically active portions of an AAP may have an amino acid sequence shown in SEQ ID NO:4, or substantially homologous to SEQ ID NO: 4, and retains the functional activity of the protein of SEQ ID NO: 4, yet differs in amino acid sequence due to natural allelic variation or mutagenesis. Other biologically active AAP may comprise an amino acid sequence at least 45% homologous to the amino acid sequence of SEQ ID NO: 4, and retains the functional activity of native AAP.

5. *Determining homology between two or more sequences*

"AAP variant" means an active AAP having at least: (1) about 80% amino acid sequence identity with a full-length native sequence AAP sequence, (2) an AAP sequence lacking the signal peptide, (3) an extracellular domain of an AAP, with or without the signal peptide, or (4) any other fragment of a full-length AAP sequence. For example, AAP variants include an AAP wherein one or more amino acid residues are added or deleted at the N- or C- terminus of the full-length native amino acid sequence. An AAP variant will have at least about 80% amino acid sequence identity, preferably at least about 81% amino acid sequence identity, more preferably at least about 82%, 83%, 84%, 85%, 86%, 87%, 88%, 89%, 90%, 91%, 92%, 93%, 94%, 95%, 96%, 97%, 98% amino acid sequence identity and most preferably at least about 99% amino acid sequence identity with a full-length native sequence AAP sequence. An AAP variant may have a sequence lacking the signal peptide, an extracellular domain of an AAP, with or without the signal peptide, or any other fragment of a full-length AAP sequence. Ordinarily, AAP variant polypeptides are at least about 10 amino acids in length, often at least about 20 amino acids in length, more often at least about 30, 40, 50, 60, 70, 80, 90,100, 150, 200, or 300 amino acids in length, or more.

"Percent (%) amino acid sequence identity" is defined as the percentage of amino acid residues that are identical with amino acid residues in a disclosed AAP sequence in a candidate sequence when the two sequences are aligned. To determine % amino acid identity, sequences are aligned and if necessary, gaps are introduced to achieve the maximum % sequence identity; conservative substitutions are not considered as part of the sequence identity. Amino acid sequence alignment procedures to determine percent identity are well known to those of skill in the art. Often publicly available computer software such as BLAST, BLAST2, ALIGN2 or Megalign (DNASTAR) software is used to align peptide sequences. Those skilled in the art can determine appropriate parameters for measuring alignment, including any algorithms needed to achieve maximal alignment over the full length of the sequences being compared.

When amino acid sequences are aligned, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) can be calculated as:

$$\%\text{amino acid sequence identity} = X/Y \cdot 100$$

where

X is the number of amino acid residues scored as identical matches by the sequence alignment program's or algorithm's alignment of A and B

and

Y is the total number of amino acid residues in B.

If the length of amino acid sequence A is not equal to the length of amino acid sequence B, the % amino acid sequence identity of A to B will not equal the % amino acid sequence identity of B to A.

6. *Chimeric and fusion proteins*

Fusion polypeptides are useful in expression studies, cell-localization, bioassays, and AAP purification. An AAP "chimeric protein" or "fusion protein" comprises an AAP fused to a non-AAP polypeptide. A non-AAP polypeptide is not substantially homologous to an AAP (SEQ ID NO: 4). An AAP fusion protein may include any portion to an entire AAP, including any number of the biologically active portions. An AAP may be fused to the C-terminus of the GST (glutathione S-transferase) sequences. Such fusion proteins facilitate the purification of a recombinant AAP. In certain host cells, *(e.g.* mammalian), heterologous signal sequences fusions may ameliorate AAP expression and/or secretion. Additional exemplary fusions are presented in Table C.

Other fusion partners can adapt an AAP therapeutically. Fusions with members of the immunoglobulin (Ig) protein family are useful in therapies that inhibit an AAP ligand or substrate interactions, consequently suppressing an AAP-mediated signal transduction *in vivo.* Such fusions, incorporated into pharmaceutical compositions, may be used to treat proliferative and differentiation disorders, as well as modulating cell survival. An AAP-Ig fusion polypeptides can also be used as immunogens to produce an anti-AAP Abs in a subject, to purify AAP ligands, and to screen for molecules that inhibit interactions of an AAP with other molecules.

Fusion proteins can be easily created using recombinant methods. A nucleic acid encoding an AAP can be fused in-frame with a non-AAP encoding nucleic acid, to an AAP $NH_2$- or COO- -terminus, or internally. Fusion genes may also be synthesized by conventional techniques, including automated DNA synthesizers. PCR amplification using anchor primers that give rise to complementary overhangs between two consecutive gene fragments that can subsequently be annealed and reamplified to generate a chimeric gene sequence (Ausubel *et al.*, 1987) is also useful. Many vectors are commercially available that facilitate sub-cloning an *AAP* in-frame to a fusion moiety.

Table C Useful non-AAP fusion polypeptides

| Reporter | *in vitro* | *in vivo* | Notes | Reference |
|---|---|---|---|---|
| Human growth hormone (hGH) | Radioimmuno-assay | none | Expensive, insensitive, narrow linear range. | (Selden *et al.,* 1986) |
| β-glucuronidase (GUS) | Colorimetric, fluorescent, or chemiluminescent | colorimetric (histo-chemical staining with X- gluc) | sensitive, broad linear range, non-iostopic. | (Gallagher, 1992) |
| Green fluorescent protein (GFP) and related molecules (RFP, BFP, AAP, *etc.)* | Fluorescent | fluorescent ’ | can be used in live cells; resists photo-bleaching | (Chalfie *et al.,* 1994) |
| Luciferase (firefly) | bioluminsecent | Bio-luminescent | protein is unstable, difficult to reproduce, signal is brief | (de Wet *et al.,* 1987) |
| Chloramphenico al acetyltransferas e (CAT) | Chromatography, differential extraction, fluorescent, or immunoassay | none | Expensive radioactive substrates, time-consuming, insensitive, narrow linear range | (Gorman *et al.,* 1982) |
| β-galacto-sidase | colorimetric, fluorescence, chemiluminscence | colorimetric (histochemical staining with X-gal), bio-luminescent in live cells | sensitive, broad linear range; some cells have high endogenous activity | (Alam and Cook, 1990) |
| Secrete alkaline phosphatase (SEAP) | colorimetric, bioluminescent, chemiluminescent | none | Chemiluminscence assay is sensitive and broad linear range; some cells have endogenouse alkaline phosphatase activity | (Berger *et al.,* 1988) |

*Therapeutic applications of AAP*

1. *Agonists and antagonists*

"Antagonist" includes any molecule that partially or fully blocks, inhibits, or neutralizes a biological activity of an endogenous AAP. Similarly, "agonist" includes any molecule that mimics a biological activity of an endogenous AAP. Molecules that can act as agonists or antagonists include Abs or antibody fragments, fragments or variants of an endogenous AAP, peptides, antisense oligonucleotides, small organic molecules, *etc.*

2. *Identifying antagonists and agonists*

To assay for antagonists, an AAP is added to, or expressed in, a cell along with the compound to be screened for a particular activity. If the compound inhibits the activity of interest in the presence of an AAP, that compound is an antagonist to the AAP; if an AAP activity is enhanced, the compound is an agonist.

(a) *Specific examples of potential antagonists and agonist*

Any molecule that alters AAP cellular effects is a candidate antagonist or agonist. Screening techniques well known to those skilled in the art can identify these molecules. Examples of antagonists and agonists include: (1) small organic and inorganic compounds, (2) small peptides, (3) Abs and derivatives, (4) polypeptides closely related to an AAP, (5) antisense DNA and RNA, (6) ribozymes, (7) triple DNA helices and (8) nucleic acid aptamers.

Small molecules that bind to an AAP active site or other relevant part of the polypeptide and inhibit the biological activity of the AAP are antagonists. Examples of small molecule antagonists include small peptides, peptide-like molecules, preferably soluble, and synthetic non-peptidyl organic or inorganic compounds. These same molecules, if they enhance an AAP activity, are examples of agonists.

Almost any antibody that affects an AAP's function is a candidate antagonist, and occasionally, agonist. Examples of antibody antagonists include polyclonal, monoclonal, single-chain, anti-idiotypic, chimeric Abs, or humanized versions of such Abs or fragments. Abs may be from any species in which an immune response can be raised. Humanized Abs are also contemplated.

Alternatively, a potential antagonist or agonist may be a closely related protein, for example, a mutated form of an AAP that recognizes an AAP-interacting protein but imparts no effect, thereby competitively inhibiting AAP action. Alternatively, a mutated AAP may be constitutively activated and may act as an agonist.

Antisense RNA or DNA constructs can be effective antagonists. Antisense RNA or DNA molecules block function by inhibiting translation by hybridizing to targeted mRNA Antisense technology can be used to control gene expression through triple-helix formation or antisense DNA or RNA, both of which depend on polynucleotide binding to DNA or RNA. For example, the 5' coding portion of an *AAP* sequence is used to design an antisense RNA oligonucleotide of from about 10 to 40 base pairs in length A DNA oligonucleotide is designed to be complementary to a region of the gene involved in transcription (triple helix) (Beal and Dervan, 1991; Cooney *et al.,* 1988; Lee *et al.,* 1979), thereby preventing transcription and the production of the AAP. The antisense RNA oligonucleotide hybridizes to the mRNA *in vivo* and blocks translation of the mRNA molecule into the AAP (antisense) (Cohen, 1989; Okano *et al.,* 1991). These oligonucleotides can also be delivered to cells such that the antisense RNA or DNA may be expressed *in vivo* to inhibit production of the AAP. When antisense DNA is used, oligodeoxyribonucleotides derived from the translation-initiation site, *e.g.,* between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques (WO 97/33551,1997; Rossi, 1994).

To inhibit transcription, triple-helix nucleic acids that are single-stranded and comprise deoxynucleotides are useful antagonists. These oligonucleotides are designed such that triple-helix formation via Hoogsteen base-pairing rules is promoted, generally requiring stretches of purines or pyrimidines (WO 97/33551, 1997).

Aptamers are short oligonucleotide sequences that can be used to recognize and specifically bind almost any molecule. The systematic evolution of ligands by exponential enrichment (SELEX) process (Ausubel *et al.,* 1987; Ellington and Szostak, 1990; Tuerk and Gold, 1990) is powerful and can be used to find such aptamers. Aptamers have many diagnostic and clinical uses; almost any use in which an antibody has been used clinically or diagnostically, aptamers too may be used In addition, they are cheaper to make once they have been identified, and can be easily applied in a variety of formats, including administration in pharmaceutical compositions, in bioassays, and diagnostic tests (Jayasena, 1999).

*Anti-AAP Abs*

The invention encompasses Abs and antibody fragments, such as $F_{ab}$ or $(F_{ab})_2$, that bind immunospecifically to any AAP epitopes.

"Antibody" (Ab) comprises single Abs directed against an AAP (anti-AAP Ab; including agonist, antagonist, and neutralizing Abs), anti-AAP Ab compositions with poly-epitope specificity, single chain anti-AAP Abs, and fragments of anti-AAP Abs. A "monoclonal antibody" is obtained from a population of substantially homogeneous Abs, *i.e.,* the individual Abs comprising the population are identical except for possible naturally-occurring mutations that may be present in minor amounts. Exemplary Abs include polyclonal (pAb), monoclonal (mAb), humanized, bi-specific (bsAb), and heteroconjugate Abs.

1. *Polyclonal Abs (pAbs)*

Polyclonal Abs can be raised in a mammalian host, for example, by one or more injections of an immunogen and, if desired, an adjuvant. Typically, the immunogen and/or adjuvant are injected in the mammal by multiple subcutaneous or intraperitoneal injections. The immunogen may include an AAP or a fusion protein. Examples of adjuvants include Freund's complete and monophosphoryl Lipid A synthetic-trehalose dicorynomycolate (MPL-TDM). To improve the immune response, an immunogen may be conjugated to a protein that is immunogenic in the host, such as keyhole limpet hemocyanim (KLH), serum albumin, bovine thyroglobulin, and soybean trypsin inhibitor. Protocols for antibody production are described by (Ausubel *et al.,* 1987; Harlow and Lane, 1988). Alternatively, pAbs may be made in chickens, producing IgY molecules (Schade *et al.,* 1996).

2. *Monoclonal Abs (mAbs)*

Anti-AAP mAbs may be prepared using hybridoma methods (Milstein and Cuello, 1983). Hybridoma methods comprise at least four steps: (1) immunizing a host, or lymphocytes from a host; (2) harvesting the mAb secreting (or potentially secreting) lymphocytes, (3) fusing the lymphocytes to immortalized cells, and (4) selecting those cells that secrete the desired (anti-AAP) mAb.

A mouse, rat, guinea pig, hamster, or other appropriate host is immunized to elicit lymphocytes that produce or are capable of producing Abs that will specifically bind to the immunogen. Alternatively, the lymphocytes may be immunized *in vitro.* If human cells are desired, peripheral blood lymphocytes (PBLs) are generally used; however, spleen cells or lymphocytes from other mammalian sources are preferred. The immunogen typically includes an AAP or a fusion protein.

The lymphocytes are then fused with an immortalized cell line to form hybridoma cells, facilitated by a fusing agent such as polyethylene glycol (Goding, 1996). Rodent, bovine, or human myeloma cells immortalized by transformation may be used, or rat or mouse myeloma cell lines. Because pure populations of hybridoma cells and not unfused immortalized cells are preferred, the cells after fusion are grown in a suitable medium that contains one or more substances that inhibit the growth or survival of unfused, immortalized cells. A common technique uses parental cells that lack the enzyme hypoxanthine guanine phosphoribosyl transferase (HGPRT or BPRT). In this case, hypoxanthine, aminopterin and thymidine are added to the medium (HAT medium) to prevent the growth of HGPRT-deficient cells while permitting hybridomas to grow.

Preferred immortalized cells fuse efficiently, can be isolated from mixed populations by selecting in a medium such as HAT, and support stable and high-level expression of antibody after fusion. Preferred immortalized cell lines are murine myeloma lines, available from the American Type Culture Collection (Manassas, VA). Human myeloma and mouse-human heteromyeloma cell lines also have been described for the production of human mAbs (Kozbor *et al.,* 1984; Schook, 1987).

Because hybridoma cells secrete antibody extracellularly, the culture media can be assayed for the presence of mAbs directed against an AAP (anti-AAP mAbs). Immunoprecipitation or *in vitro* binding assays, such as radio immunoassay (RIA) or enzyme-linked immunoabsorbent assay (ELISA), measure the binding specificity of mAbs (Harlow and Lane, 1988; Harlow and Lane, 1999), including Scatchard analysis (Munson and Rodbard, 1980).

Anti-AAP mAb secreting hybridoma cells may be isolated as single clones by limiting dilution procedures and sub-cultured (Goding, 1996). Suitable culture media include Dulbecco's Modified Eagle's Medium, RPMI-1640, or if desired, a protein-free or -reduced or serum-free medium (*e.g.*, Ultra DOMA PF or HL-1; Biowhittaker; Walkersville, MD). The hybridoma cells may also be grown *in vivo* as ascites.

The mAbs may be isolated or purified from the culture medium or ascites fluid by conventional Ig purification procedures such as protein A-Sepharose, hydroxylapatite chromatography, gel electrophoresis, dialysis, ammonium sulfate precipitation or affinity chromatography (Harlow and Lane, 1988; Harlow and Lane, 1999).

The mAbs may also be made by recombinant methods (U.S. Patent No. 4166452, 1979). DNA encoding anti-AAP mAbs can be readily isolated and sequenced using conventional procedures, *e.g.*, using oligonucleotide probes that specifically bind to murine heavy and light antibody chain genes, to probe preferably DNA isolated from anti-AAP-secreting mAb hybridoma cell lines. Once isolated, the isolated DNA fragments are sub-cloned into expression vectors that are then transfected into host cells such as simian COS-7 cells, Chinese hamster ovary (CHO) cells, or myeloma cells that do not otherwise produce Ig protein, to express mAbs. The isolated DNA fragments can be modified, for example, by substituting the coding sequence for human heavy and light chain constant domains in place of the homologous murine sequences (U.S. Patent No. 4816567, 1989; Morrison *et al.,* 1987), or by fusing the Ig coding sequence to all or part of the coding sequence for a non-Ig polypeptide. Such a non-Ig polypeptide can be substituted for the constant domains of an antibody, or can be substituted for the variable domains of one antigen-combining site to create a chimeric bivalent antibody.

*3. Monovalent Abs*

The Abs may be monovalent Abs that consequently do not cross-link with each other. For example, one method involves recombinant expression of Ig light chain and modified heavy chain. Heavy chain truncations generally at any point in the $F_c$ region will prevent heavy chain cross-linking. Alternatively, the relevant cysteine residues are substituted with another amino acid residue or are deleted, preventing crosslinking. *In vitro* methods are also suitable for preparing monovalent Abs. Abs can be digested to produce fragments, such as $F_{ab}$ fragments (Harlow and Lane, 1988; Harlow and Lane; 1999).

4. *Humanized and human Abs*

Anti-AAP Abs may further comprise humanized or human Abs. Humanized forms of non-human Abs are chimeric Igs, Ig chains or fragments (such as $F_v$, $F_{ab}$, $F_{ab'}$, $F_{(ab')2}$ or other antigen-binding subsequences of Abs) that contain minimal sequence derived from non-human Ig.

Generally, a humanized antibody has one or more amino acid residues introduced from a non-human source. These non-human amino acid residues are often referred to as "import" residues, which are typically taken from an "import" variable domain. Humanization is accomplished by substituting rodent CDRs or CDR sequences for the corresponding sequences of a human antibody (Jones *et al.,* 1986; Riechmann *et al.,* 1988; Verhoeyen *et al.,* 1988). Such "humanized" Abs are chimeric Abs (U.S. Patent No. 4816567, 1989), wherein substantially less than an intact human variable domain has been substituted by the corresponding sequence from a non-human species. In practice, humanized Abs are typically human Abs in which some CDR residues and possibly some FR residues are substituted by residues from analogous sites in rodent Abs. Humanized Abs include human Igs (recipient antibody) in which residues from a complementary determining region (CDR) of the recipient are replaced by residues from a CDR of a non-human species (donor antibody) such as mouse, rat or rabbit, having the desired specificity, affinity and capacity. In some instances, corresponding non-human residues replace $F_v$ framework residues of the human Ig. Humanized Abs may comprise residues that are found neither in the recipient antibody nor in the imported CDR or framework sequences. In general, the humanized antibody comprises substantially all of at least one, and typically two, variable domains, in which most if not all of the CDR regions correspond to those of a non-human Ig and most if not all of the FR regions are those of a human Ig consensus sequence. The humanized antibody optimally also comprises at least a portion of an Ig constant region ($F_c$), typically that of a human Ig (Jones *et al.,* 1986; Presta, 1992; Riechmann *et al.,* 1988).

Human Abs can also be produced using various techniques, including phage display libraries (Hoogenboom *et al.,* 1991; Marks *et al.,* 1991) and the preparation of human mAbs (Boerner *et al.,* 1991; Reisfeld and Sell, 1985). Similarly, introducing human Ig genes into transgenic animals in which the endogenous Ig genes have been partially or completely inactivated can be exploited to synthesize human Abs. Upon challenge, human antibody production is observed, which closely resembles that seen in humans in all respects, including gene rearrangement, assembly, and antibody repertoire (U.S. Patent No. 5545807, 1996; U.S. Patent No. 5545806, 1996; U.S. Patent No. 5569825, 1996; U.S. Patent No. 5633425, 1997; U.S. Patent No. 5661016, 1997; U.S. Patent No. 5625126, 1997; Fishwild *et al.,* 1996; Lonberg and Huszar, 1995; Lonberg *et al.,* 1994; Marks *et al.,* 1992).

5. *Bi-specific mAbs*

Bi-specific Abs are monoclonal, preferably human or humanized, that have binding specificities for at least two different antigens. For example, a binding specificity is an AAP; the other is for any antigen of choice, preferably a cell-surface protein or receptor or receptor subunit.

Traditionally, the recombinant production ofbi-specific Abs is based on the co-expression of two Ig heavy-chain/light-chain pairs, where the two heavy chains have different specificities (Milstein and Cuello, 1983). Because of the random assortment of Ig heavy and light chains, the resulting hybridomas (quadromas) produce a potential mixture of ten different antibody molecules, of which only one has the desired bi-specific structure. The desired antibody can be purified using affinity chromatography or other techniques (WO 93/08829, 1993; Traunecker *et al.,* 1991).

To manufacture a bi-specific antibody (Suresh *et al.,* 1986), variable domains with the desired antibody-antigen combining sites are fused to Ig constant domain sequences. The fusion is preferably with an Ig heavy-chain constant domain, comprising at least part of the hinge, CH2, and CH3 regions. Preferably, the first heavy-chain constant region (CH1) containing the site necessary for light-chain binding is in at least one of the fusions. DNAs encoding the Ig heavy-chain fusions and, if desired, the Ig light chain, are inserted into separate expression vectors and are co-transfected into a suitable host organism.

The interface between a pair of antibody molecules can be engineered to maximize the percentage ofheterodimers that are recovered from recombinant cell culture (WO 96/27011,1996). The preferred interface comprises at least part of the CH3 region of an antibody constant domain. In this method, one or more small amino acid side chains from the interface of the first antibody molecule are replaced with larger side chains (*e.g.* tyrosine or tryptophan). Compensatory "cavities" of identical or similar size to the large side chain(s) are created on the interface of the second antibody molecule by replacing large amino acid side chains with smaller ones (*e.g.* alanine or threonine). This mechanism increases the yield of the heterodimer over unwanted end products such as homodimers.

Bi-specific Abs can be prepared as full length Abs or antibody fragments (*e.g.* $F_{(ab')2}$ bi-specific Abs). One technique to generate bi-specific Abs exploits chemical linkage. Intact Abs can be proteolytically cleaved to generate $F_{(ab')2}$ fragments (Brennan *et al.,* 1985). Fragments are reduced with a dithiol complexing agent, such as sodium arsenite, to stabilize vicinal dithiols and prevent intermolecular disulfide formation. The generated $F_{ab'}$ fragments are then converted to thionitrobenzoate (TNB) derivatives. One of the $F_{ab'}$-TNB derivatives is then reconverted to the $F_{ab'}$-thiol by reduction with mercaptoethylamine and is mixed with an equimolar amount of the other $F_{ab'}$-TNB derivative to form the bi-specific antibody. The produced bi-specific Abs can be used as agents for the selective immobilization of enzymes.

$F_{ab'}$ fragments may be directly recovered from *E. coli* and chemically coupled to form bi-specific Abs. For example, fully humanized bi-specific $F_{(ab')2}$ Abs can be produced (Shalaby *et al.,* 1992). Each $F_{ab}$, fragment is separately secreted from *E. coli* and directly coupled chemically *in vitro,* forming the bi-specific antibody.

Various techniques for making and isolating bi-specific antibody fragments directly from recombinant cell culture have also been described. For example, leucine zipper motifs can be exploited (Kostelny *et al.,* 1992). Peptides from the *Fos* and *Jun* proteins are linked to the $F_{ab'}$ portions of two different Abs by gene fusion. The antibody homodimers are reduced at the hinge region to form monomers and then re-oxidized to form antibody heterodimers. This method can also produce antibody homodimers. The "diabody" technology (Holliger *et al.,* 1993) provides an alternative method to generate bi-specific antibody fragments. The fragments comprise a heavy-chain variable domain ($V_H$) connected to a light-chain variable domain ($V_L$) by a linker that is too short to allow pairing between the two domains on the same chain. The $V_H$ and $V_L$ domains of one fragment are forced to pair with the complementary $V_L$ and $V_H$ domains of another fragment, forming two antigen-binding sites. Another strategy for making bi-specific antibody fragments is the use of single-chain $F_v$ ($sF_v$) dimers (Gruber *et al.,* 1994). Abs with more than two valencies are also contemplated, such as tri-specific Abs (Tutt *et al.,* 1991).

Exemplary bi-specific Abs may bind to two different epitopes on a given AAP. Alternatively, cellular defense mechanisms can be restricted to a particular cell expressing the particular AAP: an anti-AAP arm may be combined with an arm that binds to a leukocyte triggering molecule, such as a T-cell receptor molecule (*e.g.* CD2, CD3, CD28, or B7), or to $F_c$ receptors for IgG ($F_c\gamma R$), such as $F_c\gamma RI$ (CD64), $F_c\gamma RII$ (CD32) and $F_c\gamma RIII$ (CD 16). Bi-specific Abs may also be used to target cytotoxic agents to cells that express a particular AAP. These Abs possess an AAP-binding arm and an arm that binds a cytotoxic agent or a radionuclide chelator.

6. *Heteroconjugate Abs*

Heteroconjugate Abs, consisting of two covalently joined Abs, have been proposed to target immune system cells to unwanted cells (4,676,980, 1987) and for treatment of human immunodeficiency virus (HIV) infection (WO 91/00360, 1991; WO 92/20373, 1992). Abs prepared *in vitro* using synthetic protein chemistry methods, including those involving cross-linking agents, are contemplated. For example, immunotoxins may be constructed using a disulfide exchange reaction or by forming a thioether bond. Examples of suitable reagents include iminothiolate and methyl-4-mercaptobutyrimidate (4,676,980, 1987).

7. *Immunoconjugates*

Immunoconjugates may comprise an antibody conjugated to a cytotoxic agent such as a chemotherapeutic agent, toxin (*e.g.*, an enzymatically active toxin or fragment of bacterial, fungal, plant, or animal origin), or a radioactive isotope (*i.e.*, a radioconjugate).

Useful enzymatically-active toxins and fragments include Diphtheria A chain, non-binding active fragments of Diphtheria toxin, exotoxin A chain from *Pseudomonas aeruginosa,* ricin A chain, abrin A chain, modeccin A chain, α-sarcin, *Aleurites fordii* proteins, Dianthin proteins, *Phytolaca americana* proteins, *Momordica charantia* inhibitor, curcin, crotin, *Sapaonaria officinalis* inhibitor, gelonin, mitogellin, restrictocin, phenomycin, enomycin, and the tricothecenes. A variety of radionuclides are available for the production of radioconjugated Abs, such as $^{212}Bi$, $^{131}I$, $^{131}In$, $^{90}Y$, and $^{186}Re$.

Conjugates of the antibody and cytotoxic agent are made using a variety of bi-functional protein-coupling agents, such as N-succinimidyl-3-(2-pyridyldithiol) propionate (SPDP), iminothiolane (IT), bi-functional derivatives of imidoesters (such as dimethyl adipimidate HCl), active esters (such as disuccinimidyl suberate), aldehydes (such as glutareldehyde), *bis*-azido compounds (such as bis (p-azidobenzoyl) hexanediamine), *bis*-diazonium derivatives (such as bis-(p-diazoniumbenzoyl)-ethylenediamine), diisocyanates (such as tolyene 2,6- diisocyanate), and *bis*-active fluorine compounds (such as 1,5-difluoro-2,4-dinitrobenzene). For example, a ricin immunotoxin can be prepared (Vitetta *et al.,* 1987). $^{14}C$-labeled 1-isothiocyanatobenzyl-3-methyldiethylene triaminepentaacetic acid (MX-DTPA) is an exemplary chelating agent for conjugating radionuclide to antibody (WO 94/11026, 1994).

In another embodiment, the antibody may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a streptavidin "ligand" *(e.g.,*

biotin) that is conjugated to a cytotoxic agent *(e.g.,* a radionuclide).

8. *Effector function engineering*

The antibody can be modified to enhance its effectiveness in treating a disease, such as cancer. For example, cysteine residue(s) may be introduced into the $F_c$ region, thereby allowing interchain disulfide bond formation in this region. Such homodimeric Abs may have improved internalization capability and/or increased complement-mediated cell killing and antibody-dependent cellular cytotoxicity (ADCC) (Caron *et al.,* 1992; Shopes, 1992). Homodimeric Abs with enhanced anti-tumor activity can be prepared using hetero-bifunctional cross-linkers (Wolff *et al.,* 1993). Alternatively, an antibody engineered with dual $F_c$ regions may have enhanced complement lysis (Stevenson *et al.,* 1989).

9. *Immunoliposomes*

Liposomes containing the antibody may also be formulated (U.S. Patent No. 4485045, 1984; U.S. Patent No. 4544545, 1985; U.S. Patent No. 5013556, 1991; Eppstein *et al.,* 1985; Hwang *et al.,* 1980). Useful liposomes can be generated by a reverse-phase evaporation method with a lipid composition comprising phosphatidylcholine, cholesterol, and PEG-derivatized phosphatidylethanolamine (PEG- PE). Such preparations are extruded through filters of defined pore size to yield liposomes with a desired diameter. $F_{ab'}$ fragments of the antibody can be conjugated to the liposomes (Martin and Papahadjopoulos, 1982) via a disulfide-interchange reaction. A chemotherapeutic agent, such as Doxorubicin, may also be contained in the liposome (Gabizon *et al.,* 1989). Other useful liposomes with different compositions are contemplated.

10. *Diagnostic applications ofAbs directed against an AAP*

Anti-AAP Abs can be used to localize and/or quantitate anAAP (*e.g.*, for use in measuring levels of an AAP within tissue samples or for use in diagnostic methods, *etc.*). Anti-AAP epitope Abs can be utilized as pharmacologically-active compounds.

Anti-AAP Abs can be used to isolate an AAP by standard techniques, such as immunoaffinity chromatography or immunoprecipitation. These approaches facilitate purifying an endogenous AAP antigen-containing polypeptides from cells and tissues. These approaches, as well as others, can be used to detect an AAP in a sample to evaluate the abundance and pattern of expression of the antigenic protein. Anti-AAP Abs can be used to monitor protein levels in tissues as part of a clinical testing procedure; for example, to determine the efficacy of a given treatment regimen. Coupling the antibody to a detectable substance (label) allows detection of Ab-antigen complexes. Classes of labels include fluorescent, luminescent, bioluminescent, and radioactive materials, enzymes and prosthetic groups. Useful labels include horseradish peroxidase, alkaline phosphatase, β-galactosidase, acetylcholinesterase, streptavidin/biotin, avidin/biotin, umbelliferone, fluorescein, fluorescein isothiocyanate, rhodamine, dichlorotriazinylamine fluorescein, dansyl chloride, phycoerythrin, luminol, luciferase, luciferin, aequorin, and $^{125}I$, $^{131}I$, $^{35}S$ or $^{3}H$.

11. *Antibody therapeutics*

Abs of the invention, including polyclonal, monoclonal; humanized and fully human Abs, can be used therapeutically. Such agents will generally be employed to treat or prevent a disease or pathology in a subject An antibody preparation, preferably one having high antigen specificity and affinity generally mediates an effect by binding the target epitope(s). Generally, administration of such Abs may mediate one of two effects: (1) the antibody may prevent ligand binding, eliminating endogenous ligand binding and subsequent signal transduction, or (2) the antibody elicits a physiological result by binding an effector site on the target molecule, initiating signal transduction.

A therapeutically effective amount of an antibody relates generally to the amount needed to achieve a therapeutic objective, epitope binding affinity, administration rate, and depletion rate of the antibody from a subject. Common ranges for therapeutically effective doses may be, as a nonlimiting example, from about 0.1 mg/kg body weight to about 50 mg/kg body weight. Dosing frequencies may range, for example, from twice daily to once a week.

12. *Pharmaceutical compositions of Abs*

Anti-AAP Abs, as well as other AAP interacting molecules (such as aptamers) identified in other assays, can be administered in pharmaceutical compositions to treat various disorders. Principles and considerations involved in preparing such compositions, as well as guidance in the choice of components can be found in (de Boer, 1904; Gennaro, 2000; Lee, 1990).

Since some AAP are intracellular, Abs that are internalized are preferred used when whole Abs are used as inhibitors. Liposomes may also be used as a delivery vehicle for intracellular introduction. Where antibody fragments are used, the smallest inhibitory fragment that specifically binds to the epitope is preferred. For example, peptide molecules can be designed that bind a preferred epitope based on the variable-region sequences of a useful antibody. Such peptides can be synthesized chemically and/or produced by recombinant DNA technology (Marasco *et al.,* 1993). Formulations may also contain more than one active compound for a particular treatment, preferably those with activities that do not adversely affect each other. The composition may comprise an agent that enhances function, such as a cytotoxic agent, cytokine, chemotherapeutic agent, or growth-inhibitory agent.

The active ingredients can also be entrapped in microcapsules prepared by coacervation techniques or by interfacial polymerization; for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions.

The formulations to be used for *in vivo* administration are highly preferred to be sterile. This is readily accomplished by filtration through sterile filtration membranes or any of a number of techniques.

Sustained-release preparations may also be prepared, such as semi-permeable matrices of solid hydrophobic polymers containing the antibody, which matrices are in the form of shaped articles, *e.g.,* films, or microcapsules. Examples of sustained-release matrices include polyesters, hydrogels (for example, poly(2-hydroxyethyl-methacrylate), or poly(vinylalcohol)), polylactides (Boswell and Scribner, U.S. Patent No. 3,773,919, 1973), copolymers of L-glutamic acid and y ethyl-L-glutamate, non-degradable ethylene-vinyl acetate, degradable lactic acid-glycolic acid copolymers such as injectable microspheres composed of lactic acid-glycolic acid copolymer, and poly-D-(-)-3-hydroxybutyric acid. While polymers such as ethylene-vinyl acetate and lactic acid-glycolic acid enable release of molecules for over 100 days, certain hydrogels release proteins for shorter time periods and may be preferred.

*AAP recombinant expression vectors and host cells*

Vectors are tools used to shuttle DNA between host cells or as a means to express a nucleotide sequence. Some vectors function only in prokaryotes, while others function in both prokaryotes and eukaryotes, enabling large-scale DNA preparation from prokaryotes for expression in eukaryotes. Inserting the DNA of interest, such as an AAP nucleotide sequence or a fragment, is accomplished by ligation techniques and/or mating protocols well-known to the skilled artisan. Such DNA is inserted such that its integration does not disrupt any necessary components of the vector. In the case of vectors that are used to express the inserted DNA protein, the introduced DNA is operably-linked to the vector elements that govern its transcription and translation.

Vectors can be divided into two general classes: Cloning vectors are replicating plasmid or phage with regions that are non-essential for propagation in an appropriate host cell, and into which foreign DNA can be inserted; the foreign DNA is replicated and propagated as if it were a component of the vector. An expression vector (such as a plasmid, yeast, or animal virus genome) is used to introduce foreign genetic material into a host cell or tissue in order to transcribe and translate the foreign DNA. In expression vectors, the introduced DNA is operably-linked to elements, such as promoters, that signal to the host cell to transcribe the inserted DNA. Some promoters are exceptionally useful, such as inducible promoters that control gene transcription in response to specific factors. Operably-linking an *AAP* or anti-sense construct to an inducible promoter can control the expression of an *AAP* or fragments, or anti-sense constructs. Examples of classic inducible promoters include those that are responsive to $\alpha$-interferon, heat-shock, heavy metal ions, and steroids such as glucocorticoids (Kaufman, 1990) and tetracycline. Other desirable inducible promoters include those that are not endogenous to the cells in which the construct is being introduced, but, however, is responsive in those cells when the induction agent is exogenously supplied.

Vectors have many difference manifestations. A "plasmid" is a circular double stranded DNA molecule into which additional DNA segments can be introduced. Viral vectors can accept additional DNA segments into the viral genome. Certain vectors are capable of autonomous replication in a host cell (*e.g.*, bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. In general, useful expression vectors are often plasmids. However, other forms of expression vectors, such as viral vectors (*e.g.*, replication defective retroviruses, adenoviruses and adeno-associated viruses) are contemplated.

Recombinant expression vectors that comprise an *AAP* (or fragments) regulate an *AAP* transcription by exploiting one or more host cell-responsive (or that can be manipulated *in vitro)* regulatory sequences that is operably-linked to an *AAP.* "Operably-linked" indicates that a nucleotide sequence of interest is linked to regulatory sequences such that expression of the nucleotide sequence is achieved.

Vectors can be introduced in a variety of organisms and/or cells (Table D). Alternatively, the vectors can be transcribed and translated *in vitro,* for example using T7 promoter regulatory sequences and T7 polymerase.

Table D Examples of hosts for cloning or expression

| Organisms | Examples | Sources and References* |
|---|---|---|
| Prokaryotes | | |
| Enterobacteriaceae | *E. coli*<br>K 12 strain MM294<br>X1776<br>W3110<br>K5 772 | <br>ATCC 31,446<br>ATCC 31,537<br>ATCC 27,325<br>ATCC 53,635 |
| | *Enterobacter* | |
| | *Erwinia* | |
| | *Klebsiella* | |
| | *Proteus* | |
| | *Salmonella (S. tyhpimurium)* | |
| | *Serratia (S. marcescans)* | |
| | *Shigella* | |
| | *Bacilli* (B. *subtilis* and *B. licheniformis)* | |
| | *Pseudomonas* (P. *aeruginosa)* | |
| | *Streptomyces* | |
| Eukaryotes | | |
| Yeasts | *Saccharomyces cerevisiae* | |
| | *Schizosaccharomyces pombe* | |
| | *Kluyveromyces*<br>*K. lactis* MW98-8C, CBS683, CBS4574<br>*K. fragilis*<br>*K. bulgaricus*<br>*K. wickeramii* | (Fleer *et al.,* 1991)<br>(de Louvencourt *et al.,* 1983)<br>ATCC 12,424<br>ATCC 16,045<br>ATCC 24,178 |
| | *K. waltii*<br>*K. drosophilarum*<br>*K. thermotolerans*<br>*K. marxianus; yarrowia* | ATCC 56,500<br>ATCC 36,906<br><br>(EPO 402226, 1990) |
| | *Pichia pastoris* | (Sreelaishna *et al.,* 1988) |
| | *Candida* | |
| | *Trichoderma reesia* | |
| | *Neurospora crassa* | (Case *et al.,* 1979) |
| | *Torulopsis* | |
| | *Rhodotorula* | |
| | *Schwanniomyces (S. occidentalis)* | |

(continued)

| Organisms | Examples | Sources and References* |
|---|---|---|
| Filamentous Fungi | *Neurospora* | |
| | *Penicillium* | |
| | *Tolypocladium* | (WO 91/00357, 1991) |
| | *Aspergillus (A. nidulans* and *A. niger)* | (Kelly and Hynes, 1985; Tilburn *et al.,* 1983; Yelton *et al.,* 1984) |
| Invertebrate cells | *Drosophila* S2 | |
| | *Spodoptera* Sf9 | |
| Vertebrate cells | Chinese Hamster Ovary (CHO) | |
| | simian COS<br>COS-7 | ATCC CRL 1651 |
| | HEK 293 | |
| *Unreferenced cells are generally available from American Type Culture Collection (Manassas, VA). | | |

Vector choice is dictated by the organism or cells being used and the desired fate of the vector. Vectors may replicate once in the target cells, or may be "suicide" vectors. In general, vectors comprise signal sequences, origins of replication, marker genes, enhancer elements, promoters, and transcription termination sequences. The choice of these elements depends on the organisms in which the vector will be used and are easily determined. Some of these elements may be conditional, such as an inducible or conditional promoter that is turned "on" when conditions are appropriate. Examples of inducible promoters include those that are tissue-specific, which relegate expression to certain cell types, steroid-responsive, or heat-shock reactive. Some bacterial repression systems, such as the lac operon, have been exploited in mammalian cells and transgenic animals (Fieck *et al.*,1992; Wyborski *et al.,* 1996; Wyborski and Short, 1991). Vectors often use a selectable marker to facilitate identifying those cells that have incorporated the vector. Many selectable markers are well known in the art for the use with prokaryotes, usually antibiotic-resistance genes or the use of autotrophy and auxotrophy mutants.

Using antisense and sense AAP oligonucleotides can prevent an AAP polypeptide expression. These oligonucleotides bind to target nucleic acid sequences, forming duplexes that block transcription or translation of the target sequence by enhancing degradation of the duplexes, terminating prematurely transcription or translation, or by other means.

Antisense or sense oligonucleotides are singe-stranded nucleic acids, either RNA or DNA, which can bind a target AAP mRNA (sense) or an AAP DNA (antisense) sequences. According to the present invention, antisense or sense oligonucleotides comprise a fragment of an AAP DNA coding region of at least about 14 nucleotides, preferably from about 14 to 30 nucleotides. In general, antisense RNA or DNA molecules can comprise at least 5, 10, 15, 20, 25, 30, 35, 40, 45, 50, 55, 60, 65, 70, 75, 80, 85, 90, 95, 100 bases in length or more. Among others, (Stein and Cohen, 1988; van der Krol *et al.,* 1988a) describe methods to derive antisense or a sense oligonucleotides from a given cDNA sequence.

Modifications of antisense and sense oligonucleotides can augment their effectiveness. Modified sugar-phosphodiester bonds or other sugar linkages (WO 91/06629, 1991), increase *in vivo* stability by conferring resistance to endogenous nucleases without disrupting binding specificity to target sequences. Other modifications can increase the affinities of the oligonucleotides for their targets, such as covalently linked organic moieties (WO 90/10448, 1990) or poly-(L)-lysine. Other attachments modify binding specificities of the oligonucleotides for their targets, including metal complexes or intercalating *(e.g.* ellipticine) and alkylating agents.

To introduce antisense or sense oligonucleotides into target cells (cells containing the target nucleic acid sequence), any gene transfer method may be used and are well known to those of skill in the art Examples of gene transfer methods include 1) biological, such as gene transfer vectors like Epstein-Barr virus or conjugating the exogenous DNA to a ligand-binding molecule (WO 91/04753, 1991), 2) physical, such as electroporation, and 3) chemical, such as $CaPO_4$ precipitation and oligonucleotide-lipid complexes (WO 90/10448,1990).

The terms "host cell" and "recombinant host cell" are used interchangeably. Such terms refer not only to a particular subject cell but also to the progeny or potential progeny of such a cell. Because certain modifications may occur in succeeding generations due to either mutation or environmental influences, such progeny may not, in fact, be identical to the parent cell, but are still included within the scope of the term.

Methods of eukaryotic cell transfection and prokaryotic cell transformation are well known in the art. The choice of host cell will dictate the preferred technique for introducing the nucleic acid of interest. Table E, which is not meant to be limiting, summarizes many of the known techniques in the art. Introduction of nucleic acids into an organism may also be done with *ex vivo* techniques that use an *in vitro* method of transfection, as well as established genetic techniques, if any, for that particular organism.

Table E Methods to introduce nucleic acid into cells

| Cells | Methods | References | Notes |
|---|---|---|---|
| Prokaryotes (bacteria) | Calcium chloride | (Cohen *et al.,* 1972; Hanahan, 1983; Mandel and Higa, 1970) | |
| | Electroporation | (Shigekawa and Dower, 1988) | |
| Eukaryotes<br>Mammalian cells | Calcium phosphate transfection | *N*-(2-Hydroxyethyl) piperazine-*N'*-(2-ethanesulfonic acid (HEPES) buffered saline solution (Chen and Okayama, 1988; Graham and van der Eb, 1973; Wigler *et al.,* 1978)<br>BES (*N,N*-bis(2-hydroxyethyl)-2-aminoethanesulfonic acid) buffered solution (Ishiura *et al*., 1982) | Cells may be "shocked" with glycerol or dimethylsulfoxide (DMSO) to increase transfection efficiency (Ausubel *et al.,* 1987). |

(continued)

| Cells | Methods | References | Notes |
|---|---|---|---|
| | Diethylaminoethyl (DEAF,)-Dextran transfection | (Fujita *et al*., 1986; Lopata *et al.,* 1984; Selden *et al.*, 1986) | Most useful for transient, but not stable, transfections. Chloroquine can be used to increase be efficiency. |
| | Electroporation | (Neumann *et al* , 1982; Potter, 1988; Potter *et al.,* 1984; Wong and Neumann, 1982) | Especially useful for hard-to-transfect lymphocytes. |
| | Cationic lipid reagent transfection | (Elroy-Stein and Moss, 1990; Felgner *et al.,* 1987; Rose *et al.,* 1991; Whitt *et al.,* 1990) | Applicable to both *in vivo* and *in vitro* transfection. |
| | Retroviral | Production exemplified by (Cepko *et al*., 1984; Miller and Buttimore, 1986; Pear et *al.,* 1993) Infection *in vitro* and *in vivo*: (Austin and Cepko, 1990; Bodine *et al.,* 1991; Fekete and Cepko, 1993; Lemischka *et al.,* 1986; Turner *et al.,* 1990; Williams *et al.,* 1984) | Lengthy process, many packaging lines available at ATCC. Applicable to both *in vivo* and *in vitro* transfection. |
| | Polybrene | (Chaney *et al.,* 1986; Kawai and Nishizawa, 1984) | |
| | Microinjection | (Capecchi, 1980) | Can be used to establish cell lines carrying integrated copies of AAP DNA sequences. |
| | Protoplast fusion | (Rassoulzadegan *et al.,* 1982; Sandri-Goldin *et al.,* 1981; Schaffner, 1980) | |
| Insect cells (*in vitro*) | Baculovirus systems | (Luckow, 1991; Miller, 1988; O'Reilly *et al*., 1992) | Useful for in *vitro* production of proteins with eukaryotic modifications. |
| Yeast | Electroporation | (Becker and Guarente, 1991) | |
| | Lithium acetate | (Gietz *et al.,* 1998; Ito *et al.,* 1983) | |
| | Spheroplast fusion | (Beggs, 1978; Hinnen *et al.,* 1978) | Laborious, can produce aneuploids. |

(continued)

| Cells | Methods | References | Notes |
|---|---|---|---|
| Plant cells (general reference: (Hansen and Wright, 1999)) | Agrobacterium transformation | (Bechtold and Pelletier, 1998; Escudero and Hohn, 1997; Hansen and Chilton, 1999; Touraev and al., 1997) | |
| | Biolistics (microprojectiles) | (Finer *et al.,* 1999; Hansen and Chilton, 1999; Shillito, 1999) | |
| | Electroporation (protoplasts) | (Fromm *et al.*, 1985; Ou-Lee *et al.,* 1986; Rhodes *et al., .* 1988; Saunders *et al.*, 1989) May be combined with liposomes (Trick and al., 1997) | |
| | Polyethylene glycol (PEG) treatment | (Shillito, 1999) | |
| | Liposomes | May be combined with electroporation (Trick and al., 1997) | |
| | *in planta* microinjection | (Leduc and al., 1996; Zhou and al., 1983) | |
| | Seed imbibition | (Trick and al., 1997) | |
| | Laser beam | (Hoffman, 1996) | |
| | Silicon carbide whiskers | (Thompson and al., 1995) | |

Vectors often use a selectable marker to facilitate identifying those cells that have incorporated the vector. Many selectable markers are well known in the art for the use with prokaryotes, usually antibiotic-resistance genes or the use of autotrophy and auxotrophy mutants. Table F lists often-used selectable markers for mammalian cell transfection.

Table F Useful selectable markers for eukaryote cell transfection

| Selectable Marker | Selection | Action | Reference |
|---|---|---|---|
| Adenosine deaminase (ADA) | Media includes 9-β-D-xylofuranosyl adenine (Xyl-A) | Conversion of Xyl-A to Xyl-ATP, which incorporates into nucleic acids, killing cells. ADA detoxifies | (Kaufman *et al.,* 1986) |
| Dihydrofolate reductase (DHFR) | Methotrexate(MTX) and dialyzed serum (purine-free media) | MTX competitive inhibitor of DHFR. In absence of exogenous purines, cells require DHFR, a necessary enzyme in purine biosynthesis. | (Simonsen and Levinson, 1983) |
| Aminoglycoside phosphotransferase ("APH", "neo", "G418") | G418 | G418, an aminoglycoside detoxified by APH, interferes with ribosomal function and consequently, translation. | (Southern and Berg, 1982) |

(continued)

| Selectable Marker | Selection | Action | Reference |
|---|---|---|---|
| Hygromycin-B-phosphotransferase (HPH) | hygromycin-B | Hygromycin-B, an aminocyclitol detoxified by HPH, disrupts protein translocation and promotes mistranslation. | (Palmer *et al.*, 1987) |
| Thymidine kinase (TK) | Forward selection (TK+): Media (HAT) incorporates aminopterin. Reverse selection (TK-): Media incorporates 5-bromodeoxyuridine (BrdU). | Forward: Aminopterin forces cells to synthesze dTTP from thymidine, a pathway requiring TK. Reverse: TK phosphorylates BrdU, which incorporates into nucleic acids, killing cells. | (Littlefield, 1964) |

A host cell of the invention, such as a prokaryotic or eukaryotic host cell in culture, can be used to produce an AAP. Accordingly, the invention provides methods for producing an AAP using the host cells of the invention. In one embodiment, the method comprises culturing the host cell of the invention (into which a recombinant expression vector encoding an AAP has been introduced) in a suitable medium, such that an AAP is produced. In another embodiment, the method further comprises isolating an AAP from the medium or the host cell.

*Transgenic AAP animals*

Transgenic animals are useful for studying the function and/or activity of an *AAP* and for identifying and/or evaluating modulators of AAP activity. "Transgenic animals" are non-human animals, preferably mammals, more preferably a rodents such as rats or mice, in which one or more of the cells include a transgene. Other transgenic animals include primates, sheep; dogs, cows, goats, chickens, amphibians, *etc.* A "transgene" is exogenous DNA that is integrated into the genome of a cell from which a transgenic animal develops, and that remains in the genome of the mature animal. Transgenes preferably direct the expression of an encoded gene product in one or more cell types or tissues of the transgenic animal with the purpose of preventing expression of a naturally encoded gene product in one or more cell types or tissues (a "knockout" transgenic animal), or serving as a marker or indicator of an integration, chromosomal location, or region of recombination *(e.g. cre/loxP* mice). A "homologous recombinant animal" is a non-human animal, such as a rodent, in which an endogenous *AAP* has been altered by an exogenous DNA molecule that recombines homologously with an endogenous *AAP* in a (*e.g.* embryonic) cell prior to development the animal. Host cells with an exogenous *AAP* can be used to produce non-human transgenic animals, such as fertilized oocytes or embryonic stem cells into which an *AAP*-coding sequences have been introduced Such host cells can then be used to create non-human transgenic animals or homologous recombinant animals.

1. *Approaches to transgenic animal production*

A transgenic animal can be created by introducing an *AAP* into the male pronuclei of a fertilized oocyte (*e.g.*, by microinjection, retroviral infection) and allowing the oocyte to develop in a pseudopregnant female foster animal (pffa). An *AAP* cDNA sequences (SEQ ID NO: 3) can be introduced as a transgene into the genome of a non-human animal. Alternatively, a homologue of an *AAP,* such as the naturally-occuring variant of an *AAP,* can be used as a transgene. Intronic sequences and polyadenylation signals can also be included in the transgene to increase transgene expression. Tissue-specific regulatory sequences can be operably-linked to the *AAP* transgene to direct expression of the *AAP* to particular cells. Methods for generating transgenic animals via embryo manipulation and microinjection, particularly animals such as mice, have become conventional in the art, *e.g.* (Evans *et al.,* U.S. Patent No. 4,870,009, 1989; Hogan, 0879693843, 1994; Leder and Stewart, U.S. Patent No. 4,736,866, 1988; Wagner and Hoppe, US Patent No. 4,873,191, 1989). Other non-mice transgenic animals may be made by similar methods. A transgenic founder animal, which can be used to breed additional transgenic animals, can be identified based upon the presence of the transgene in its genome and/or expression of the transgene mRNA in tissues or cells of the animals. Transgenic animals can be bred to other transgenic animals carrying other transgenes.

2. *Vectors for transgenic animal production*

To create a homologous recombinant animal, a vector containing at least a portion of an *AAP* into which a deletion, addition or substitution has been introduced to thereby alter, *e.g.,* disrupt or alter the expression of, an *AAP.* An *AAP* can be a murine gene, or other *AAP* homologue, such as a naturally occurring variant In one approach, a knockout vector functionally disrupts an endogenous *AAP* gene upon homologous recombination, and thus a non-functional AAP protein, if any, is expressed.

Alternatively, the vector can be designed such that, upon homologous recombination, an endogenous *AAP* is mutated or otherwise altered but still encodes functional protein *(e.g.,* the upstream regulatory region can be altered to thereby alter the expression of an endogenous AAP). In this type of homologous recombination vector, the altered portion of the *AAP* is flanked at its 5'- and 3'-termini by additional nucleic acid of the *AAP* to allow for homologous recombination to occur between the exogenous *AAP* carried by the vector and an endogenous *AAP* in an embryonic stem cell. The additional flanking AAP nucleic acid is sufficient to engender homologous recombination with the endogenous *AAP.* Typically, several kilobases of flanking DNA (both at the 5'- and 3'-termini) are included in the vector (Thomas and Capecchi, 1987). The vector is then introduced into an embryonic stem cell line *(e.g.,* by electroporation), and cells in which the introduced *AAP* has homologously-recombined with an endogenous *AAP* are selected (Li *et al.,* 1992).

3. *Introduction of an* AAP *transgene cells during development*

Selected cells are then injected into a blastocyst of an animal (*e.g.*, a mouse) to form aggregation chimeras (Bradley, 1987). A chimeric embryo can then be implanted into a suitable pffa and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described (Berns *et al.,* WO 93/04169, 1993; Bradley, 1991; Kucherlapati *et al.,* WO 91/01140, 1991; Le Mouellic and Brullet, WO 90/11354,1990).

Alternatively, transgenic animals that contain selected systems that allow for regulated expression of the transgene can be produced. An example of such a system is the *cre*/*loxP* recombinase system of bacteriophage P1 (Lakso *et al.,* 1992). Another recombinase system is the FLP recombinase system of *Saccharomyces cerevisiae* (O'Gorman *et al.,* 1991). If a *cre*/*loxP* recombinase system is used to regulate expression of the transgene, animals containing transgenes encoding both the *Cre* recombinase and a selected protein are required. Such animals can be produced as "double" transgenic animals, by mating an animal containing a transgene encoding a selected protein to another containing a transgene encoding a recombinase.

Clones of transgenic animals can also be produced (Wilmut *et al.,* 1997). In brief, a cell from a transgenic animal can be isolated and induced to exit the growth cycle and enter $G_0$ phase. The quiescent cell can then be fused to an enucleated oocyte from an animal of the same species from which the quiescent cell is isolated The reconstructed oocyte is then cultured to develop to a morula or blastocyte and then transferred to a pffa. The offspring borne of this female foster animal will be a clone of the "parent" transgenic animal.

*Pharmaceutical compositions*

The *AAP* nucleic acid molecules, AAP polypeptides, and anti-AAP Abs (active compounds) of the invention, and derivatives, fragments, analogs and homologs thereof, can be incorporated into pharmaceutical compositions. Such compositions typically comprise the nucleic acid molecule, protein, or antibody and a pharmaceutically acceptable carrier. A "pharmaceutically acceptable carrier" includes any and all solvents, dispersion media, coatings, antibacterial and antifungal agents, isotonic and absorption delaying agents, and the like, compatible with pharmaceutical administration (Gennaro, 2000). Preferred examples of such carriers or diluents include, but are not limited to, water, saline, finger's solutions, dextrose solution, and 5% human serum albumin. Liposomes and non-aqueous vehicles such as fixed oils may also be used. Except when a conventional media or agent is incompatible with an active compound, use of these compositions is contemplated. Supplementary active compounds can also be incorporated into the compositions.

1. *General considerations*

A pharmaceutical composition of the invention is formulated to be compatible with its intended route of administration, including intravenous, intradermal, subcutaneous, oral *(e.g.,* inhalation), transdermal *(i.e.,* topical), transmucosal, and rectal administration. Solutions or suspensions used for parenteral, intradermal, or subcutaneous application can include: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The *p*H can be adjusted with acids or bases, such as hydrochloric acid or sodium hydroxide. The parenteral preparation can be enclosed in ampoules, disposable syringes or multiple dose vials made of glass or plastic.

2. *Injectable formulations*

Pharmaceutical compositions suitable for injection include sterile aqueous solutions (where water soluble) or dispersions and sterile powders for the extemporaneous preparation of sterile injectable solutions or dispersion. For intravenous administration, suitable carriers include physiological saline, bacteriostatic water, CREMOPHOR EL™ (BASF, Parsippany, N.J.) or phosphate buffered saline (PBS). In all cases, the composition must be sterile and should be fluid so as to be administered using a syringe. Such compositions should be stable during manufacture and storage and must be preserved against contamination from microorganisms such as bacteria and fungi. The carrier can be a solvent or dispersion medium containing, for example, water, ethanol, polyol (such as glycerol, propylene glycol, and liquid polyethylene glycol), and suitable mixtures. Proper fluidity can be maintained, for example, by using a coating such as lecithin, by maintaining the required particle size in the case of dispersion and by using surfactants. Various antibacterial and antifungal agents, for example, parabens, chlorobutanol, phenol, ascorbic acid, and thimerosal, can contain microorganism contamination. Isotonic agents, for example, sugars, polyalcohols such as manitol, sorbitol, and sodium chloride can be included in the composition. Compositions that can delay absorption include agents such as aluminum monostearate and gelatin.

Sterile injectable solutions can be prepared by incorporating the active compound (*e.g.*, an AAP or anti-AAP antibody) in the required amount in an appropriate solvent with one or a combination of ingredients as required, followed by sterilization. Generally, dispersions are prepared by incorporating the active compound into a sterile vehicle that contains a basic dispersion medium, and the other required ingredients as discussed. Sterile powders for the preparation of sterile injectable solutions, methods of preparation include vacuum drying and freeze-drying that yield a powder containing the active ingredient and any desired ingredient from a sterile solutions.

3. *Oral compositions*

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally. Pharmaceutically compatible binding agents, and/or adjuvant materials can be included. Tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, PRIMOGEL, or corn starch; a lubricant such as magnesium stearate or STEROTES; a glidant such as colloidal silicon dioxide; a sweetening agent such as sucrose or saccharin; or a flavoring agent such as peppermint, methyl salicylate, or orange flavoring.

4. *Compositions for inhalation*

For administration by inhalation, the compounds are delivered as an aerosol spray from a nebulizer or a pressurized container that contains a suitable propellant, *e.g.*, a gas such as carbon dioxide.

5. *Systemic administration*

Systemic administration can also be transmucosal or transdermal. For transmucosal or transdermal administration, penetrants that can permeate the target barrier(s) are selected Transmucosal penetrants include, detergents, bile salts, and fusidic acid derivatives. Nasal sprays or suppositories can be used for transmucosal administration. For transdermal administration, the active compounds are formulated into ointments, salves, gels, or creams.

The compounds can also be prepared in the form of suppositories (*e.g* , with bases such as cocoa butter and other glycerides) or retention enemas for rectal delivery.

6. *Carriers*

In one embodiment, the active compounds are prepared with carriers that protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid Such materials can be obtained commercially from ALZA Corporation (Mountain View, CA) and NOVA Pharmaceuticals, Inc. (Lake Elsinore, CA), or prepared by one of skill in the art Liposomal suspensions can also be used as pharmaceutically acceptable carriers. These can be prepared according to methods known to those skilled in the art, such as in (Eppstein *et al.,* US Patent No. 4,522,811, 1985),

7. *Unit dosage.*

Oral formulations or parenteral compositions in unit dosage form can be created to facilitate administration and dosage uniformity. Unit dosage form refers to physically discrete units suited as single dosages for the subject to be treated, containing a therapeutically effective quantity of active compound in association with the required pharmaceutical carrier. The specification for the unit dosage forms of the invention are dictated by, and directly dependent on, the unique characteristics of the active compound and the particular desired therapeutic effect, and the inherent limitations of compounding the active compound.

8. *Gene therapy compositions*

The nucleic acid molecules of the invention can be inserted into vectors and used as gene therapy vectors. Gene therapy vectors can be delivered to a subject by, for example, intravenous injection, local administration (Nabel and Nabel, US Patent No. 5,328,470, 1994), or by stereotactic injection (Chen *et al.,* 1994). The pharmaceutical preparation of a gene therapy vector can include an acceptable diluent, or can comprise a slow release matrix in which the gene delivery vehicle is imbedded Alternatively, where the complete gene delivery vector can be produced intact from recombinant cells, *e.g.*, retroviral vectors, the pharmaceutical preparation can include one or more cells that produce the gene delivery system.

9. *Dosage*

The pharmaceutical composition and method of the present invention may further comprise other therapeutically active compounds as noted herein that are usually applied in the treatment of the above mentioned pathological conditions.

In the treatment or prevention of conditions which require AAP modulation an appropriate dosage level will generally be about 0.01 to 500 mg per kg patient body weight per day which can be administered in single or multiple doses. Preferably, the dosage level will be about 0.1 to about 250 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0. 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be administered on a regimen of 1 to 4 times per day, preferably once or twice per day.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

10. *Kits for pharmaceutical compositions*

The pharmaceutical compositions can be included in a kit, container, pack, or dispenser together with instructions for administration. When the invention is supplied as a kit, the different components of the composition may be packaged in separate containers and admixed immediately before use. Such packaging of the components separately may permit long-term storage without losing the active components' functions.

Kits may also include reagents in separate containers that facilitate the execution of a specific test, such as diagnostic tests or tissue typing. For example, *AAP* DNA templates and suitable primers may be supplied for internal controls.

(a) *Containers or vessels*

The reagents included in the kits can be supplied in containers of any sort such that the life of the different components are preserved, and are not adsorbed or altered by the materials of the container. For example, sealed glass ampules may contain lyophilized luciferase or buffer that have been packaged under a neutral, non-reacting gas, such as nitrogen. Ampoules may consist of any suitable material, such as glass, organic polymers, such as polycarbonate, polystyrene, etc., ceramic, metal or any other material typically employed to hold reagents. Other examples of suitable containers include simple bottles that may be fabricated from similar substances as ampules, and envelopes, that may consist of foil-lined interiors, such as aluminum or an alloy. Other containers include test tubes, vials, flasks, bottles, syringes, or the like. Containers may have a sterile access port, such as a bottle having a stopper that can be pierced by a hypodermic injection needle. Other containers may have two compartments that are separated by a readily removable membrane that upon removal permits the components to mix. Removable membranes may be glass, plastic, rubber, etc.

(b) *Instructional materials*

Kits may also be supplied with instructional materials. Instructions may be printed on paper or other substrate, and/or may be supplied as an electronic-readable medium, such as a floppy disc, CD-ROM, DVD-ROM, Zip disc, videotape, audiotape, *etc.* Detailed instructions may not be physically associated with the kit; instead, a user may be directed to an internet web site specified by the manufacturer or distributor of the kit, or supplied as electronic mail.

*Screening and detection methods*

The isolated nucleic acid molecules of the invention can be used to express an AAP (*e.g.*, via a recombinant expression vector in a host cell in gene therapy applications), to detect an *AAP* mRNA (*e.g.*, in a biological sample) or a genetic lesion in an *AAP,* and to modulate AAP activity, as described below. In addition, AAP polypeptides can be used to screen drugs or compounds that modulate the AAP activity or expression as well as to treat disorders characterized by insufficient or excessive production of an AAP or production of AAP forms that have decreased or aberrant activity compared to an AAP wild-type protein, or modulate biological function that involve an AAP. In addition, the anti-AAP Abs of the invention can be used to detect and isolate an AAP and modulate AAP activity.

1. *Screening assays*

The invention provides a method (screening assay) for identifying modalities, *i.e.,* candidate or test compounds or agents (*e.g.*, peptides, peptidomimetics, small molecules or other drugs), foods, combinations thereof, *etc.,* that effect an AAP, a stimulatory or inhibitory effect, inlcuding translation, transcription, activity or copies of the gene in cells. The invention also includes compounds identified in screening assays.

Testing for compounds that increase or decrease AAP activity are desirable. A compound may modulate an AAP activity by affecting: (1) the number of copies of the gene in the cell (amplifiers and deamplifiers); (2) increasing or decreasing transcription of an *AAP* (transcription up-regulators and down-regulators); (3) by increasing or decreasing the translation of an *AAP* mRNA into protein (translation up-regulators and down-regulators); or (4) by increasing or decreasing the activity of an AAP itself (agonists and antagonists).

(a) *effects of compounds*

To identify compounds that affect an AAP at the DNA, RNA and protein levels, cells or organisms are contacted with a candidate compound and the corresponding change in an AAP DNA, RNA or protein is assessed (Ausubel *et al.,* 1987). For DNA amplifiers and deamplifiers, the amount of an *AAP* DNA is measured, for those compounds that are transcription up-regulators and down-regulators the amount of an *AAP* mRNA is determined; for translational up- and down-regulators, the amount of an AAP polypeptide is measured. Compounds that are agonists or antagonists may be identified by contacting cells or organisms with the compound, and then examining, for example, the model of angiogenesis *in vitro.*

In one embodiment, many assays for screening candidate or test compounds that bind to or modulate the activity of an AAP or polypeptide or biologically-active portion are available. Ttest compounds can be obtained using any of the numerous approaches in combinatorial library methods, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptides, while the other four approaches encompass peptide, non-peptide oligomer or small molecule libraries of compounds (Lam, 1997).

(b) *small molecules*

A "small molecule" refers to a composition that has a molecular weight of less than about 5 kD and more preferably less than about 4 kD, most preferably less than 0.6 kD. Small molecules can be, nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention. Examples of methods for the synthesis of molecular libraries can be found in: (Carell *et al.,* 1994a; Carell *et al.,* 1994b; Cho *et al.,* 1993; DeWitt *et al.,* 1993; Gallop *et al.,* 1994; Zuckermann *et al.,* 1994).

Libraries of compounds may be presented in solution (Houghten *et al.*, 1992) or on beads (Lam *et al.,* 1991), on chips (Fodor *et al.,* 1993), bacteria, spores (Ladner *et al.,* US Patent No. 5,223,409, 1993), plasmids (Cull *et al.,* 1992) or on phage (Cwirla *et al.,* 1990; Devlin *et al.*, 1990; Felici *et al.,* 1991; Ladner *et al.,* US Patent No. 5,223,409, 1993; Scott and Smith, 1990). A cell-free assay comprises contacting an AAP or biologically-active fragment with a known compound that binds the AAP to form an assay mixture, contacting the assay mixture with a test compound, and determining the ability of the test compound to interact with the AAP, where determining the ability of the test compound to interact with the AAP comprises determining the ability of the AAP to preferentially bind to or modulate the activity of an AAP target molecule.

(c) *cell-free assays*

The cell-free assays of the invention may be used with both soluble or a membrane-bound forms of an AAP. In the case of cell-free assays comprising the membrane-bound form, a solubilizing agent to maintain the AAP in solution. Examples of such solubilizing agents include non-ionic detergents such as n-octylglucoside, n-dodecylglucoside, n-dodecylmaltoside, octanoyl-N-methylglucamide, decanoyl-N-methylglucamide, TRITON® X-100 and others from the TRITON® series, THESIT®, Isotridecypoly(ethylene glycol ether)$_n$, N-dodecyl--N,N-dimethyl-3-ammonio-1-propane sulfonate, 3-(3-cholamidopropyl) dimethylamminiol-1-propane sulfonate (CHAPS); or 3-(3-cholamidopropyl)dimethylamminiol-2-hydroxy-1-propane sulfonate (CHAPSO).

(d) *immobilization of target molecules to facilitate screening*

In more than one embodiment of the assay methods, immobilizing either an AAP or its partner molecules can facilitate separation of complexed from uncomplexed forms of one or both of the proteins, as well as to accommodate high throughput assays. Binding of a test compound to an AAP, or interaction of an AAP with a target molecule in the presence and absence of a candidate compound, can be accomplished in any vessel suitable for containing the reactants, such as microtiter plates, test tubes, and micro-centrifuge tubes. A fusion protein can be provided that adds a domain that allows one or both of the proteins to be bound to a matrix. For example, GST-AAP fusion proteins or GST-target fusion proteins can be adsorbed onto glutathione sepharose beads (SIGMA Chemical, St. Louis, MO) or glutathione derivatized microtiter plates that are then combined with the test compound or the test compound and either the non-adsorbed target protein or an AAP, and the mixture is incubated under conditions conducive to complex formation (*e.g.*, at physiological conditions for salt and pH). Following incubation, the beads or microtiter plate wells are washed to remove any unbound components, the matrix immobilized in the case of beads, complex determined either directly or indirectly, for example, as described. Alternatively, the complexes can be dissociated from the matrix, and the level of AAP binding or activity determined using standard techniques.

Other techniques for immobilizing proteins on matrices can also be used in screening assays. Either an AAP or its target molecule can be immobilized using biotin-avidin or biotin-streptavidin systems. Biotinylation can be accomplished using many reagents, such as biotin-NHS (N-hydroxy-succinimide; PIERCE Chemicals, Rockford, IL), and immobilized in wells of streptavidin-coated 96 well plates (PIERCE Chemical). Alternatively, Abs reactive with an AAP or target molecules, but which do not interfere with binding of the AAP to its target molecule, can be derivatized to the wells of the plate, and unbound target or an AAP trapped in the wells by antibody conjugation. Methods for detecting such complexes, in addition to those described for the GST-immobilized complexes, include immunodetection of complexes using Abs reactive with an AAP or its target, as well as enzyme-linked assays that rely on detecting an enzymatic activity associated with the AAP or target molecule.

(e) *screens to identify modulators*

Modulators of AAP expression can be identified in a method where a cell is contacted with a candidate compound and the expression of an AAP mRNA or protein in the cell is determined. The expression level of the *AAP* mRNA or protein in the presence of the candidate compound is compared to the AAP mRNA or protein levels in the absence of the candidate compound. The candidate compound can then be identified as a modulator of the AAP mRNA or protein expression based upon this comparison. For example, when expression of an AAP mRNA or protein is greater (*i.e.,* statistically significant) in the presence of the candidate compound than in its absence, the candidate compound is identified as a stimulator of the AAP mRNA or protein expression. Alternatively, when expression of the AAP mRNA or protein is less (statistically significant) in the presence of the candidate compound than in its absence, the candidate compound is identified as an inhibitor of the AAP mRNA or protein expression. The level of an AAP mRNA or protein expression in the cells can be determined by methods described for detecting an AAP mRNA or protein.

(i) *hybrid assays*

In yet another aspect of the invention, an AAP can be used as "bait" in two-hybrid or three hybrid assays (Bartel *et al.,* 1993; Brent *et al.,* WO94/10300, 1994; Iwabuchi *et al.,* 1993; Madura *et al.,* 1993; Saifer *et al.,* US Patent No. 5,283,317, 1994; Zervos *et al.,* 1993) to identify other proteins that bind or interact with the AAP and modulate AAP activity. Such AAP-bps are also likely to be involved in the propagation of signals by the AAP as, for example, upstream or downstream elements of an AAP pathway.

The two-hybrid system is based on the modular nature of most transcription factors, which consist of separable DNA-binding and activation domains. Briefly, the assay utilizes two different DNA constructs. In one construct, the gene that codes for an AAP is fused to a gene encoding the DNA binding domain of a known transcription factor (*e.g.*, GAL4). The other construct, a DNA sequence from a library of DNA sequences that encodes an unidentified protein ("prey" or "sample") is fused to a gene that codes for the activation domain of the known transcription factor. If the "bait" and the "prey" proteins are able to interact *in vivo,* forming an AAP-dependent complex, the DNA-binding and activation domains of the transcription factor are brought into close proximity. This proximity allows transcription of a reporter gene *(e.g.,* LacZ) that is operably-linked to a transcriptional regulatory site responsive to the transcription factor. Expression of the reporter gene can be detected, and cell colonies containing the functional transcription factor can be isolated and used to obtain the cloned gene that encodes the AAP-interacting protein.

The invention further pertains to novel agents identified by the aforementioned screening assays and uses thereof for treatments as described herein.

2. *Detection assays*

Portions or fragments of an *AAP* cDNA sequences identified herein (and the complete *AAP* gene sequences) are useful in themselves. By way of non-limiting example, these sequences can be used to: (1) identify an individual from a minute biological sample (tissue typing); and (2) aid in forensic identification of a biological sample.

(a) *Tissue typing*

The AAP sequences of the invention can be used to identify individuals from minute biological samples. In this technique, an individual's genomic DNA is digested with one or more restriction enzymes and probed on a Southern blot to yield unique bands. The sequences of the invention are useful as additional DNA markers for "restriction fragment length polymorphisms" (RFLP; (Smulson *et al.,* US Patent No. 5,272,057, 1993)).

Furthermore, the *AAP* sequences can be used to determine the actual base-by-base DNA sequence of targeted portions of an individual's genome. *AAP* sequences can be used to prepare two PCR primers from the 5'- and 3'-termini of the sequences that can then be used to amplify an the corresponding sequences from an individual's genome and then sequence the amplified fragment

Panels of corresponding DNA sequences from individuals can provide unique individual identifications, as each individual will have a unique set of such DNA sequences due to allelic differences. The sequences of the invention can be used to obtain such identification sequences from individuals and from tissue. The *AAP* sequences of the invention uniquely represent portions of an individual's genome. Allelic variation occurs to some degree in the coding regions of these sequences, and to a greater degree in the noncoding regions. The allelic variation between individual humans occurs with a frequency of about once ever 500 bases. Much of the allelic variation is due to single nucleotide polymorphisms (SNPs), which include RFLPs.

Each of the sequences described herein can, to some degree, be used as a standard against which DNA from an individual can be compared for identification purposes. Because greater numbers of polymorphisms occur in noncoding regions, fewer sequences are necessary to differentiate individuals. Noncoding sequences can positively identify individuals with a panel of 10 to 1,400 primers that each yield a noncoding amplified sequence of 100 bases. If predicted coding sequences, such as SEQ ID NO: 3 are used, a more appropriate number of primers for positive individual identification would be 500-2,000.

*Predictive medicine*

The invention also pertains to the field of predictive medicine in which diagnostic assays, prognostic assays, pharmacogenomics, and monitoring clinical trials are used for prognostic (predictive) purposes to treat an individual prophylactically. Accordingly, one aspect of the invention relates to diagnostic assays for determining an AAP and/or nucleic acid expression as well as AAP activity, in the context of a biological sample (*e.g.*, blood, serum, cells, tissue) to determine whether an individual is afflicted with a disease or disorder, or is at risk of developing a disorder, associated with aberrant AAP expression or activity, including cancer. The invention also provides for prognostic (or predictive) assays for determining whether an individual is at risk of developing a disorder associated with an AAP, nucleic acid expression or activity. For example, mutations in an *AAP* can be assayed in a biological sample. Such assays can be used for prognostic or predictive purpose to prophylactically treat an individual prior to the onset of a disorder characterized by or associated with the AAP, nucleic acid expression, or biological activity.

Another aspect of the invention provides methods for determining AAP activity, or nucleic acid expression, in an individual to select appropriate therapeutic or prophylactic agents for that individual (referred to herein as "pharmacogenomics"). Pharmacogenomics allows for the selection of modalities (*e.g.*, drugs, foods) for therapeutic or prophylactic treatment of an individual based on the individual's genotype (*e.g.*, the individual's genotype to determine the individual's ability to respond to a particular agent). Another aspect of the invention pertains to monitoring the influence of modalities (*e.g.*, drugs, foods) on the expression or activity of an AAP in clinical trials.

1. *Diagnostic assays*

An exemplary method for detecting the presence or absence of an AAP in a biological sample involves obtaining a biological sample from a subject and contacting the biological sample with a compound or an agent capable of detecting the AAP or the *AAP* nucleic acid (*e.g.*, mRNA, genomic DNA) such that the presence of the AAP is confirmed in the sample. An agent for detecting the *AAP* mRNA or genomic DNA is a labeled nucleic acid probe that can hybridize to the *AAP* mRNA or genomic DNA. The nucleic acid probe can be, for example, a full-length *AAP* nucleic acid, such as the nucleic acid of SEQ ID NO:3, or a portion thereof, such as an oligonucleotide of at least 15, 30, 50, 100, 250 or 500 nucleotides in length and sufficient to specifically hybridize under stringent conditions to an *AAP* mRNA or genomic DNA.

An agent for detecting an AAP polypeptide is an antibody capable of binding to the AAP, preferably an antibody with a detectable label. Abs can be polyclonal, or more preferably, monoclonal. An intact antibody, or a fragment (*e.g.*, $F_{ab}$ or $F(ab')_2$) can be used A labeled probe or antibody is coupled *(i.e.,* physically linking) to a detectable substance, as well as indirect detection of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" includes tissues, cells and biological fluids isolated from a subject, as well as tissues, cells and fluids present within a subject. The detection method of the invention can be used to detect an *AAP* mRNA, protein, or genomic DNA in a biological sample *in vitro* as well as *in vivo.* For example, *in vitro* techniques for detection of an *AAP* mRNA include Northern hybridizations and *in situ* hybridizations. *In vitro* techniques for detection of an AAP polypeptide include enzyme linked immunosorbent assays (ELISAs), Western blots, immunoprecipitations, and immunofluorescence. *In vitro* techniques for detection of an *AAP* genomic DNA include Southern hybridizations and fluorescence in situ hybridization (FISH). Furthermore, *in vivo* techniques for detecting an AAP include introducing into a subject a labeled anti-AAP antibody. For example, the antibody can be labeled with a radioactive marker whose presence and location in a subject can be detected by standard imaging techniques.

In one embodiment, the biological sample from the subject contains protein molecules, and/or mRNA molecules, and/or genomic DNA molecules. A preferred biological sample is blood.

In another embodiment, the methods further involve obtaining a biological sample from a subject to provide a control, contacting the sample with a compound or agent to detect an *AAP,* mRNA, or genomic DNA, and comparing the presence of the *AAP,* mRNA or genomic DNA in the control sample with the presence of the AAP, mRNA or genomic DNA in the test sample.

The invention also encompasses kits for detecting an AAP in a biological sample. For example, the kit can comprise: a labeled compound or agent capable of detecting an AAP or an *AAP* mRNA in a sample; reagent and/or equipment for determining the amount of an AAP in the sample; and reagent and/or equipment for comparing the amount of an AAP in the sample with a standard. The compound or agent can be packaged in a suitable container. The kit can further comprise instructions for using the kit to detect the AAP or nucleic acid.

2. *Prognostic assays*

The diagnostic methods described herein can furthermore be utilized to identify subjects having or at risk of developing a disease or disorder associated with an aberrant AAP expression or activity. For example, the assays described herein, can be used to identify a subject having or at risk of developing a disorder associated with AAP, nucleic acid expression or activity. Alternatively, the prognostic assays can be used to identify a subject having or at risk for developing a disease or disorder. Tthe invention provides a method for identifying a disease or disorder associated with an aberrant AAP expression or activity in which a test sample is obtained from a subject and the AAP or nucleic acid (*e.g.*, mRNA, genomic DNA) is detected. A test sample is a biological sample obtained from a subject. For example, a test sample can be a biological fluid (*e.g.*, serum), cell sample, or tissue.

Pognostic assays can be used to determine whether a subject can be administered a modality (*e.g.*, an agonist, antagonist, peptidomimetic, protein, peptide, nucleic acid, small molecule, food, *etc.*) to treat a disease or disorder associated with an aberrant AAP expression or activity. Such methods can be used to determine whether a subject can be effectively treated with an agent for a disorder. The invention provides methods for determining whether a subject can be effectively treated with an agent for a disorder associated with an aberrant AAP expression or activity in which a test sample is obtained and the AAP or nucleic acid is detected (*e.g.*, where the presence of the AAP or nucleic acid is diagnostic for a subject that can be administered the agent to treat a disorder associated with the aberrant AAP expression or activity).

The methods of the invention can also be used to detect genetic lesions in an *AAP* to determine if a subject with the genetic lesion is at risk for a disorder characterized by aberrant angiogenesis. Methods include detecting, in a sample from the subject, the presence or absence of a genetic lesion characterized by at an alteration affecting the integrity of a gene encoding an AAP polypeptide, or the mis-expression of an *AAP.* Such genetic lesions can be detected by ascertaining: (1) a deletion of one or more nucleotides from an *AAP;* (2) an addition of one or more nucleotides to an *AAP;* (3) a substitution of one or more nucleotides in an *AAP,* (4) a chromosomal rearrangement of an *AAP* gene; (5) an alteration in the level of an *AAP* mRNA transcripts, (6) aberrant modification of an *AAP,* such as a change genomic DNA methylation, (7) the presence of a non-wild-type splicing pattern of an *AAP* mRNA transcript, (8) a non-wild-type level of an *AAP,* (9) allelic loss of an *AAP,* and/or (10) inappropriate post-translational modification of an AAP polypeptide. There are a large number of known assay techniques that can be used to detect lesions in an *AAP*. Any biological sample containing nucleated cells may be used.

In certain embodiments, lesion detection may use a probe/primer in a polymerase chain reaction (PCR) *(e.g.,* (Mullis, US Patent No. 4,683,202, 1987; Mullis *et al.,* US Patent No. 4,683,195, 1987), such as anchor PCR or rapid amplification of cDNA ends (RACE) PCR, or, alternatively, in a ligation chain reaction (LCR) *(e.g.,* (Landegren *et al.,* 1988; Nakazawa *et al.,* 1994), the latter is particularly useful for detecting point mutations in *AAP*-genes (Abravaya *et al.,* 1995). This method may include collecting a sample from a patient, isolating nucleic acids from the sample, contacting the nucleic acids with one or more primers that specifically hybridize to an *AAP* under conditions such that hybridization and amplification of the *AAP* (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli *et al.,* 1990), transcriptional amplification system (Kwoh *et al.,* 1989); Qβ Replicase (Lizardi *et al.,* 1988), or any other nucleic acid amplification method, followed by the detection of the amplified molecules using techniques well known to those of skill in the art These detection schemes are especially useful for the detection of nucleic acid molecules present in low abundance.

Mutations in an *AAP* from a sample can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes can be used to score for the presence of specific mutations by development or loss of a ribozyme cleavage site.

Hybridizing a sample and control nucleic acids, *e.g.,* DNA or RNA, to high-density arrays containing hundreds or thousands of oligonucleotides probes, can identify genetic mutations in an *AAP* (Cronin *et al.,* 1996; Kozal *et al.,* 1996). For example, genetic mutations in an *AAP* can be identified in two-dimensional arrays containing light-generated DNA probes as described in Cronin, *et al.,* supra. Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence an *AAP* and detect mutations by comparing the sequence of the sample AAP-with the corresponding wild-type (control) sequence. Examples of sequencing reactions include those based on classic techniques (Maxam and Gilbert, 1977; Sanger *et al.,* 1977). Any of a variety of automated sequencing procedures can be used when performing diagnostic assays (Naeve *et al.,* 1995) including sequencing by mass spectrometry (Cohen *et al.,* 1996; Griffin and Griffin, 1993; Koster, WO94/16101, 1994).

Other methods for detecting mutations in an *AAP* include those in which protection from cleavage agents is used to detect mismatched bases in RNA/RNA or RNA/DNA heteroduplexes (Myers *et al.,* 1985). In general, the technique of "mismatch cleavage" starts by providing heteroduplexes formed by hybridizing (labeled) RNA or DNA containing a wild-type *AAP* sequence with potentially mutant RNA or DNA obtained from a sample. The double-stranded duplexes are treated with an agent that cleaves single-stranded regions of the duplex such as those that arise from base pair mismatches between the control and sample strands. For instance, RNA/DNA duplexes can be treated with RNase and DNA/DNA hybrids treated with $S_1$ nuclease to enzymatically digest the mismatched regions. In other embodiments, either DNA/DNA or RNA/DNA duplexes can be treated with hydroxylamine or osmium tetroxide and with piperidine in order to digest mismatched regions. The digested material is then separated by size on denaturing polyacrylamide gels to determine the mutation site (Grompe *et al.,* 1989; Saleeba and Cotton, 1993). The control DNA or RNA can be labeled for detection.

Mismatch cleavage reactions may employ one or more proteins that recognize mismatched base pairs in double-stranded DNA (DNA mismatch repair) in defined systems for detecting and mapping point mutations in an *AAP* cDNAs obtained from samples of cells. For example, the mutY enzyme of *E. coli* cleaves A at G/A mismatches and the thymidine DNA glycosylase from HeLa cells cleaves T at G/T mismatches (Hsu *et al.,* 1994). According to an exemplary embodiment, a probe based on a wild-type *AAP* sequence is hybridized to a cDNA or other DNA product from a test cell(s). The duplex is treated with a DNA mismatch repair enzyme, and the cleavage products, if any, can be detected from electrophoresis protocols or the like (Modrich *et al*., US Patent No. 5,459,039, 1995).

Electrophoretic mobility alterations can be used to identify mutations in an *AAP.* For example, single strand conformation polymorphism (SSCP) may be used to detect differences in electrophoretic mobility between mutant and wild type nucleic acids (Cotton, 1993; Hayashi, 1992; Orita *et al.,* 1989). Single-stranded DNA fragments of sample and control *AAP* nucleic acids are denatured and then renatured. The secondary structure of single-stranded nucleic acids varies according to sequence; the resulting alteration in electrophoretic mobility allows detection of even a single base change. The DNA fragments may be labeled or detected with labeled probes. The sensitivity of the assay may be enhanced by using RNA (rather than DNA), in which the secondary structure is more sensitive to a sequence changes. The subject method may use heteroduplex analysis to separate double stranded heteroduplex molecules on the basis of changes in electrophoretic mobility (Keen *et al.,* 1991).

The migration of mutant or wild-type fragments can be assayed using denaturing gradient gel electrophoresis (DGGE; (Myers *et al*., 1985). In DGGE, DNA is modified to prevent complete denaturation, for example by adding a GC clamp of approximately 40 bp of high-melting GC-rich DNA by PCR. A temperature gradient may also be used in place of a denaturing gradient to identify differences in the mobility of control and sample DNA (Rossiter and Caskey, 1990).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found (Saiki *et al.,* 1986; Saiki *et al.,* 1989). Such allele-specific oligonucleotides are hybridized to PCR-amplified target DNA or a number of different mutations when the oligonucleotides are attached to the hybridizing membrane and hybridized with labeled target DNA.

Alternatively, allele specific amplification technology that depends on selective PCR amplification may be used. Oligonucleotide primers for specific amplifications may carry the mutation of interest in the center of the molecule (so that amplification depends on differential hybridization (Gibbs *et al.,* 1989)) or at the extreme 3'-terminus of one primer where, under appropriate conditions, mismatch can prevent, or reduce polymerase extension (Prosser, 1993). Novel restriction site in the region of the mutation may be introduced to create cleavage-based detection (Gasparini *et al.,* 1992). Certain amplification may also be performed using *Taq* ligase for amplification (Barany, 1991). In such cases, ligation occurs only if there is a perfect match at the 3'-terminus of the 5' sequence, allowing detection of a known mutation by scoring for amplification.

The described methods may be performed, for example, by using pre-packaged kits comprising at least one probe (nucleic acid or antibody) that may be conveniently used, for example, in clinical settings to diagnose patients exhibiting symptoms or family history of a disease or illness involving an AAP.

Furthermore, any cell type or tissue in which an AAP is expressed may be utilized in the prognostic assays described herein.

3. *Pharmacogenomics*

Agents, or modulators that have a stimulatory or inhibitory effect on AAP activity or expression, as identified by a screening assay can be administered to individuals to treat, prophylactically or therapeutically, disorders, including those associated with angiogenesis. In conjunction with such treatment, the pharmacogenomics (*i.e.*, the study of the relationship between a subject's genotype and the subject's response to a foreign modality, such as a food, compound or drug) may be considered Metabolic differences of therapeutics can lead to severe toxicity or therapeutic failure by altering the relation between dose and blood concentration of the pharmacologically active drug. Thus, the pharmacogenomics of the individual permits the selection of effective agents (*e.g.*, drugs) for prophylactic or therapeutic treatments based on a consideration of the individual's genotype. Pharmacogenomics can further be used to determine appropriate dosages and therapeutic regimens. Accordingly, the activity of an AAP, expression of an *AAP* nucleic acid, or an *AAP* mutation(s) in an individual can be determined to guide the selection of appropriate agent(s) for therapeutic or prophylactic treatment.

Pharmacogenomics deals with clinically significant hereditary variations in the response to modalities due to altered modality disposition and abnormal action in affected persons (Eichelbaum and Evert, 1996; Linder *et al.,* 1997). In general, two pharmacogenetic conditions can be differentiated: (1) genetic conditions transmitted as a single factor altering the interaction of a modality with the body (altered drug action) or (2) genetic conditions transmitted as single factors altering the way the body acts on a modality (altered drug metabolism). These pharmacogenetic conditions can occur either as rare defects or as nucleic acid polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g.*, N-acetyltransferase 2 (NAT 2) and cytochrome P450 enzymes CYP2D6 and CYP2C19) explains the phenomena of some patients who show exaggerated drug response and/or serious toxicity after taking the standard and safe dose of a drug. These polymorphisms are expressed in two phenotypes in the population, the extensive metabolizer (EM) and poor metabolizer (PM). The prevalence of PM is different among different populations. For example, the *CYP2D6* gene is highly polymorphic and several mutations have been identified in PM, which all lead to the absence of functional CYP2D6. Poor metabolizers due to mutant *CYP2D6* and *CYP2C19* frequently experience exaggerated drug responses and side effects when they receive standard doses. If a metabolite is the active therapeutic moiety, PM shows no therapeutic response, as demonstrated for the analgesic effect of codeine mediated by its CYP2D6-formed metabolite morphine. At the other extreme are the so-called ultra-rapid metabolizers who are unresponsive to standard doses. Recently, the molecular basis of ultra-rapid metabolism has been identified to be due to CYP2D6 gene amplification.

The activity of an AAP, expression of an *AAP* nucleic acid, or mutation content of an *AAP* in an individual can be determined to select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with an AAP modulator, such as a modulator identified by one of the described exemplary screening assays.

4. *Monitoring effects during clinical trials*

Monitoring the influence of agents (*e.g.*, drugs, compounds) on the expression or activity of an AAP (*e.g.*, the ability to modulate angiogenesis) can be applied not only in basic drug screening, but also in clinical trials. For example, the effectiveness of an agent determined by a screening assay to increase an *AAP* expression, protein levels, or up-regulate an AAP's activity can be monitored in clinical trails of subjects exhibiting decreased *AAP* expression, protein levels, or down-regulated AAP activity. Alternatively, the effectiveness of an agent determined to decrease an *AAP* expression, protein levels, or down-regulate an AAP's activity, can be monitored in clinical trails of subjects exhibiting increased the *AAP* expression, protein levels, or up-regulated AAP activity. In such clinical trials, the expression or activity of the AAP and, preferably, other genes that have been implicated in, for example, angiogenesis can be used as a "read out" or markers for a particular cell's responsiveness.

For example, genes, including an *AAP,* that are modulated in cells by treatment with a modality (*e.g.*, food, compound, drug or small molecule) can be identified. To study the effect of agents on angiogenesis, for example, in a clinical trial, cells can be isolated and RNA prepared and analyzed for the levels of expression of an *AAP* and other genes implicated in the disorder. The gene expression pattern can be quantified by Northern blot analysis, nuclear run-on or RT-PCR experiments, or by measuring the amount of protein, or by measuring the activity level of the AAP or other gene products. In this manner, the gene expression pattern itself can serve as a marker, indicative of the cellular physiological response to the agent Accordingly, this response state may be determined before, and at various points during, treatment of the individual with the agent.

The invention provides but does not claim a method for monitoring the effectiveness of treatment of a subject with an agent (*e.g.*, an agonist, antagonist, protein, peptide, peptidomimetic, nucleic acid, small molecule, food or other drug candidate identified by the screening assays described herein) comprising the steps of (1) obtaining a pre-administration sample from a subject; (2) detecting the level of expression of an AAP, mRNA, or genomic DNA in the preadministration sample; (3) obtaining one or more post-administration samples from the subject; (4) detecting the level of expression or activity of the AAP, mRNA, or genomic DNA in the post-administration samples; (5) comparing the level of expression or activity of the AAP, mRNA, or genomic DNA in the pre-administration sample with the AAP, mRNA, or genomic DNA in the post administration sample or samples; and (6) altering the administration of the agent to the subject accordingly. For example, increased administration of the agent may be desirable to increase the expression or activity of the AAP to higher levels than detected, *i.e.*, to increase the effectiveness of the agent. Alternatively, decreased administration of the agent may be desirable to decrease expression or activity of the AAP to lower levels than detected, *i.e.*, to decrease the effectiveness of the agent.

5. *Methods of treatment*

The invention provides but does not claim for both prophylactic and therapeutic methods of treating a subject at risk of (or susceptible to) a disorder or having a disorder associated with aberrant AAP expression or activity. Furthermore, these same methods of treatment may be used to induce or inhibit angiogenesis, by changing the level of expression or activity of an AAP.

6. *Disease and disorders*

Diseases and disorders that are characterized by increased AAP levels or biological activity may be treated with therapeutics that antagonize *(i.e.,* reduce or inhibit) activity. Antognists may be administered in a therapeutic or prophylactic manner. Therapeutics that may be used include: (1) AAP peptides, or analogs, derivatives, fragments or homologs thereof; (2) Abs to an AAP peptide; (3) *AAP* nucleic acids; (4) administration of antisense nucleic acid and nucleic acids that are "dysfunctional" (*i.e.*, due to a heterologous insertion within the coding sequences) that are used to eliminate endogenous function of by homologous recombination (Capecchi, 1989); or (5) modulators (*i.e.,* inhibitors, agonists and antagonists, including additional peptide mimetic of the invention or Abs specific to an AAP) that alter the interaction between an AAP and its binding partner.

Diseases and disorders that are characterized by decreased AAP levels or biological activity may be treated with therapeutics that increase (*i.e.*, are agonists to) activity. Therapeutics that upregulate activity may be administered therapeutically or prophylactically. Therapeutics that may be used include peptides, or analogs, derivatives, fragments or homologs thereof; or an agonist that increases bioavailability.

Increased or decreased levels can be readily detected by quantifying peptide and/or RNA, by obtaining a patient tissue sample *(e.g.,* from biopsy tissue) and assaying *in vitro* for RNA or peptide levels, structure and/or activity of the expressed peptides (or *AAP* mRNAs). Methods include, but are not limited to, immunoassays *(e.g.,* by Western blot analysis, immunoprecipitation followed by sodium dodecyl sulfate (SDS) polyacrylamide gel electrophoresis, immunocytochemistry, *etc.)* and/or hybridization assays to detect expression of mRNAs (*e.g.*, Northern assays, dot blots, in situ hybridization, and the like).

7. *Prophylactic methods*

The invention provides but does not claim method for preventing, in a subject, a disease or condition associated with an aberrant AAP expression or activity, by administering an agent that modulates an AAP expression or at least one AAP activity. Subjects at risk for a disease that is caused or contributed to by an aberrant AAP expression or activity can be identified by, for example, any or a combination of diagnostic or prognostic assays. Administration of a prophylactic agent can occur prior to the manifestation of symptoms characteristic of the AAP aberrancy, such that a disease or disorder is prevented or, alternatively, delayed in its progression Depending on the type of AAP aberrancy, for example, an AAP agonist or AAP antagonist can be used to treat the subject. The appropriate agent can be determined based on screening assays.

*8. Therapeutic methods*

Another aspect of the invention which is not claimed pertains to methods of modulating an AAP expression or activity for therapeutic purposes. The modulatory method of the invention involves contacting a cell with an agent that modulates one or more of the activities of AAP activity associated with the cell An agent that modulates AAP activity can be a nucleic acid or a protein, a naturally occurring cognate ligand of an AAP, a peptide, an AAP peptidomimetic, or other small molecule. The agent may stimulate AAP activity. Examples of such stimulatory agents include an active AAP and an *AAP* nucleic acid molecule that has been introduced into the cell. In another embodiment, the agent inhibits AAP activity. Examples of inhibitory agents include antisense *AAP* nucleic acids and anti-AAP Abs. Modulatory methods can be performed *in vitro* (*e.g.,* by culturing the cell with the agent) or, alternatively, *in vivo* (*e.g.,* by administering the agent to a subject). As such, the invention provides methods of treating an individual afflicted with a disease or disorder characterized by aberrant expression or activity of an AAP or nucleic acid molecule. In one embodiment, the method involves administering an agent (*e.g.*, an agent identified by a screening assay), or combination of agents that modulates (*e.g.*, up-regulates or down-regulates) AAP expression or activity. In another embodiment, the method involves administering an AAP or nucleic acid molecule as therapy to compensate for reduced or aberrant AAP expression or activity.

Stimulation of AAP activity is desirable in situations in which AAP is abnormally down-regulated and/or in which increased AAP activity is likely to have a beneficial effect. One example of such a situation is where a subject has a disorder characterized by aberrant angiogenesis (*e.g.*, cancer).

9. *Determination of the biological effect of the therapeutic*

Suitable *in vitro* or *in vivo* assays can be performed to determine the effect of a specific therapeutic and whether its administration is indicated for treatment of the affected tissue.

In various specific embodiments, *in vitro* assays may be performed with representative cells of the type(s) involved in the patient's disorder, to determine if a given therapeutic exerts the desired effect upon the cell type(s). Modalities for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

10. *Prophylactic and therapeutic uses, of the compositions of the invention*

AAP nucleic acids and proteins are useful in potential prophylactic and therapeutic applications implicated in a variety of disorders including, but not limited to those related to angiogenesis.

As an example, a cDNA encoding an AAP may be useful in gene therapy, and the protein may be useful when administered to a subject in need thereof

*AAP* nucleic acids, or fragments thereof, may also be useful in diagnostic applications, wherein the presence or amount of the nucleic acid or the protein is to be assessed. A further use could be as an anti-bacterial molecule *(i.e.,* some peptides have been found to possess anti-bacterial properties). These materials are further useful in the generation of Abs that immunospecifically bind to the novel substances of the invention for use in therapeutic or diagnostic methods.

The following example is meant to not be limiting.

**EXAMPLE**

*Identification of genes differentially-regulated*

A comprehensive mRNA profiling technique (GeneCalling) was used to determine differential gene expression profiles of human endothelial cells undergoing differentiation into tube-like structures (Kahn *et al.,* 2000). To confirm the expression data from GeneCalling, independent experiments were undertaken that used gene-specific PCR oligonucleotide primer pairs and an oligonucleotide probe labeled with a fluorescent dye at the 5' end and quencher fluorescent dye at the 3' end.. Total RNA (50 ng) was added to a 50 μl RT-PCR mixture and run. The following data were collected:

| | | |
|---|---|---|
| hEF G | collagen gel 24 hr versus 4h | 4.5 fold upregulated |
| hTRG | collagen gel 24 hr versus 4h | 3.5 fold upregulated |
| KLP | collagen gel 24 hr versus 4h | 3.5 fold upregulated |
| myosin X | collagen gel 24 hr versus 4h | 3.5 fold upregulated |
| NHR | collagen gel 24 hr versus 4h | 7.3 fold downregulated |
| HBAZF | collagen gel 24 hr versus 4h | 2.1 fold upregulated |

**REFERENCES**

U.S. Patent No. 4166452. Apparatus for testing human responses to stimuli. 1979.
U.S. Patent No. 4485045. Synthetic phosphatidyl cholines useful in forming liposomes. 1984.
U.S. Patent No. 4544545. Liposomes containing modified cholesterol for organ targeting. 1985.
4,676,980. Target specific cross-linked heteroantibodies. 1987.

U.S. Patent No. 4816567. Recombinant immunoglobin preparations. 1989.
WO 90/10448. Covalent conjugates of lipid and oligonucleotide. 1990.
WO 90/13641. Stably transformed eucaryotic cells comprisng a foreign transcribable DNA under the control of a pol III promoter. 1990.
EPO 402226. Transformation vectors for yeast *Yarrowia.* 1990.
WO 91/00360. Bispecific reagents for AIDS therapy. 1991.
WO 91/04753. Conjugates of antisense oligonucleotides and therapeutic uses thereof. 1991.
U.S. Patent No. 5013556. Liposomes with enhanced circulation time. 1991.
WO 91/00357. New strain with filamentous fungi mutants, process for the production of recombinant proteins using said strain, and strains and proteins. 1991.
WO 91/06629. Oligonucleotide analogs with novel linkages. 1991.
WO 92/20373. Heteroconjugate antibodies for treatment of HIV infection. 1992.
WO 93/08829. Compositions that mediate killing of HIV-infected cells. 1993.
WO 94/11026. Therapeutic application of chimeric and radiolabeled antibodies to human B lymphocyte restricted differentiation antigen for treatment of B cells. 1994.
WO 96/27011. A method for making heteromultimeric polypeptides. 1996.
U.S. Patent No. 5545807. Production of antibodies from transgenic animals. 1996.
U.S. Patent No. 5545806. Ransgenic <sic> non-human animals for producing heterologous antibodies. 1996.
U.S. Patent No. 5569825. Transgenic non-human animals capable of producing heterologous antibodies of various isotypes. 1996.
WO 97/33551. Compositions and methods for the diagnosis, prevention, and treatment of neoplastic cell growth and proliferation. 1997.
U.S. Patent No. 5633425. Transgenic non-human animals capable of producing heterologous antibodies. 1997.
U.S. Patent No. 5661016. Transgenic non-human animals capable of producing heterologous antibodies of various isotypes. 1997.
U.S. Patent No. 5625126. Transgenic non-human animals for producing heterologous antibodies. 1997.
Abravaya, K., J.J. Carrino, S. Muldoon, and H.H. Lee. 1995. Detection of point mutations with a modified ligase chain reaction (Gap- LCR). *Nucleic Acids Res.* 23:675-82.
Adams, J., R. Kelso, and L. Cooley. 2000. The kelch repeat superfamily of proteins: propellers of cell function. *Trends Cell Biol.* 10:17-24.
Alam, J., and J.L. Cook. 1990. Reporter genes: Application to the study of mammalian gene transcription. *Anal. Biochem.* 188:245-254:
Albagli, O., D. Lantoine, S. Quief, F. Quignon, *et al.* 1999. Overexpressed BCL6 (LAZ3) . oncoprotein triggers apoptosis, delays S phase progression and associates with replication foci. *Oncogene.* 18:5063-75.
Alberts, B., D. Bray, J. Lewis, M. Raff, *et al.* 1994. Molecular Biology of the Cell. Garland Publishing, Inc., New York, NY. 1294 pp.
Altschul, S.F., T.L. Madden, A.A. Schaffer; J. Zhang, *et al.* 1997. Gapped BLAST and PSI-BLAST: a new generation of protein database search programs. *Nucleic Acids Res.* 25:3389-402.
Austin, C.P., and C.L. Cepko. 1990. Cellular migration patterns in the developing mouse cerebral cortex. *Development.* 110:713-732.
Ausubel, F.M., R. Brent, R.E. Kingston, D.D. *Moore, et al.* 1987. Current protocols in molecular biology. John Wiley & Sons, New York.
Barany, F. 1991. Genetic disease detection and DNA amplification using cloned thermostable ligase. *Proc Natl Acad Sci USA.* 88:189-93.
Barker, C., A. Makris, C. Patriotis, S.E. Bear, *et al.* 1993. Identification of the gene encoding the mitochondrial elongation factor G in mammals. *Nucleic Acids Res.* 21:2641-7.
Bartel, D.P., and J.W. Szostak. 1993. Isolation of new ribozymes from a large pool of random sequences [see comment]. *Science.* 261:1411-8.
Bartel, P., C.T. Chien, R. Stemglanz, and S. Fields. 1993. Elimination of false positives that arise in using the two-hybrid system. *Biotechniques.* 14:920-4.
Beal, P.A., and P.B. Dervan. 1991. Second structural motif for recognition of DNA by oligonucleotide- directed triple-helix formation. *Science.* 251:1360-3.
Bechtold, N., and G. Pelletier. 1998. In planta Agrobacterium-mediated transformation of adult Arabidopsis thaliana plants by vacuum infiltration. *Methods Mol Biol.* 82:259-66.
Becker, D.M., and L. Guarente. 1991. High-efficiency transformation of yeast by electroporation. *Methods Enzymol.* 194:182-187.
Beggs, J.D. 1978. Transformation of yeast by a replicating hybrid plasmid. *Nature.* 275:104-109.
Berg, J.S., B.H. Derfler, C.M. Pennisi, D.P. Corey, *et al.* 2000. Myosin-X, a novel myosin with pleckstrin homology domains, associates with regions of dynamic actin. *J Cell Sci.* 113 Pt 19:3439-51.
Berger, J., J. Hauber, R Hauber, R. Geiger, *et al.* 1988. Secreted placental alkaline phosphatase: A powerful new qunatitative indicator of gene expression in eukaryotic cells. *Gene.* 66:1-10.
WO 93/04169. GENE TARGETING IN ANIMAL CELLS USING ISOGENIC DNA CONSTRUCTS. 1993.
Bodine, D.M., K.T. McDonagh, N.E. Seidel, and A.W. Nienhuis. 1991. Survival and retrovirus infection of murine hematopoietic stem cells in vitro: effects of 5-FU and method of infection. *Exp. Hematol.* 19:206-212;
Boerner, P., R. Lafond, W.Z. Lu, P. Brams, *et al.* 1991. Production of antigen-specific human monoclonal antibodies from in vitro-primed human splenocytes. *J Immunol.* 147:86-95.
Bonapace, I.M., M. Sanchez, S. Obici, A. Gallo, *et al.* 1990. Extinction and activation of the thyroglobulin promoter in hybrids of differentiated and transformed thyroid cells. *Mol Cell Biol.* 10:1033-40.
U.S. Patent No. 3,773,919. Polylactide-drug mixtures. 1973.
Bradley. 1987. Teratocarcinomas and Embryonic Stem Cells: A Practical Approach. Oxford University Press, Inc., Oxford. 268 pp.
Bradley, A. 1991. Modifying the mammalian genome by gene targeting. *Curr Opin Biotechnol.* 2:823-9.
Brennan, M., P.F. Davison, and H. Paulus. 1985. Preparation of bispecific antibodies by chemical recombination of monoclonal immunoglobulin G1 fragments. *Science.* 229:81-3.
WO94/10300. INTERACTION TRAP SYSTEM FOR ISOLATING NOVEL PROTEINS. 1994.
Capecchi, M.R. 1980. High efficiency transformation by direct microinjection of DNA into cultured mammalian cells. *Cell.* 22:479.
Capecchi, M.R. 1989. Altering the genome by homologous recombination. *Science.* 244:1288-92.
Carell, T., E.A. Wintner, and J. Rebek Jr. 1994a. A novel procedure for the synthesis of libraries containing small organic molecules. *Angewandte Chemie International Edition.* 33:2059-2061.
Carell, T., E.A. Wintner, and J. Rebek Jr. 1994b. A solution phase screening procedure for the isolation of active compounds from a molecular library. *Angewandte. Chemie International Edition.* 33:2061-2064.
Caron, P.C., W. Laird, M.S. Co, N.M. Avdalovic, *et al.* 1992. Engineered humanized dimeric forms of IgG are more effective antibodies. *J Exp Med.* 176:1191-5.
Carter, P. 1986. Site-directed mutagenesis. *Biochem J.* 237:1-7.
Case, M.E., M. Schweizer, S.R. Kushner, and N.H. Giles. 1979. Efficient transformation of Neurospora crassa by utilizing hybrid plasmid DNA. *Proc Natl Acad Sci USA.* 76:5259-63.
U.S. Patent No. 5,116,742. RNA ribozyme restriction endoribonucleases and methods. 1992.
U.S. Patent No. 4,987,071. RNA ribozyme polymerases, dephosphorylases, restriction endoribonucleases and methods. 1991.
Cepko, C.L., B.E. Roberts, and R.E. Mulligan. 1984. Construction and applications of a highly transmissible murine retrovirus shuttle vector. *Cell.* 37:1053-1062.
Chalfie, M., Y. tu, G. Euskirchen, W.W. Ward, *et al.* 1994. Green fluorescent protein as a marker for gene expression. *Science.* 263:802-805.
Chaney, W.G., D.R. Howard, J.W. Pollard, S. Sallustio, *et al.* 1986. High-frequency transfection of CHO cells using Polybrene. *Somatic Cell Mol. Genet.* 12:237.
Chen, C., and H. Okayama. 1988. Calcium phosphate-mediated gene transfer: A highly efficient system for stably transforming cells with plasmid DNA. *BioTechniques.* 6:632-638.
Chen; S.H., H.D. Shine, J.C. Goodman, R.G. Grossman, *et al.* 1994. Gene therapy for brain tumors: regression of experimental gliomas by adenovirus-mediated gene transfer in vivo. *Proc Natl Acad Sci USA.* 91:3054-7.
Cho, C.Y., E.J. Moran, S.R. Cherry, J.C. Stephans, *et al.* 1993. An unnatural biopolymer. *Science.* 261:1303-5.
Cohen, A.S., D.L. Smisek, and B.H. Wang. 1996. Emerging technologies for sequencing antisense oligonucleotides: capillary electrophoresis and mass spectrometry. *Adv Chromatogr.* 36:127-62.
Cohen, J.S. 1989. Oligodeoxynucleotides: Antisense inhibitors of gene expression. CRC Press, Boca Raton, FL. 255 pp.
Cohen, S.M.N., A.C.Y. Chang, and L. Hsu. 1972. Nonchromosomal antibiotic resistance in bacteria: Genetic transformation of *Escherichia coli* by R-factor DNA. *Proc. Natl. Acad. Sci. USA.* 69:2110.
Cooney, M., G. Czernuszewicz, E.H. Postel, S.J. Flint, *et al.* 1988. Site-specific oligonucleotide binding represses transcription of the human c-myc gene in vitro. *Science.* 241:456-9.
Cotton, R.G. 1993. Current methods of mutation detection. *Mutat Res.* 285:125-44.
Cronin, M.T., R.V. Fucini, S.M. Kim, R.S. Masino, *et al.* 1996. Cystic fibrosis mutation detection by hybridization to light-generated DNA probe arrays. *Hum Mutat.* 7:244-55.
Cull, M.G., J.F. Miller, and P.J. Schatz. 1992. Screening for receptor ligands using large libraries of peptides linked to the C terminus of the lac repressor. *Proc Natl Acad Sci USA.* 89:1865-9.
Cwirla, S.E., E.A. Peters, R.W. Barrett, and W.J. Dower. 1990. Peptides on phage: a vast library of peptides for identifying ligands. *Proc Natl Acad Sci USA.* 87:6378-82.

de Boer, A.G. 1994. Drug absorption enhancement: Concepts, possibilities, limitations and trends. Harwood Academic Publishers, Langhome, PA.
de Louvencourt, L., H. Fukuhara, H. Heslot, and M. Wesolowski. 1983. Transformation of Kluyveromyces lactis by killer plasmid DNA. *J Bacteriol.* 154:737-42.
de Wet, J.R., K.V. Wood, M. DeLuca, D.R. Helinski, *et al.* 1987. Sturcture and expression in mammalian cells. *Mol. Cell BioL* 7:725-737.
Demerec, M., E.A. Adelberg, A.J. Clark, and P.E. Hartman. 1966. A proposal for a uniform nomenclature in bacterial genetics. *Genetics.* 54:61-76.
Devlin, J.J., L.C. Panganiban, and P.E. Devlin. 1990. Random peptide libraries: a source of specific protein binding molecules. *Science.* 249:404-6.
De Witt, S.H., J.S. Kiely, C.J. Stankovic, M.C. Schroeder, *et al.* 1993. "Diversomers": an approach to nonpeptide, nonoligomeric chemical diversity. *Proc Natl Acad Sci U S A.* 90:6909-13.
Eichelbaum, M., and B. Evert. 1996. Influence of pharniacogenetics on drug disposition and response. *Clin Exp Pharmacol Physiol.* 23:983-5.
Ellington, A.D., and J.W. Szostak. 1990. In vitro selection of RNA molecules that bind specific ligands. *Nature.* 346: 818-22.
Elroy-Stein, O., and B. Moss. 1990. Cytoplasmic expression system based on constitutive synthesis of bacteriophage T7 RNA polymerase in mammalian cells. *Proc. Natl. Acad. Sci. USA.* 87:6743-6747.
US Patent No. 4,522,811. Serial injection of muramyldipeptides and liposomes enhances the anti-infective activity of muramyldipeptides Serial injection of muramyldipeptides and liposomes enhances the anti-infective activity of muramyldipeptides. 1985.
Eppstein, D.A., Y.V: Marsh, M. van der Pas, P.L. Felgner, *et al.* 1985. Biological activity of liposome-encapsulated murine interferon gamma is mediated by a cell membrane receptor. *Proc Natl Acad Sci USA.* 82:3688-92.
Escudero, J., and B. Hohn. 1997. Transfer and integration ofT-DNA without cell injury in the host plant. *Plant Cell.* 9:2135-2142.
U.S. Patent No. 4,870,009. Method of obtaining gene product through the generation of transgenic animals. 1989.
Fekete, D.M., and C.L. Cepko. 1993. Retroviral infection coupled with tissue transplantation limits gene transfer in the chick embryo. *Proc. Natl. Acad. Sci. USA.* 90:2350-2354.
Felgner, P.L., T.R. Gadek, M. Holm, R. Roman, *et al.* 1987. Lipofectin: A highly efficient, lipid-mediated DNA/ transfection procedure. *Proc. Natl. Acad Sci. USA.* 84:7413-7417.
Felici, F., L. Castagnoli, A. Musacchio, R. Jappelli, *et al.* 1991. Selection of antibody ligands from a large library of oligopeptides expressed on a multivalent exposition vector. *J Mol Biol.* 222:301-10.
Fieck, A., D.L. Wyborski, and J.M. Short. 1992. Modifications of the E.coli Lac repressor for expression in eukaryotic cells: effects of nuclear signal sequences on protein activity and nuclear accumulation. *Nucleic Acids Res.* 20: 1785-91.
Finer, J.J., K.R. Finer, and T. Ponappa. 1999. Particle bombardment-mediated transformation. *Current Topics in microbiology and immunology.* 240:59-80.
Finn, P.J., N.J. Gibson, R. Fallon, A. Hamilton, *et al.* 1996. Synthesis and properties of DNA-PNA chimeric oligomers. *Nucleic Acids Res.* 24:3357-63.
Fishwild, D.M., S.L. O'Donnell, T. Bengoechea, D.V. Hudson, *et al.* 1996. High-avidity human IgG kappa monoclonal antibodies from a novel strain of minilocus transgenic mice [see comments]. *Nat Biotechnol.* 14:845-51.
Fleer, R., P. Yeh, N. Amellal, I. *Maury, et al.* 1991. Stable multicopy vectors for high-level secretion of recombinant human serum albumin by Kluyveromyces yeasts. *Biotechnology (NY).* 9:968-75.
Fodor, S.P., R.P. Rava, X.C. Huang, A.C. Pease, *et al.* 1993. Multiplexed biochemical assays with biological chips. *Nature.* 364:555-6.
Fromm, M., L.P. Taylor, and V. Walbot. 1985. Expression of genes transferred into monocot and dicot plant cells by electroporation. *Proc. Natl. Acad. Sci. USA.* 82:5824-5828.
Fujita, T., H. Shubiya, T. Ohashi, K. Yamanishi, *et al.* 1986. Regulation of human interleukin-2 gene: Functional DNA sequences in the 5' flanking region for the gene expression in activated T lymphocytes. *Cell.* 46:401-407.
Gabizon, A., R. Shiota, and D. Papahadjopoulos. 1989. Pharmacokinetics and tissue distribution of doxorubicin encapsulated in stable liposomes with long circulation times. *J Natl Cancer Inst.* 81:1484-8.
Gallagher, S.R. 1992. GUS protocols: Using the GUS gene as a reporter of gene expression. Academic Press, San Diego, CA.
Gallop, M.A., R.W. Barrett, W.J. Dower, S.P. Fodor, *et al.* 1994. Applications of combinatorial technologies to drug discovery. 1. Background and peptide combinatorial libraries. *J Med Chem.* 37:1233-51.
Gasparini, P., A. Bonizzato, M. Dognini, and P.F. Pignatti. 1992. Restriction site generating-polymerase chain reaction (RG-PCR) for the probeless detection of hidden genetic variation: application to the study of some common cystic fibrosis mutations. *Mol Cell Probes.* 6:1-7.

Gautier, C., F. Morvan, B. Rayner, T. Huynh-Dinh, *et al.* 1987. Alpha-DNA. IV: Alpha-anomeric and beta-anomeric tetrathymidylates covalently linked to intercalating oxazolopyridocarbazole. Synthesis, physicochemical properties and poly (rA) binding. *Nucleic Acids Res.* 15:6625-41.
Gennaro, A.R 2000. Remington: The science and practice of pharmacy. Lippincott, Williams & Wilkins, Philadelphia, PA.
Gibbs, RA., P.N. Nguyen, and C.T. Caskey. 1989. Detection of single DNA base differences by competitive oligonucleotide priming. *Nucleic Acids Res.* 17:2437-48.
Gietz, R.D., R.A. Woods, P. Manivasakam, and R.H. Schiestl. 1998. Growth and transformation of *Saccharomyces cerevisiae. In* Cells: A laboratory manual. Vol. I. D. Spector, R. Goldman, and L. Leinwand, editors. Cold Spring Harbor Press, Cold Spring Harbor, NY.
Goding, J.W. 1996. Monoclonal antibodies: Principles and Practice. Academic Press, San Diego. 492 pp.
Gorman, C.M., L.F. Moffat, and B.H. Howard. 1982. Recombinant genomes which express chloramphenicol acetyltransferase in mammalian cells. *Mol. Cell. Biol.* 2:1044-1051.
Graham, F.L., and A.J. van der Eb. 1973. A new technique for the assay of infectivity of human adenovirus 5 DNA. *Virology.* 52:456-.
Griffin, H.G., and A.M. Griffin. 1993. DNA sequencing. Recent innovations and future trends. *Appl Biochem Biotechnol.* 38:147-59.
Grompe, M., D.M. Muzny, and C.T. Caskey. 1989. Scanning detection of mutations in human ornithine transcarbamoylase by chemical mismatch cleavage. *Proc Natl Acad Sci USA.* 86:5888-92.
Gruber, M., B.A. Schodin, E.R. Wilson, and D.M. Kranz. 1994. Efficient tumor cell lysis mediated by a bispecific single chain antibody expressed in Escherichia coli. *J Immunol.* 152:5368-74.
Guatelli, J.C., K.M. Whitfield, D.Y. Kwoh, K.J. Barringer, *et al.* 1990. Isothermal, in vitro amplification of nucleic acids by a multienzyme reaction modeled after retroviral replication. *Proc Natl Acad Sci USA.* 87:1874-8.
Hanahan, D. 1983. Studies on transformation of *Escherichia coli* with plasmids. *J. Mol. Biol.* 166:557-580.
Hansen, G., and M.-D. Chilton. 1999. Lessons in gene transfer to plants by a gifted microbe. *Curr. Top. Microbiol. Immunol.* 240:21-57.
Hansen, G., and M.S. Wright. 1999. Recent advances in the transformation of plants. *Trends Plant Sci.* 4:226-231.
Harlow, E., and D. Lane. 1988. Antibodies: A laboratory manual. Cold Spring Harbor Laboratory Press, Cold Spring Harbor. 726 pp.
Harlow, E., and D. Lane. 1999. Using antibodies: A laboratory manual. Cold Spring Harbor Laboratory PRess, Cold Spring Harbor, New York.
Haseloff, J., and W.L. Gerlach. 1988. Simple RNA enzymes with new and highly specific endoribonuclease activities. *Nature.* 334:585-91.
Hayashi, K. 1992. PCR-SSCP: A method for detection of mutations. *Genetic and Analytical Techniques Applications.* 9:73-79.
Helene, C. 1991. The anti-gene strategy: control of gene expression by triplex-forming-oligonucleotides. *Anticancer Drug Des.* 6:569-84.
Helene, C., N.T. Thuong, and A. Harel-Bellan. 1992. Control of gene expression by triple helix-forming oligonucleotides. The antigene strategy. *Ann N YAcad Sci.* 660:27-36.
Hibi, M., and T. Hirano. 2000. Gab-family adapter molecules in signal transduction of cytokine and growth factor receptors, and T and B cell antigen receptors. Leuk *Lymphoma.* 37:299-307.
Hinnen, A., J.B. Hicks, and G.R. Fink. 1978. Transformation of yeast. *Proc. Natl. Acad. Sci. USA.* 75:1929-1933.
Hoffman, F. 1996. Laser microbeams for the manipulation of plant cells and subcellular structures. *Plant Sci.* 113: 1-11.
Hogan, B., Beddington, R., Costantini, F., Lacy, E. 1994. Manipulating the Mouse Embryo: A Laboratory Manual. Cold Spring Harbor Laboratory Press. 500 pp.
Holliger, P., T. Prospero, and G. Winter. 1993. "Diabodies": small bivalent and bispecific antibody fragments. *Proc Natl Acad Sci USA.* 90:6444-8.
Hoogenboom, H.R., A.D. Griffiths, K.S. Johnson, D.J. Chiswell, *et al.* 1991. Multi-subunit proteins on the surface of filamentous phage: methodologies for displaying antibody (Fab) heavy and light chains. *Nucleic Acids Res.* 19: 4133-7.
Houghten, RA., J.R. Appel, S.E. Blondelle, J.H. Cuervo, *et al.* 1992. The use of synthetic peptide combinatorial libraries for the identification of bioactive peptides. *Biotechniques.* 13:412-21.
Hsu, I.C., Q. Yang, M.W. Kahng, and J.F. Xu. 1994. Detection of DNA point mutations with DNA mismatch repair enzymes. *Carcinogenesis.* 15:1657-62.
Hwang, K.J., K.F. Luk, and P.L. Beaumier. 1980. Hepatic uptake and degradation of unilamellar sphingomyelin/cholesterol liposomes: a kinetic study. *Proc Natl Acad Sci USA.* 77:4030-4.
Hyrup, B., and P.E. Nielsen. 1996. Peptide nucleic acids (PNA): synthesis, properties and potential applications.

*Bioorg Med Chem.* 4:5-23.
Inoue, H., Y. Hayase, A. Imura, S. Iwai, *et al.* 1987a. Synthesis and hybridization studies on two complementary nona(2'-O- methyl)ribonucleotides. *Nucleic Acids Res.* 15:6131-48.
Inoue, H., Y. Hayase, S. Iwai, and E. Ohtsuka. 1987b. Sequence-dependent hydrolysis of RNA using modified oligonucleotide splints and RNase *H. FEBS Lett.* 215:327-30.
Ishiura, M., S. Hirose, T. Uchida, Y. Hamada, *et al.* 1982. Phage particle-mediated gene transfer to cultured mammalian cells. *Molecular and Cellular Biology.* 2:607-616.
Ito, H., Y. Fukuda, K. Murata, and A. Kimura. 1983. Transformation of intact yeast cells treated with alkali cations. *J. Bacteriol.* 153:163-168.
Iwabuchi, K., B. Li, P. Bartel, and S. Fields. 1993. Use of the two-hybrid system to identify the domain of p53 involved in oligomerization. *Oncogene.* 8:1693-6.
Jayasena, S.D. 1999. Aptamers: an emerging class of molecules that rival antibodies in diagnostics. *Clin Chem.* 45:1628-50.
Jones, P.T., P.H. Dear, J. Foote, M.S. Neuberger, *et al.* 1986. Replacing the complementarity-determining regions in a human antibody with those from a mouse. *Nature.* 321:522-5.
Kahn, J., F. Mehraban, G. Ingle, *X.* Xin, *et al.* 2000. Gene expression profiling in an in vitro model of angiogenesis. *Am J Pathol.* 156:1887-900.
Kaufman, R.J. 1990. Vectors used for expression in mammalian cells. *Methods Enzymol.* 185:487-511.
Kaufman, R.J., P. Murtha, D.E. Ingolia, C.-Y. Yeung *et al.* 1986. Selection and amplification of heterologous genes encoding adenosine deaminase in mammalian cells. *Proc. Natl. Acad. Sci.USA.* 83:3136-3140.
Kawai, S., and M. Nishizawa. 1984. New procedure for DNA transfection with polycation and dimethyl sulfoxide. *Mol. Cell. Biol.* 4:1172.
Keen, J., D. Lester, C. Inglehearn, A. Curtis, *et al.* 1991. Rapid detection of single base mismatches as heteroduplexes on Hydrolink gels. *Trends Genet.* 7:5.
Kelly, J.M., and M.J. Hynes. 1985. Transformation of Aspergillus niger by the amdS gene of Aspergillus nidulans. *Embo J.* 4:475-9.
Kostelny, S.A., M.S. Cole, and J.Y. Tso. 1992. Formation of a bispecific antibody by the use of leucine zippers. *J Immunol.* 148:1547-53.
WO94/16101. DNA SEQUENCING BY MASS SPECTROMETRY. 1994.
Kozal, M.J., N. Shah, N. Shen, R. Yang, *et al.* 1996. Extensive polymorphisms observed in HIV-1 clade B protease gene using high-density oligonucleotide arrays. *Nat Med.* 2:753-9.
Kozbor, D., P. Tripputi, J.C. Roder, and C.M. Croce. 1984. A human hybrid myeloma for production of human monoclonal antibodies. *J Immunol.* 133:3001-5.
Kriegler, M. 1990. Gene transfer and expression: A laboratory manual. Stockton Press, New York. 242 pp.
WO 91/01140. HOMOLOGOUS RECOMBINATION FOR UNIVERSAL DONOR CELLS AND CHIMERIC MAMMALIAN HOSTS. 1991.
Kwoh, D.Y., G.R. Davis, K.M. Whitfield, H.L. Chappelle, *et al.* 1989. Transcription-based amplification system and detection of amplified human immunodeficiency virus type 1 with a bead-based sandwich hybridization format. *Proc Natl Acad Sci USA.* 86:1173-7.
US Patent No. 5,223,409. Directed evolution of novel binding proteins. 1993.
Lakso, M., B. Sauer, B. Mosinger, E.J. Lee, *et al.* 1992. Targeted oncogene activation by site-specific recombination in transgenic mice. *Proc Natl Acad Sci USA.* 89:6232-6.
Lam, K.S. 1997. Application of combinatorial library methods in cancer research and drug discovery. *Anticancer Drug Design.* 12:145-167.
Lam, K.S., S.E. Salmon, E.M. Hersh, V.J. Hruby, *et al.* 1991. General method for rapid synthesis of multicomponent peptide mixtures. *Nature.* 354:82-84.
Landegren, U., R. Kaiser, J. Sanders, and L. Hood. 1988. A ligase-mediated gene detection technique. *Science.* 241:1077-80.
Lansink, M., P. Koolwijk, V. van Hinsbergh, and T. Kooistra. 1998. Effect of steroid hormones and retinoids on the formation of capillary- like tubular structures of human microvascular endothelial cells in fibrin matrices is related to urokinase expression. *Blood.* 92:927-38.
WO 90/11354. Process for the specific replacement of a copy of a gene present in the receiver genome via the integration of a gene. 1990.
U.S. Patent No. 4,736,866. Transgenic non-human animals. 1988.
Leduc, N., and e. al. 1996. Isolated maize zygotes mimic *in vivo* embryogenic development and express microinjected genes when cultured *in vitro. Dev. Biol.* 10:190-203.
Lee, J.S., D.A. Johnson, and A.R. Morgan. 1979. Complexes formed by (pyrimidine)n. (purine)n DNAs on lowering the pH are three-stranded. *Nucleic Acids Res.* 6:3073-91.

Lee, V.H.L. 1990. Peptide and protein drug delivery. Marcel Dekker, New York, NY.
Lemaitre, M:, B. Bayard, and B. Lebleu. 1987. Specific antiviral activity of a poly(L-lysine)-conjugated oligodeoxyribonucleotide sequence complementary to vesicular stomatitis virus N protein mRNA initiation site. *Proc Natl Acad Sci U S A.* 84:648-52.
Lemischka, I.R., D.H. Raulet, and R.C. Mulligan, 1986. Developmental potential and dynamic behavior of hematopoietic stem cells. *Cell.* 45:917-927.
Letsinger, R.L., G.R. Zhang, D.K. Sun, T. Ikeuchi, *et al.* 1989. Cholesteryl-conjugated oligonucleotides: synthesis, properties, and activity as inhibitors of replication of human immunodeficiency virus in cell culture. *Proc Natl Acad Sci USA.* 86:6553-6.
Li, E., T.H. Bestor, and R Jaenisch. 1992. Targeted mutation of the DNA methyltransferase gene results in embryonic lethality. *Cell.* 69:915-26.
Linder, M.W., R.A. Prough, and R Valdes. 1997. Pharmacogenetics: a laboratory tool for optimizing therapeutic efficiency. *Clin Chem.* 43:254-66.
Littlefield, J.W. 1964. Selection of hybrids from matings of fibroblasts in vitro and their presumed recombinants. *Science.* 145:709-710.
Lizardi, P.M., C.E. Guerra, H. Lomeli, I. Tussie-Luna, *et al.* 1988. Exponential amplification ofrecombinant-RNA hybridization probes. *Biotechnology.* 6:1197-1202.
Lonberg, N., and D. Huszar. 1995. Human antibodies from transgenic mice. *Int Rev Immunol.* 13:65-93.
Lonberg, N., L.D. Taylor, F.A. Harding, M. Trounstine, *et al.* 1994. Antigen-specific human antibodies from mice comprising four distinct genetic modifications [see comments]. *Nature.* 368:856-9.
Lopata, M.A., D.W. Cleveland, and B. Sollner-Webb. 1984. High-level expression of a chloramphenicol acetyltransferase gene by DEAEdextran-mediated DNA traansfection couled with a dimethylsulfoxide or glycerol shock treatment. *Nucleic Acids Research.* 12:5707.
Louvet-Vallee, S. 2000. ERM proteins: from cellular architecture to cell signaling. *Biol Cell.* 92:305-16.
Luckow, V.A. 1991. Cloning and expression of heterologous genes in insect cells with baculovirus vectors. *In* Recombinant DNA technology and applications. A. Prokop, R.K. Bajpai, and C. Ho, editors. McGraw-Hill, New York. 97-152.
Madura, K., R.J. Dohmen, and A. Varshavsky. 1993. N-recognin/Ubc2 interactions in the N-end rule pathway. *J Biol Chem.* 268:12046-54.
Maher, L.J. 1992. DNA triple-helix formation: an approach to artificial gene repressors? *Bioessays.* 14:807-15.
Mandel, M., and A. Higa. 1970. Calcium-dependent bacteriophage DNA infection. *J. Mol. biol.* 53:159-162.
Marasco, W.A., W.A. Haseltine, and S.Y. Chen. 1993. Design, intracellular expression, and activity of a human anti-human immunodeficiency virus type 1 gp 120 single-chain antibody, *Proc Natl Acad Sci USA.* 90:7889-93.
Marks, J.D., A.D. Griffiths, M. Malmqvist, T.P. Clackson, *et al.* 1992. By-passing immunization: building high affinity human antibodies by chain shuffling. *Biotechnology (N Y).* 10:779-83.
Marks, J.D., H.R. Hoogenboom, T.P. Bonnert, J. McCafferty, *et al.* 1991. By-passing immunization. Human antibodies from V-gene libraries displayed on phage. *J Mol Biol.* 222:581-97.
Martin, F.J., and D. Papahadjopoulos. 1982. Irreversible coupling of immunoglobulin fragments to preformed vesicles. An improved method for liposome targeting. *J Biol Chem.* 257:286-8.
Maxam, A.M., and W. Gilbert. 1977. A new method for sequencing DNA. *Proc Natl Acad Sci USA.* 74:560-4.
Miller, A.D., and C. Buttimore. 1986. Redesign of retrovirus packaging cell lines to avoid recombination leading to helper virus production. *Mol. Cell biol.* 6:2895-2902.
Miller, L.K. 1988. Baculoviruses as gene expression vectors. *Annu. Rev. Microbiol.* 42:177-199.
Milstein, C., and A.C. Cuello. 1983. Hybrid hybridomas and their use in immunohistochemistry. *Nature.* 305:537-40.
US Patent No. 5,459,039. Methods for mapping genetic mutations. 1995.
Morrison, S.L., L. Wims, S. Wallick, L. Tan, *et al.* 1987. Genetically engineered antibody molecules and their application. *Ann N Y Acad Sci.* 507:187-98.
US Patent No. 4,683,202. Process for amplifying nucleic acid sequences. 1987.
US Patent No. 4,683,195. Process for amplifying, detecting, and/or cloning nucleic acid sequences. 1987.
Munson, P.J., and D. Rodbard. 1980. Ligand: a versatile computerized approach for characterization of ligand-binding systems. *Anal Biochem.* 107:220-39.
Myers, R.M., Z. Larin, and T. Maniatis. 1985. Detection of single base substitutions by ribonuclease cleavage at mismatches in RNA:DNA duplexes. *Science.* 230:1242-6.
US Patent No. 5,328,470. Treatment of diseases by site-specific instillation of cells or site-specific transformation of cells and kits therefor. 1994.
Naeve, C.W., G.A. Buck, R.L. Niece, R.T. Pon, *et al.* 1995. Accuracy of automated DNA sequencing: a multi-laboratory comparison of sequencing results. *Biotechniques.* 19:448-53.
Nakai, K., and P. Horton. 1999. PSORT: a program for detecting sorting signals in proteins and predicting their subcellular localization. *Trends Biochem Sci.* 24:34-6.
Nakazawa, H., D. English, P.L. Randell, K. Nakazawa, *et al.* 1994. UV and skin cancer: specific p53 gene mutation in normal skin as a biologically relevant exposure measurement. *Proc Natl Acad Sci USA.* 91:360-4.
Neumann, E., M. Schaefer-Ridder, Y. Wang, and P.H. Hofschneider. 1982. Gene transfer into mouse lyoma cells by electroporation in high electric fields. *EMBO J.* 1:841-845.
O'Gorman, S., D.T. Fox, and G.M. Wahl. 1991. Recombinase-mediated gene activation and site-specific integration in mammalian cells. *Science.* 251:1351-5.
Okabe, S., T. Fukuda, K. Ishibashi, S. Kojima, *et al.* 1998. BAZF, a novel Bc16 homolog, functions as a transcriptional repressor. *Mol Cell Biol.* 18:4235-44.
Okano, H., J. Aruga, T. Nakagawa, C. Shiota, *et al.* 1991. Myelin basic protein gene and the function of antisense RNA in its repression in myelin-deficient mutant mouse. *J Neurochem.* 56:560-7.
OReilly, D.R., L.K. Miller, and V.A. Luckow. 1992. Baculovirus expression vectors. W.H. Freeman and Company, New York.
Orita, M., H. Iwahana, H. Kanazawa, K. Hayashi, *et al.* 1989. Detection of polymorphisms of human DNA by gel electrophoresis as single-strand conformation polymorphisms. *Proc Natl Acad Sci U S A.* 86:2766-70.
Ou-Lee, T.M., R. Turgeon, and R. Wu. 1986. Uptake and expression of a foreign gene linked to either a plant virus or *Drosophila* promoter in protoplasts of rice, wheat and sorghum. *Proc. Natl. Acad. Sci. USA.* 83:6815-6819.
Palmer, T.D., R.A. Hock, W.R.A. osbome, and A.D. Miller. 1987. Efficient retrovirus-mediated transfer and expression of a human adenosine deaminase gene in diploid skin fibroblasts from an adenosie-deficient human. *Proc. Natl. Acad. Sci. USA.* 84:1055-1059.
Pear, W., G. Nolan, M. Scott, and D. Baltimore. 1993. Production of high-titer helper-free retroviruses by transient transfection. *Proc. Natl. Acad. Sci. USA.* 90:8392-8396.
Peny-O'Keefe, H., X.W. Yao, J.M. Coull, M. Fuchs, *et al.* 1996. Peptide nucleic acid pre-gel hybridization: an alternative to southern hybridization. *Proc Natl Acad Sci U S* A. 93:14670-5.
Petersen, K.H., D.K. Jensen, M. Egholm, O. Buchardt, *et al.* 1976. A PNA-DNA linker synthesis of N-((4,4'-dimethoxytrityloxy)ehtyl)-N-(thymin-1-ylacetyl)glycine. *Biorganic and Medicianl Chemistry Letters.* 5:1119-1124.
Potter, H. 1988. Electroporation in biology: Methods, applications,, and instrumentation. *Analytical Biochemistry.* 174:361-373.
Potter, H., L. Weir, and P. Leder. 1984. Enhancer-dependent expression of human kappa immunoglobulin genes introduced into mouse pre-B lymphocytes by electroporation. *Proc. Natl. Acad. Sci. USA.* 81:7161-7165.
Presta, L.G. 1992. Antibody engineering. *Curr Opin Biotechnol. 3*:394-8.
Prosser, J. 1993. Detecting single-base mutations. *Trends Biotechnol.* 11:238-46.
Rassoulzadegan, M., B. Binetruy, and F. Cuzin. 1982. High frequency of gene transfer after fusion between bacteria and eukaryotic cells. *Nature.* 295:257.
Reisfeld, RA., and S. Sell. 1985. Monoclonal antibodies and cancer therapy: Proceedings of the Roche-UCLA symposium held in Park City, Utah, January 26-February 2, 1985. Alan R. Liss, New York. 609 pp.
Rhodes, C.A., D.A. Pierce, I.J. Mettler, D. Mascarenhas, *et al.* 1988. Genetically transformed maize plants from protoplasts. *Science.* 240:204-207.
Riechmann, L., M. Clark, H. Waldmann, and G. Winter. 1988. Reshaping human antibodies for therapy. *Nature.* 332:323-7.
Risau, W. 1995. Differentiation of endothelium. *Faseb J.* 9:926-33.
Risau, W., and I. Flamme. 1995. Vasculogenesis. *Annu Rev Cell Dev Biol.* 11:73-91.
Rojas, K., L. Serrano de la Pena, T. Gallardo, A. Simmons, *et al.* 1999. Physical map and characterization of transcripts in the candidate interval for familial chondrocalcinosis at chromosome 5p15.1. *Genomics.* 62:177-83.
Rose, J.K., L. Buonocore, and M. Whitt. 1991. A new cationic liposome reagent mediating nearly quantitative transfection of animal cells. *BioTechniques.* 10:520-525.
Rossi, J.J. 1994. Practical ribozymes. Making ribozymes work in cells. *Curr Biol.* 4:469-71.
Rossiter, B.J., and C.T. Caskey. 1990. Molecular scanning methods of mutation detection. *JBiol Chem.* 265:12753-6.
US Patent No. 5,283,317. Intermediates for conjugation of polypeptides with high molecular weight polyalkylene glycols. 1994.
Saiki, R.K., T.L. Bugawan, G.T. Horn, K.B. Mullis, *et al.* 1986. Analysis of enzymatically amplified beta-globin and HLA-DQ alpha DNA with allele-specific oligonucleotide probes. *Nature.* 324:163-6.
Saiki, R.K., P.S. Walsh, C.H. Levenson, and H.A. Erlich. 1989. Genetic analysis of amplified DNA with immobilized sequence-specific oligonucleotide probes. *Proc Natl Acad Sci USA.* 86:6230-4.
Saleeba, J.A., and R.G. Cotton. 1993. Chemical cleavage of mismatch to detect mutations. *Methods Enzymol. 217: 286-95.*
Sambrook, J. 1989. Molecular cloning: a laboratory manual. Cold Spring Harbor Laboratory, Cold Spring Harbor.
Sandri-Goldin, RM., A.L. Goldin, J.C. Glorioso, and M. Levine. 1981. High-frequency transfer of cloned herpes simjplex virus type I sequences to mammalian cells by protoplast fusion. *Mol. Cell. Biol.* 1:7453-752.
Sanger, F., S. Nicklen, and A.R. Coulson. 1977. DNA sequencing with chain-terminating inhibitors. *Proc Natl Acad Sci USA.* 74:5463-7.
Saunders, J.A., B.F. Matthews, and P.D. Miller. 1989. Plant gene transfer using electrofusion and electroporation. *In* Electroporation and electrofusion in cell biology. E. Neumann, A.E. Sowers, and C.A. Jordan, editors. Plenum Press, New York. 343-354.
Schade, R., C. Staak, C. Hendriksen, M. Erhard, *et al.* 1996. The production of avian (egg yold) antibodies: IgY. The report and recommendations of ECVAM workshop. *Alternatives to Laboratory Animals (ATLA).* 24:925-934.
Schaffner, W. 1980. Direct transfer of cloned genes from bacteria to mammalian cells. *Proc. Natl. Acad. Sci. USA.* 77:2163.
Schook, L.B. 1987. Monoclonal antibody production techniques and applications. Marcel Dekker, Inc., New York. 336 pp.
Scott, J.K., and G.P. Smith. 1990. Searching for peptide ligands with an epitope library. *Science.* 249:386-90.
Selden, R.F., K. Burke-Howie, M.E. Rowe, H.M. Goodman*, et al.* 1986. Human growth hormone as a reporter gene in regulation studies employing transient gene expression. *Molecular and Cellular Biololgy.* 6:3173-3179.
Shalaby, M.R., H.M. Shepard, L. Presta, M.L. Rodrigues, *et al.* 1992. Development of humanized bispecific antibodies reactive with cytotoxic lymphocytes and tumor cells overexpressing the HER2 protooncogene. *J Exp Med.* 175: 217-25.
Shigekawa, K., and W.J. Dower. 1988. Electroporation of eukaryotes and prokaryotes: A general approach to the introduction of macomolecules into cells. *BioTechniques.* 6:742-751.
Shillito, R. 1999. Methods of genetic transformations: Electroporation and polyethylene glycol treatment. *In* Molecular improvement of cereal crop. I. Vasil, editor. Kluwer, Dordrecht, The Netherlands. 9-20.
Shilo, B.Z., and R.A. Weinberg. 1981. DNA sequences homologous to vertebrate oncogenes are conserved in Drosophila melanogaster. *Proc Natl Acad Sci U S A.* 78:6789-92.
Shimkets, R.A., D.G. Lowe, J.T. Tai, P. Sehl, *et al.* 1999. Gene expression analysis by transcript profiling coupled to a gene database query. *NatBiotechnol.* 17:798-803.
Shopes, B. 1992. A genetically engineered human IgG mutant with enhanced cytolytic activity. *J Immunol.* 148: 2918-22:
Simonsen, C.C., and A.D. Levinson. 1983. Isolation and expression of an altered mouse dihydrofolate reductase cDNA. *Proc. Natl. Acad. Sci. USA.* 80:2495-2499.
US Patent No. 5,272,057. Method of detecting a predisposition to cancer by the use of restriction fragment length polymorphism of the gene for human poly (ADP-ribose) polymerase. 1993.
Southern, P.J., and P. Berg. 1982. Transformation of mammalian cells to antibiotic resistanced with a bacterial gene under control of the SV40 early region promoter. *J. Mol. Appl. Gen.* 1:327-341.
Sreekrishna, K., R.H. Potenz, J.A. Cruze, W.R McCombie, *et al.* 1988. High level expression of heterologous proteins in methylotrophic yeast Pichia pastoris. *J Basic Microbiol.* 28:265-78.
Stein, C.A., and J.S. Cohen. 1988. Oligodeoxynucleotides as inhibitors of gene expression: a review. *CancerRes.* 48:2659-68.
Stevenson, G.T., A. Pindar, and C.J. Slade.1989. A chimeric antibody with dual Fc regions (bisFabFc) prepared by manipulations at the IgG hinge. *Anticancer Drug Des.* 3:219-30.
Suresh, M.R, A.C. Cuello, and C. Milstein. 1986. Bispecific monoclonal antibodies from hybrid hybridomas. *Methods Enzymol.* 121:210-28.
Taylor, A., K. Obholz, G. Linden, S. Sadiev, *et al.* 1998. DNA sequence and muscle-specific expression of human sarcosin transcripts. *Mol Cell Biochem.* 183:105-12.
Thomas, K.R., and M.R. Capecchi. 1987. Site-directed mutagenesis by gene targeting in mouse embryo-derived stem cells. *Cell.* 51:503-12.
Thompson, J.A., and e. al. 1995. Maize transformation utilizing silicon carbide whiskers: A review. *Euphytica.* 85: 75-80.
Tilburn, J., C. Scazzocchio, G.G. Taylor, J.H. Zabicky-Zissman, *et al.* 1983.
Transformation by integration in Aspergillus nidulans. *Gene.* 26:205-21.
Touraev, A., and e. al. 1997. Plant male germ line transformation. *Plant J.* 12:949-956.
Traunecker, A., F. Oliveri, and K. Karjalainen. 1991. Myeloma based expression system for production of large mammalian proteins. *Trends Biotechnol.* 9:109-13.
Trick, H.N., and e. al. 1997. Recent advances in soybean transformation. *Plant Tissue Cult. Biotechnol. 3:9-26.*
Tuerk, C., and L. Gold. 1990. Systematic evolution of ligands by exponential enrichment: RNA ligands to bacteriophage T4 DNA polymerase. *Science.* 249:505-10.
Turner, D.L., E.Y. Snyder, and C.L. Cepko. 1990. Lineage-independent determinationh of cell type in the embryonic mouse retina. *Neuron.* 4:833-845.

Tutt, A., G.T. Stevenson, and M.J. Glennie. 1991. Trispecific F(ab')3 derivatives that use cooperative signaling via the TCR/CD3 complex and CD2 to activate and redirect resting cytotoxic T cells. *J Immunol.* 147:60-9.
van der Krol, A.R., J.N. Mol, and A.R. Stuitje. 1988b. Modulation of eukaryotic gene expression by complementary RNA or DNA sequences. *Biotechniques.* 6:958-76.
van der Krol, A.R., J.N. Mol, and A.R. Stuitje. 1988a. Modulation of eukaryotic gene expression by complementary RNA or DNA sequences. *Biotechniques.* 6:958-76.
Verhoeyen, M., C. Milstein, and G. Winter. 1988. Reshaping human antibodies: grafting an antilysozyme activity. *Science.* 239:1534-6.
Vitetta, E.S., R.J. Fulton, R.D. May, M. Till, *et al.* 1987. Redesigning nature's poisons to create anti-tumor reagents. *Science.* 238:1098-104.
US Patent No. 4,873,191. Genetic transformation of zygotes. 1989.
Wells, J.A., M. Vasser, and D.B. Powers. 1985. Cassette mutagenesis: an efficient method for generation of multiple mutations at defined sites. *Gene.* 34:315-23.
Whitt, M.A., L. Buonocore, J.K. Rose, V. Ciccarone, *et al.* 1990. TransfectACE reagent promotes transient transfection frequencies greater than 90%. *Focus.* 13:8-12.
Wigler, M., A. Pellicer, S. Silversttein, and R. Axel. 1978. Biochemical transfer of single-copy eucaryotic genes using total cellular DNA as donor. *Cell.* 14:725.
Williams, D.A., LR. Lemischka, D.G. Nathan, and R.C. Mulligan. 1984. Introduction of a new genetic material into pluripotent haematopoietic stem cells of the mouse. *Nature:* 310:476-480.
Wilmut, I., A.E. Schnieke, J. McWhir, A.J. Kind, *et al.* 1997. Viable offspring derived from fetal and adult mammalian cells. *Nature.* 385:810-3.
Wolff, E.A., G.J. Schreiber, W.L. Cosand, and H.V. Raff. 1993. Monoclonal antibody homodimers: enhanced antitumor activity in nude mice. *Cancer Res.* 53:2560-5.
Wong, T.K., and E. Neumann. 1982. Electric field mediated gene transfer. *Biochemical and Biophysical Research Communications.* 107:584-587.
Wyborski, D.L., L.C. DuCoeur, and J.M. Short. 1996. Parameters affecting the use of the lac repressor system in eukaryotic cells and transgenic animals. *Environ Mol Mutagen.* 28:447-58.
Wyborski, D.L., and J.M. Short. 1991. Analysis of inducers of the E.coli lac repressor system in mammalian cells and whole animals. *Nucleic Acids Res.* 19:4647-53.
Yang, S., J. Graham, J.W. Kahn, E.A. Schwartz, *et al.* 1999. Functional roles for PECAM-1 (CD31) and VE-cadherin (CD144) in tube assembly and lumen formation in three-dimensional collagen gels. *Am J Pathol.* 155:887-95.
Yelton, M.M., J.E. Hamer, and W.E. Timberlake. 1984. Transformation of Aspergillus nidulans by using a trpC plasmid. *Proc Natl Acad Sci USA.* 81:1470-4.
Zervos, A.S., J. Gyuris, and R. Brent. 1993. Mxi1, a protein that specifically interacts with Max to bind Myc-Max recognition sites. *Cell.* 72:223-32.
Zhou, G., and e. al. 1983. Introduction of exogenous DNA into cotton embryos. *Methods Enzymol.* 101:433-481.
Zoller, M.J., and M. Smith. 1987. Oligonucleotide-directed mutagenesis: a simple method using two oligonucleotide primers and a single-stranded DNA template. *Methods Enzymol.* 154:329-50.
Zon, G. 1988. Oligonucleotide analogues as potential chemotherapeutic agents. *Pharm Res.* 5:539-49.
Zuckermann, R.N., E.J. Martin, D.C. Spellmeyer, G.B. Stauber, *et al.* 1994. Discovery of nanomolar ligands for 7-transmembrane G-protein-coupled receptors from a diverse N-(substituted)glycine peptoid library. *J Med Chem.* 37:2678-85.

## Claims

1. An isolated polypeptide comprising an amino acid sequence having at least 90% sequence identity to the sequence of SEQ ID NO: 4, wherein expression of the polypeptide is upregulated during angiogenesis.

2. A polypeptide according to Claim 1 wherein said amino acid sequence comprises SEQ ID NO: 4.

3. A polypeptide according to Claim 1, wherein said amino acid sequence has at least 95% sequence identity to the sequence of SEQ ID NO: 4.

4. An isolated polynucleotide encoding the polypeptide of any of Claims 1-3, or a complement of said polynucleotide.

5. An isolated polynucleotide comprising a nucleotide sequence having at least 80% sequence identity to the sequence of SEQ ID NO: 3, or a complement of said polynucleotide, wherein expression of the polynucleotide is upregulated during angiogenesis.

**6.** A polynucleotide according to Claim 5, wherein said nucleotide sequence has at least 90% sequence identity to the sequence of SEQ ID NO: 3, or a complement of said polynucleotide.

**7.** A polynucleotide according to Claim 5, wherein said nucleotide sequence has at least 98% sequence identity to the sequence of SEQ ID NO: 3, or a complement of said polynucleotide.

**8.** A vector comprising a polynucleotide according to any of Claims 4-7.

**9.** A cell comprising a vector according to Claim 8.

**10.** An antibody that specifically binds to a polypeptide according to any of Claims 1-3.

**11.** A method of detecting angiogenesis in a tissue sample, comprising measuring expression of a polypeptide according to any of Claims 1-3 or a polynucleotide according to any of Claims 4-7 in the tissue sample.

**12.** A method according to Claim 11, wherein detecting angiogenesis indicates the presence of cancer or tumour.

**13.** A method of identifying an antagonist of angiogenesis, comprising;

a) contacting a cell or tissue sample undergoing angiogenesis with a candidate agent; and
b) detecting expression of a polypeptide according to any of Claims 1-3 or a polynucleotide according to any of Claims 4-7, wherein a candidate compound that decreases expression of the polypeptide is identified as an antagonist of angiogenesis.

**14.** A method of identifying an agonist of angiogenesis, comprising:

a) contacting a cell or tissue sample with a candidate compound; and
b) detecting expression of a polypeptide according to any of Claims 1-3 or a polynucleotide according to any of Claims 4-7, wherein a candidate compound that increases expression of the polypeptide is identified as an agonist of angiogenesis.

**15.** A method of screening a tissue sample for tumorigenic potential, comprising: measuring expression of a polypeptide according to any one of Claims 1-3 or a polynucleotide according to any one of Claims 4-7 in said tissue sample.

**16.** A method of determining the clinical stage of a tumour in a subject comprising:

a) detecting expression of a polypeptide according to any of Claims 1-3 or a polynucleotide according to any of Claims 4-7 in a tissue sample from the subject; and
b) comparing expression of the polypeptide to expression of the polypeptide in a control sample.

**17.** A method of monitoring the effectiveness of a treatment for an angiogenic disorder in a subject, comprising detecting expression of a polypeptide according to any of Claims 1-3 in a tissue sample from the subject, wherein a decrease in expression of the polypeptide indicates efficacy of treatment.

**18.** A method according to Claim 14, wherein the angiogenic disorder is cancer or tumor.

**19.** An *in vitro* method for determining whether a compound up-regulates or down-regulates expression of a polynucleotide according to any of Claims 4-7, comprising contacting a cell or tissue with the compound and detecting expression of the polynucleotide.

**20.** A transgenic non-human animal, having a disruption in a polynucleotide having at least 80% sequence identity to SEQ ID NO: 3, wherein the disruption prevents expression of a polypeptide encoded by the sequence.

**21.** A transgenic non-human animal according to Claim 20, wherein the non-human animal is a mouse.

**22.** A transgenic non-human animal, comprising an exogenous polynucleotide having at least 80% sequence identity to the sequence of SEQ ID NO: 3, or a complement of said polynucleotide.

**23.** A transgenic non-human animal according to Claim 22, wherein said exogenous polynucleotide has at least 90% sequence identity to the sequence of SEQ ID NO: 3, or a complement of said polynucleotide, preferably at least 98% sequence identity to the sequence of SEQ ID NO: 3, or a complement of said polynucleotide.

## Patentansprüche

**1.** Isoliertes Polypeptid, umfassend eine Aminosäuresequenz, die mindestens 90% Sequenzidentität mit der Sequenz mit SEQ ID Nr.: 4 aufweist, wobei die Expression des Polypeptids während der Angiogenese nach oben reguliert wird.

**2.** Polypeptid nach Anspruch 1, wobei die Aminosäuresequenz SEQ ID Nr.: 4 umfasst.

**3.** Polypeptid nach Anspruch 1, wobei die Aminosäuresequenz mindestens 95% Sequenzidentität mit der Sequenz mit SEQ ID Nr.: 4 aufweist.

**4.** Isoliertes Polynukleotid, kodierend für das Polypeptid nach einem der Ansprüche 1-3, oder ein Komplement des Polynukleotids.

**5.** Isoliertes Polynukleotid, umfassend eine Nukleotidsequenz, die mindestens 80% Sequenzidentität mit der Sequenz mit SEQ ID Nr.: 3 aufweist, oder ein Komplement des Polynukleotids, wobei die Expression des Polynukleotids während der Angiogenese nach oben reguliert wird.

**6.** Polynukleotid nach Anspruch 5, wobei die Nukleotidsequenz mindestens 90% Sequenzidentität mit der Sequenz mit SEQ ID Nr.: 3 aufweist, oder ein Komplement des Polynukleotids.

**7.** Polynukleotid nach Anspruch 5, wobei die Nukleotidsequenz mindestens 98% Sequenzidentität mit der Sequenz mit SEQ ID Nr.: 3 aufweist, oder ein Komplement des Polynukleotids.

**8.** Vektor, umfassend ein Polynukleotid nach einem der Ansprüche 4-7.

**9.** Zelle, umfassend einen Vektor nach Anspruch 8.

**10.** Antikörper, der spezifisch an ein Polypeptid nach einem der Ansprüche 1-3 bindet.

**11.** Verfahren zum Nachweisen von Angiogenese in einer Gewebeprobe, umfassend das Messen der Expression eines Polypeptids nach einem der Ansprüche 1-3 oder eines Polynukleotids nach einem der Ansprüche 4-7 in der Gewebeprobe.

**12.** Verfahren nach Anspruch 11, wobei das Nachweisen einer Angiogenese das Vorhandensein von Krebs oder eines Tumors anzeigt.

**13.** Verfahren zum Identifizieren eines Angiogeneseantagonisten, umfassend:

a) In-Kontakt-Bringen einer Zell- oder Gewebeprobe, welche Angiogenese erfährt, mit einem Kandidatenagens; und
b) Nachweisen der Expression eines Polypeptids nach einem der Ansprüche 1-3 oder eines Polynukleotids nach einem der Ansprüche 4-7, wobei eine Kandidatenverbindung, welche die Expression des Polypeptids verringert, als Angiogeneseantagonist identifiziert wird.

**14.** Verfahren zum Identifizieren eines Angiogeneseagonisten, umfassend:

a) In-Kontakt-Bringen einer Zell- oder Gewebeprobe mit einer Kandidatenverbindung; und
b) Nachweisen der Expression eines Polypeptids nach einem der Ansprüche 1-3 oder eines Polynukleotids nach einem der Ansprüche 4-7, wobei eine Kandidatenverbindung, welche die Expression des Polypeptids steigert, als Angiogeneseagonist identifiziert wird.

**15.** Verfahren zum Durchsuchen einer Gewebeprobe auf tumorigenes Potential, umfassend:

Messen der Expression eines Polypeptids nach einem der Ansprüche 1-3 oder eines Polynukleotids nach einem der Ansprüche 4-7 in der Gewebeprobe.

**16.** Verfahren zum Bestimmen des klinischen Stadiums eines Tumors in einem Probanden, umfassend:

a) Nachweisen der Expression eines Polypeptids nach einem der Ansprüche 1-3 oder eines Polynukleotids nach einem der Ansprüche 4-7 in einer Gewebeprobe aus dem Probanden; und
b) Vergleichen der Expression des Polypeptids mit der Expression des Polypeptids in einer Kontrollprobe.

**17.** Verfahren zum Überwachen der Wirksamkeit einer Behandlung für eine angiogene Funktionsstörung in einem Probanden, umfassend das Nachweisen der Expression eines Polypeptids nach einem der Ansprüche 1-3 in einer Gewebeprobe aus dem Probanden, wobei eine Abnahme der Expression des Polypeptids die Effizienz der Behandlung anzeigt.

**18.** Verfahren nach Anspruch 14, wobei die angiogene Funktionsstörung Krebs oder ein Tumor ist.

**19.** *In vitro* Verfahren zum Bestimmen, ob eine Verbindung die Expression eines Polynukleotids nach einem der Ansprüche 4-7 nach oben reguliert oder nach unten reguliert, umfassend das In-Kontakt-Bringen einer Zelle oder eines Gewebes mit der Verbindung und das Nachweisen der Expression des Polynukleotids.

**20.** Transgenes nichtmenschliches Lebewesen, das eine Disruption hat in einem Polynukleotid, das mindestens 80% Sequenzidentität mit SEQ ID Nr.: 3 aufweist, wobei die Disruption der Expression eines Polypeptids vorbeugt, für das von der Sequenz kodiert wird.

**21.** Transgenes nichtmenschliches Lebewesen nach Anspruch 20, wobei das nichtmenschliche Lebewesen eine Maus ist.

**22.** Transgenes nichtmenschliches Lebewesen, umfassend ein exogenes Polynukleotid, das mindestens 80% Sequenzidentität mit der Sequenz mit SEQ ID Nr.: 3 aufweist, oder ein Komplement des Polynukleotids.

**23.** Transgenes nichtmenschliches Lebewesen nach Anspruch 22, wobei das exogene Polynukleotid mindestens 90% Sequenzidentität mit der Sequenz mit SEQ ID Nr.: 3 aufweist, oder ein Komplement des Polynukleotids, und vorzugsweise mindestens 98% Sequenzidentität mit der Sequenz mit SEQ ID Nr.: 3 aufweist, oder ein Komplement des Polynukleotids.

## Revendications

**1.** Polypeptide isolé comprenant une séquence d'acides aminés ayant une identité de séquence d'au moins 90 % avec la séquence SEQ ID NO: 4, où l'expression du polypeptide est régulée à la hausse au cours de l'angiogenèse.

**2.** Polypeptide selon la revendication 1, où ladite séquence d'acides aminés comprend la SEQ ID NO:4.

**3.** Polypeptide selon la revendication 1, où ladite séquence d'acides aminés a une identité de séquence d'au moins 95 % avec la séquence SEQ ID NO:4.

**4.** Polynucléotide isolé codant le polypeptide selon l'une quelconque des revendications 1 à 3, ou complément dudit polynucléotide.

**5.** Polynucléotide isolé comprenant une séquence de nucléotide ayant une identité de séquence d'au moins 80 % avec la séquence SEQ ID NO: 3, ou complément dudit polynucléotide, où l'expression du polynucléotide est régulée à la hausse au cours de l'angiogenèse.

**6.** Polynucléotide selon la revendication 5, où ladite séquence de nucléotide a une identité de séquence d'au moins 90 % avec la séquence SEQ ID NO: 3, ou un complément dudit polynucléotide.

**7.** Polynucléotide selon la revendication 5, où ladite séquence de nucléotide a une identité de séquence d'au moins 98 % avec la séquence SEQ ID NO: 3, ou un complément dudit polynucléotide.

**8.** Vecteur comprenant un polynucléotide selon l'une quelconque des revendications 4 à 7.

**9.** Cellule comprenant un vecteur selon la revendication 8.

**10.** Anticorps qui lie spécifiquement un polypeptide selon l'une quelconque des revendications 1 à 3.

**11.** Procédé de détection de l'angiogenèse dans un échantillon de tissu, comprenant la mesure de l'expression d'un polypeptide selon l'une quelconque des revendications 1 à 3 ou d'un polynucléotide selon l'une quelconque des revendications 4 à 7 dans l'échantillon de tissu.

**12.** Procédé selon la revendication 11, dans lequel la détection de l'angiogenèse indique la présence d'un cancer ou d'une tumeur.

**13.** Procédé d'identification d'un antagoniste de l'angiogenèse, comprenant :

a) la mise en contact d'une cellule ou d'un échantillon de tissu subissant l'angiogenèse avec un agent candidat ; et
b) la détection de l'expression d'un polypeptide selon l'une quelconque des revendications 1 à 3 ou d'un polynucléotide selon l'une quelconque des revendications 4 à 7, où un composé candidat qui diminue l'expression du polypeptide est identifié comme antagoniste de l'angiogenèse.

**14.** Procédé d'identification d'un agoniste de l'angiogenèse, comprenant :

a) la mise en contact d'une cellule ou d'un échantillon de tissu avec un composé candidat ; et
b) la détection de l'expression d'un polypeptide selon l'une quelconque des revendications 1 à 3 ou d'un polynucléotide selon l'une quelconque des revendications 4 à 7, où un composé candidat qui augmente l'expression du polypeptide est identifié comme agoniste de l'angiogenèse.

**15.** Procédé de criblage d'un échantillon de tissu pour le potentiel tumorigène, comprenant : la mesure de l'expression d'un polypeptide selon l'une quelconque des revendications 1 à 3 ou d'un polynucléotide selon l'une quelconque des revendications 4 à 7 dans ledit échantillon de tissu.

**16.** Procédé de détermination du stade clinique d'une tumeur chez un sujet, comprenant :

a) la détection de l'expression d'un polypeptide selon l'une quelconque des revendications 1 à 3 ou d'un polynucléotide selon l'une quelconque des revendications 4 à 7 dans un échantillon de tissu prélevé chez un sujet ; et
b) la comparaison de l'expression du polypeptide à l'expression du polypeptide dans un échantillon témoin.

**17.** Procédé de surveillance de l'efficacité d'un traitement pour un trouble angiogénique chez un sujet, comprenant la détection de l'expression d'un polypeptide selon l'une quelconque des revendications 1 à 3 dans un échantillon de tissu prélevé chez le sujet, où une diminution de l'expression du polypeptide indique l'efficacité du traitement.

**18.** Procédé selon la revendication 14, où le trouble angiogénique est le cancer ou la tumeur.

**19.** Procédé *in vitro* permettant de déterminer si un composé régule à la hausse ou à la baisse l'expression d'un polynucléotide selon l'une quelconque des revendications 4 à 7, comprenant la mise en contact d'une cellule ou d'un tissu avec le composé et la détection de l'expression du polynucléotide.

**20.** Animal non humain transgénique, ayant une perturbation dans un polynucléotide, ayant une identité de séquence d'au moins 80 % avec la SEQ ID NO: 3, où la perturbation empêche l'expression d'un polypeptide codé par la séquence.

**21.** Animal non humain transgénique selon la revendication 20, où l'animal non humain est une souris.

**22.** Animal non humain transgénique, comprenant un polynucléotide exogène ayant une identité de séquence d'au moins 80 % avec la SEQ ID NO: 3, ou un complément dudit polynucléotide.

**23.** Animal non humain transgénique selon la revendication 22, où ledit polynucléotide exogène a une identité de séquence d'au moins 90 % avec la séquence SEQ ID NO: 3, ou complément dudit polynucléotide, de préférence une identité de séquence d'au moins 98 % avec la séquence de SEQ ID NO: 3, ou un complément dudit polynucléotide.